(19) Europäisches Patentamt
European Patent Office
Office européen des brevets

(11) EP 1 742 947 B1

(12) **FASCICULE DE BREVET EUROPEEN**

(45) Date de publication et mention de la délivrance du brevet:
**20.08.2008 Bulletin 2008/34**

(21) Numéro de dépôt: **05759933.4**

(22) Date de dépôt: **20.04.2005**

(51) Int Cl.:
*C07D 471/04* (2006.01) *A61K 31/437* (2006.01)
*A61K 31/407* (2006.01)

(86) Numéro de dépôt international:
**PCT/FR2005/000971**

(87) Numéro de publication internationale:
**WO 2005/108398 (17.11.2005 Gazette 2005/46)**

(54) **DERIVES DE PYRIDOINDOLONE SUBSTITUES EN -6, LEUR PREPARATION, LEUR APPLICATION EN THERAPEUTIQUE.**

HERSTELLUNG VON 6-SUBSTITUIERTEN PYRIDOINDOLON-DERIVATEN UND IHRE THERAPEUTISCHE VERWENDUNG

6-SUBSTITUTED PYRIDOINDOLONE DERIVATIVES PRODUCTION AND THERAPEUTIC USE THEREOF

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **21.04.2004 FR 0404251**

(43) Date de publication de la demande:
**17.01.2007 Bulletin 2007/03**

(73) Titulaire: **Sanofi-Aventis**
**75013 Paris (FR)**

(72) Inventeurs:
- **BOURRIE, Bernard**
**F-34980 Saint-Gély-du-Fesc (FR)**
- **CASELLAS, Pierre**
**F-34090 Montpellier (FR)**
- **CIAPETTI, Paola**
**F-67120 ALTORF (FR)**
- **DEROCQ, Jean Marie**
**F-34570 Murviel-les-Montpellier (FR)**
- **JEGHAM, Samir**
**F-34980 Montferrier-sur-Lez (FR)**
- **MUNEAUX, Yvette**
**F-34270 Les Matelles (FR)**
- **WERMUTH, Camille-George**
**F-67100 Strasbourg (FR)**

(74) Mandataire: **Weber, Mathieu et al**
**Sanofi-Aventis**
**174 avenue de France**
**75013 Paris (FR)**

(56) Documents cités:
**WO-A-02/087574** **WO-A-02/087575**
**WO-A-20/04041817** **FR-A- 2 003 999**
**FR-A- 2 765 581**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 742 947 B1

Printed by Jouve, 75001 PARIS (FR)

## Description

La présente invention se rapporte à des dérivés de pyridoindolone substitués en -6, à leur préparation et à leur application en thérapeutique.

L'art antérieur

Le brevet français N° 97 08409 décrit des composés de formule :

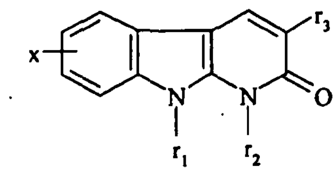

(A)

dans laquelle :

- x représente un atome d'hydrogène ou de chlore ou un groupe méthyle ou méthoxy ;
- $r_1$ représente un atome d'hydrogène ou un groupe méthyle ou éthyle ;
- $r_2$ représente un groupe méthyle ou éthyle ; ou bien
- $r_1$ et $r_2$ forment ensemble un groupe $(CH_2)_3$ ;
- $r_3$ représente, soit un groupe phényle éventuellement substitué par un atome d'halogène ou un groupe méthyle ou méthoxy, soit un groupe thiényle.

Dans la description de ce brevet, il est mentionné que les composés de formule (A) ayant une affinité pour les sites modulateurs oméga associés aux récepteurs $GABA_A$, peuvent être utilisés dans le traitement d'affections liés aux désordres de la transmission gabaergique associés aux sous-types de récepteurs $GABA_A$, tels que l'anxiété, les troubles du sommeil, l'épilepsie etc...

Les demandes de brevet internationales WO 2002/087574 et WO 2002/087575 décrivent l'utilisation des composés de formule (A) comme agents anticancéreux et leur association avec d'autres agents anticancéreux. Les composés décrits dans ces deux demandes se différencient notamment de ceux de la présente invention par la nature du substituant x sur le cycle indole qui ne peut être qu'un atome d'hydrogène ou de chlore ou bien un groupe méthyle ou méthoxy.

La demande de brevet internationale WO 2004/041817 décrit des composés de formule :

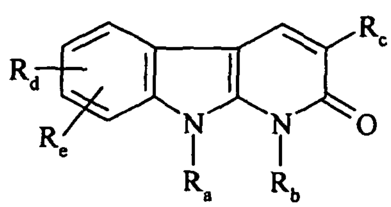

(B)

dans laquelle $R_a$, $R_b$, $R_c$, $R_d$ et $R_e$ ont différentes valeurs. Ces composés présentent une activité anticancéreuse.

La présente invention a pour objet des composés répondant à la formule :

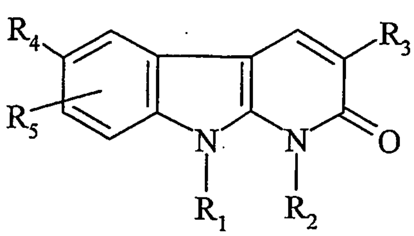

(I)

dans laquelle :

- $R_1$ représente un atome d'hydrogène ; un groupe ($C_1$-$C_4$)alkyle ; un groupe
- $(CH_2)_mOH$ ; un groupe $-(CH_2)_mCN$ ; un groupe $-(CH_2)_mNR_9R_{10}$;
- $R_2$ représente un atome d'hydrogène ou un groupe ($C_1$-$C_4$)alkyle;
- $R_3$ représente un phényle substitué par $R_6$, $R_7$, $R_8$ ;
- $R_4$ représente :
  - un groupe 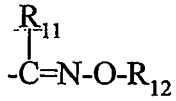
  - un radical hétérocyclique choisi parmi :

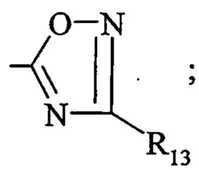 ; 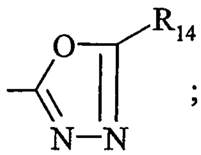 ; 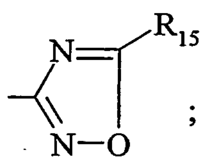 ;

- $R_5$ représente un atome d'hydrogène ou un groupe ($C_1$-$C_4$)alkyle ;
- $R_6$, $R_7$ et $R_8$ représentent chacun indépendamment l'un de l'autre un atome d'hydrogène ; un atome d'halogène ; un groupe ($C_1$-$C_4$)alkyle ; un groupe ($C_1$-$C_4$)alcoxy ; un hydroxy ; un cyano ; un groupe $-(CH_2)_nNR_9R_{10}$ ; un groupe $-O-(CH_2)_mNR_9R_{10}$ ;
- $R_9$ et $R_{10}$ représentent chacun indépendamment l'un de l'autre un atome d'hydrogène ou un groupe ($C_1$-$C_4$)alkyle ;
- ou bien $R_9$ et $R_{10}$ ensemble avec l'atome d'azote auquel ils sont liés constituent un radical hétérocyclique choisi parmi la pyrrolidin-1-yle, la pipéridin-1-yle, la morpholin-4-yle ou la pipérazin-1-yle non substitué ou substitué en position -4- par un ($C_1$-$C_4$)alkyle;
- $R_1$ représente un atome d'hydrogène ou un groupe ($C_1$-$C_4$)alkyle ;
- $R_{12}$ représente un atome d'hydrogène ; un groupe ($C_1$-$C_4$)alkyle ; un groupe $-(CH_2)_m-CO-R^{16}$ ;
- $R_{13}$ représente un atome d'hydrogène ; un groupe ($C_1$-$C_4$)alkyle ; un phényle ; un groupe $-NR_{17}R_{18}$ ; un groupe

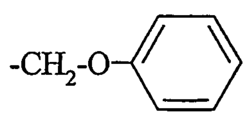 ;

- $R_{14}$ représente un atome d'hydrogène ; un groupe ($C_1$-$C_4$)alkyle ; un groupe $-NR_{17}R_{18}$ ;
- $R_{15}$ représente un atome d'hydrogène ; un groupe ($C_1$-$C_4$)alkyle ; un groupe - $NR_{19}R_{20}$ ; un groupe -COO($C_1$-$C_4$) alkyle ;
- $R_{16}$ représente un hydroxy ; un ($C_1$-$C_4$)alcoxy ; un groupe $NR_9R_{10}$ ;
- $R_{17}$ et $R_{18}$ représentent chacun indépendamment un atome d'hydrogène ou un groupe ($C_1$-$C_4$)alkyle ; $R_{18}$ peut de plus représenter un groupe $-COR_{21}$ ; un groupe $-SO_2R_{22}$ ;
- $R_{19}$ et $R_{20}$ représentent chacun indépendamment un atome d'hydrogène ou un groupe ($C_1$-$C_4$)alkyle ; $R_{20}$ peut de plus représenter un groupe ($C_3$-$C_6$)cycloalkyle, un groupe ($C_3$-$C_6$)cycloalkylméthyle, un groupe $-(CH_2)_mNR_9R_{10}$ ;
- $R_{21}$ représente un groupe ($C_1$-$C_4$)alkyle ; un groupe ($C_3$-$C_6$)cycloalkyle ; un groupe $-(CH_2)_mNR_9R_{10}$ ;
- $R_{22}$ représente un groupe ($C_1$-$C_4$)alkyle ;
- m est 1, 2 ou 3 ;
- n est 0, 1, 2 ou 3.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels

d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par :

- un atome d'halogène : un fluor, un chlore, un brome, ou un iode ;
- un groupe ($C_1$-$C_4$)alkyle : un groupe aliphatique saturé linéaire ou ramifié comportant 1 à 4 atomes de carbone. A titre d'exemple, on peut citer les groupes méthyle, éthyle, propyle, isopropyle, butyle, isobutyle, *tert*-butyle ;
- un groupe ($C_1$-$C_4$)alcoxy : un radical O-alkyle où le groupe alkyle est tel que précédemment défini.
- un groupe ($C_3$-$C_6$)cycloalkyle : un groupe alkyle cyclique de 3 à 6 atomes de carbone tel que le groupe cyclopropyle, cyclobutyle, cyclopentyle ou cyclohexyle.

Parmi les composés de formule (I) objets de l'invention, on peut citer les composés préférés qui se définissent comme suit :

- $R_1$ représente un atome d'hydrogène ou un groupe ($C_1$-$C_4$)alkyle ;
- $R_2$ représente un atome d'hydrogène ou un groupe ($C_1$-$C_4$)alkyle ;
- $R_3$ représente un radical phényle substitué par $R_6$, $R_7$, $R_8$ ;
- $R_4$ représente un groupe hydroxyimidoformyle, ($C_1$-$C_4$)alcoxyimidoformyle, un radical oxadiazolyle non substitué ou substitué par un groupe ($C_1$-$C_4$)alkyle, phényle ou amino ;
- $R_5$ représente l'hydrogène ou un groupe ($C_1$-$C_4$)alkyle ;
- $R_6$, $R_7$ et $R_8$ représentent chacun indépendamment l'un de l'autre un atome d'hydrogène ou d'halogène, un groupe ($C_1$-$C_4$)alkyle, ($C_1$-$C_4$)alcoxy, amino, monométhylamino, diméthylamino.

Parmi les composés de formule (I) objets de l'invention, on peut aussi citer les composés préférés qui se définissent comme suit :

- $R_1$ représente un atome d'hydrogène, un méthyle, un éthyle, un cyanométhyle, un 2-morpholin-4-yléthyle ;
- et/ou $R_2$ représente un méthyle ;
- et/ou $R_3$ représente un phényle, un 3-bromophényle, un 4-bromophényle, un 2-chlorophényle, un 3-chlorophényle, un 4-chlorophényle, un 3-fluorophényle, un 4-fluorophényle, un 3-méthylphényle, un 2-méthoxyphényle, un 3-méthoxyphényle, un 4-méthoxyphényle, un 3-cyanophényle, un 4-cyanophényle, un 2,4-dichlorophényle, un 3,5-difluorophényle, un 2,4-diméthylphényle, un 2,4-diméthoxyphényle, un 2-méthyl-5-fluorophényle, un 3-fluoro-4-méthylphényle, un 3-méthyl-4-fluorophényle, un 4-(aminométhyl)phényle, un 4-(morpholin-4-ylméthyl)phényle, un 4-(2-morpholin-4-yléthoxy)phényle ;
- et/ou $R_4$ représente :

  un groupe (hydroxyimino)méthyle, un groupe N-hydroxyéthanimidoyle, un groupe (éthoxyimino)méthyle, un groupe N-éthoxyéthanimidoyle, un groupe (isobutoxyimino)méthyle, un groupe [(carboxyméthoxy)imino]méthyle, un groupe [(2-éthoxy-2-oxoéthoxy)imino]méthyle, un groupe [(2-morpholin-4-yl-2-oxoéthoxy)imino] méthyle ;

  . un 3-méthyl-1,2,4-oxadiazol-5-yle, un 3-phényl-1,2,4-oxadiazol-5-yle, un 3-amino-1,2,4-oxadiazol-5-yle, un 3-(diméthylamino)-1,2,4-oxadiazol-5-yle, un 3-[(cyclopropylcarbonyl)amino]-1,2,4-oxadiazol-5-yle, un 3-[(N,N-diméthyl glycyl)amino]-1,2,4-oxadiazol-5-yle, un 3-[(méthylsulfonyl)amino]-1,2,4-oxadiazol-5-yle, un 3-(phénoxyméthyl)-1,2,4-oxadiazol-5-yle ;

  . un 5-méthyl-1,3,4-oxadiazol-2-yle, un 5-amino-1,3,4-oxadiazol-2-yle ;

  . un 5-méthyl-1,2,4-oxadiazol-3-yle, un 5-amino-1,2,4-oxadiazol-3-yle, un 5-(diméthylamino)-1,2,4-oxadiazol-3-yle, un 5-(cyclopropylamino)-1,2,4-oxadiazol-3-yle, un 5-[(cyclopropylméthyl)amino]-1,2,4-oxadiazol-3-yle, un 5-[(3-morpholin-4-ylpropyl)amino]-1,2,4-oxadiazol-3-yle, un 5-[[2-(diméthyl amino)éthyl]amino]-1,2,4-oxadiazol-3-yle, un 5-(éthoxycarbonyl)-1,2,4-oxadiazol-3-yle ;

- et/ou $R_5$ représente un atome d'hydrogène ;

à l'état de base ou de sel d'addition à un acide, ainsi qu'à l'état d'hydrate ou de solvat.

Parmi les composés de ce dernier groupe, on peut citer les composés de formule (I) pour lesquels :

- $R_1$ représente un atome d'hydrogène, un méthyle, un éthyle, un cyanométhyle, un 2-morpholin-4-yléthyle ;
- $R_2$ représente un méthyle ;
- $R_3$ représente un phényle, un 3-bromophényle, un 4-bromophényle, un 2-chlorophényle, un 3-chlorophényle, un 4-chlorophényle, un 3-fluorophényle, un 4-fluorophényle, un 3-méthylphényle, un 2-méthoxyphényle, un 3-méthoxyphényle, un 4-méthoxyphényle, un 3-cyanophényle, un 4-cyanophényle, un 2,4-dichlorophényle, un 3,5-difluorophényle, un 2,4-diméthylphényle, un 2,4-diméthoxyphényle, un 2-méthyl-5-fluorophényle, un 3-fluoro-4-méthylphényle, un 3-méthyl-4-fluorophényle, un 4-(aminométhyl)phényle, un 4-(morpholin-4-ylméthyl)phényle, un 4-(2-morpholin-4-yléthoxy)phényle ;
- $R_4$ représente :

    • un groupe (hydroxyimino)méthyle, un groupe N-hydroxyéthanimidoyle, un groupe (éthoxyimino)méthyle, un groupe N-éthoxyéthanimidoyle, un groupe (isobutoxyimino)méthyle, un groupe [(carboxyméthoxy)imino]méthyle, un groupe [(2-éthoxy-2-oxoéthoxy)imino]méthyle, un groupe [(2-morpholin-4-yl-2-oxoéthoxy)imino] méthyle ;
    • un 3-méthyl-1,2,4-oxadiazol-5-yle, un 3-phényl-1,2,4-oxadiazol-5-yle, un 3-amino-1,2,4-oxadiazol-5-yle, un 3-(diméthylamino)-1,2,4-oxadiazol-5-yle, un 3-[(cyclopropylcarbonyl)amino]-1,2,4-oxadiazol-5-yle, un 3-[(N,N-diméthyl glycyl)amino]-1,2,4-oxadiazol-5-yle, un 3-[(méthylsulfonyl)amino]-1,2,4-oxadiazol-5-yle, un 3-(phénoxyméthyl)-1,2,4-oxadiazol-5-yle ;
    • un 5-méthyl-1,3,4-oxadiazol-2-yle, un 5-amino-1,3,4-oxadiazol-2-yle ;
    • un 5-méthyl-1,2,4-oxadiazol-3-yle, un 5-amino-1,2,4-oxadiazol-3-yle, un 5-(diméthylamino)-1,2,4-oxadiazol-3-yle, un 5-(cyclopropylamino)-1,2,4-oxadiazol-3-yle, un 5-[(cyclopropylméthyl)amino]-1,2,4-oxadiazol-3-yle, un 5-[(3-morpholin-4-ylpropyl)amino]-1,2,4-oxadiazol-3-yle, un 5-[[2-(diméthyl amino)éthyl]amino]-1,2,4-oxadiazol-3-yle, un 5-(éthoxycarbonyl)-1,2,4-oxadiazol-3-yle ;

- $R_5$ représente un atome d'hydrogéne ; à l'état de base ou de sel d'addition à un acide, ainsi qu'à l'état d'hydrate ou de solvat.

Parmi les composés de formule (I), objets de l'invention, on peut notamment citer les composés suivants :

- 6-(3-Amino-1,2,4-oxadiazol-5-yl)-3-(2,4-dichlorophényl)-1,9-diméthyl-1,9-dihydro-2*H*-pyrido[2,3-b]indol-2-one.
- 3-(2,4-Dichlorophényl)-1,9-diméthyl-2-oxo-2,9-dihydro-1*H*-pyrido[2,3-b]indole-6-carbaldéhyde oxime ;
- 3-(2,4-Dichlorophényl)-1,9-diméthyl-2-oxo-2,9-dihydro-1*H*-pyrido[2,3-b]indole-6-carbaldéhyde O-éthyloxime ;
- 5-[3-(4-Chlorophényl)-1,9-diméthyl-2,9-dihydro-1*H*-pyrido[2,3-b]indole-6-yl]-1,2,4-oxadiazol-3-amine ;
- 5-[3-(3-Fluorophényl)-1,9-diméthyl-2,9-dihydro-1*H*-pyrido[2,3-b]indole-6-yl]-1,2,4-oxadiazol-3-amine ;
- 5-[1,9-diméthyl-3-(3-méthylphényl)-2,9-dihydro-1*H*-pyrido[2,3-b]indole-6-yl]-1,2,4-oxadiazol-3-amine ;
- 3-[4-(Aminométhyl)phényl]-6-(3-amino-1,2,4-oxadiazol-5-yl)-1,9-diméthyl-1,9-dihydro-2*H*-pyrido[2,3-b] indol-2-one ;
- 5-[1,9-diméthyl-3-[4-(morpholin-4-ylméthyl)phényl]-2,9-dihydro-1*H*-pyrido[2,3-b]indole-6-yl]-1,2,4-oxadiazol-3-amine ;
- 5-[3-(2,4-Dichlorophényl)-1,9-diméthyl-2,9-dihydro-1*H*-pyrido[2,3-b]indole-6-yl]-1,3,4-oxadiazol-2-amine;
- N'-[3-[3-(2,4-Dichlorophényl)-1,9-diméthyl-2,9-dihydro-1*H*-pyrido[2,3-b]indole-6-yl]-1,2,4-oxadiazol-5-yl]-N,N-diméthyléthane-1,2-diamine ;

à l'état de base ou de sel d'addition à un acide, ainsi qu'à l'état d'hydrate ou de solvat.

Dans ce qui suit, on entend par groupe protecteur Gp ou G'p un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective Groups in Organic Synthesis », Green et al., 2nd Edition (John Wiley & Sons; Inc., New York, 1991).

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc Des exemples de groupes partants ainsi que des références pour leur préparation sont donnés dans « Advanced Organic Chemistry », J. March, 3rd Edition, Wiley Interscience, p. 310-316, 1985.

Conformément à l'invention, on peut préparer les composés de formule générale (I) selon les procédés qui suivent.

Conformément à l'invention, on peut préparer les composés de formule (I) dans laquelle $R_4$ représente un

groupe $-CR_{11}=N-O-R_{12}$ selon un procédé qui est caractérisé en ce que : on fait réagir un composé de formule :

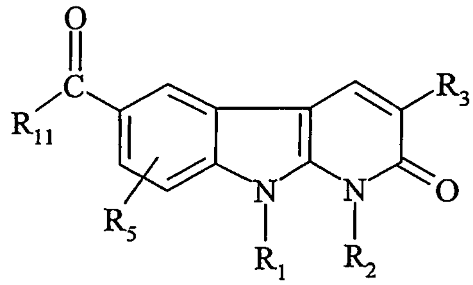

(II)

dans laquelle $R_1$, $R_2$, $R_3$, $R_5$ et $R_{11}$ sont tels que définis pour un composé de formule (I), avec un dérivé d'hydroxylamine de formule :

$H_2N-O-R_{12}$ (III)

dans laquelle $R_{12}$ est tel que défini pour un composé de formule (I).

Lorsque $R_{11}$ = H, la réaction s'effectue dans un solvant polaire tel qu'un alcool comme le méthanol ou l'éthanol à une température comprise entre la température ambiante et la température de reflux du solvant.

Lorsque $R_{11}$ = $(C_1-C_4)$alkyle, la réaction s'effectue dans un solvant polaire tel que l'éthanol, en présence d'une base telle qu'un carbonate d'un métal alcalin, le carbonate de potassium par exemple, à une température comprise entre la température ambiante et la température de reflux du solvant.

Selon une variante de ce procédé, à condition que $R_1$ et/ou $R_2 \neq$ H, on peut préparer un composé de formule (I) dans laquelle $R_4$ représente un groupe $-CR_{11} = N-O-R_{12}$ dans lequel $R_{12}$ représente un groupe $(C_1-C_4)$alkyle ou un groupe $-(CH_2)_mCOR_{16}$ par réaction d'un composé de formule (I) dans laquelle $R_4$ représente un groupe $-CR_{11} = N-OH$ avec un dérivé halogéné de formule $Hal-R_{12}$ dans laquelle Hal représente un atome d'halogène, de préférence le chlore ou le brome, en présence d'une base telle qu'un hydrure de métal alcalin, comme l'hydrure de sodium par exemple, dans un solvant tel que le N,N-diméthylformamide et à une température comprise entre la température ambiante et la température de reflux du solvant.

Conformément à l'invention, on peut préparer les composés de formule (I) dans laquelle

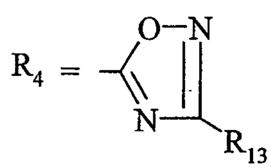

selon un procédé qui est caractérisé en ce que : on fait réagir un composé de formule :

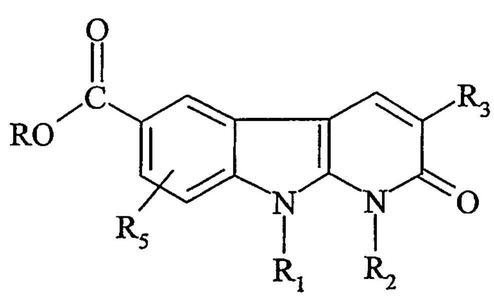

(IV)

dans laquelle $R_1$, $R_2$, $R_3$ et $R_5$ sont tels que définis pour un composé de formule (I) et R représente un atome d'hydrogène ou un groupe $(C_1-C_4)$alkyle, avec un dérivé d'oxime de formule :

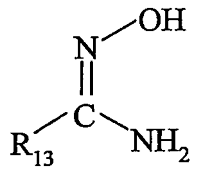

(V)

dans laquelle $R_{13}$ est tel que défini pour un composé de formule (I).

Lorsque dans le composé de formule (IV) R représente un atome l'hydrogène la réaction s'effectue en présence d'un agent de couplage tel que le dicyclohexylcarbodiimide, le carbonyldiimidazole, l'hexafluorophosphate de benzotriazol-1-yloxytris(diméthylamino)phosphonium ou l'hexafluorophosphate de benzotriazol-1-yloxytripyrrolidinophosphonium, dans un solvant tel que le dichlorométhane ou le N,N-diméthylformamide et à une température comprise entre la température ambiante et la température de reflux du solvant.

Lorsque dans le composé de formule (IV) R représente un groupe ($C_1$-$C_4$)alkyle, la réaction s'effectue en présence d'une base telle que l'hydrure de sodium ou l'éthylate de sodium, en présence d'un agent désséchant tel que des zéolithes, dans un solvant tel que le dioxane, le tétrahydrofurane ou l'éthanol et à une température comprise entre la température ambiante et la température de reflux du solvant.

Selon une variante de ce procédé, à condition que $R_1$ et/ou $R_2 \neq$ H, on peut préparer un composé de formule (I) dans laquelle

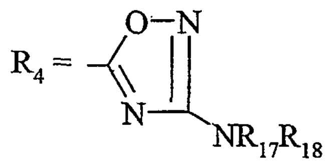

dans lequel $R_{17}$ et/ou $R_{18}$ représentent un ($C_1$-$C_4$)alkyle par réaction d'un composé de formule (I) dans laquelle

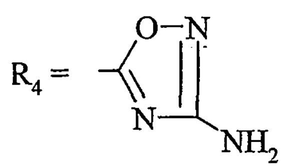

avec un halogénure de ($C_1$-$C_4$)alkyle, en présence d'une base telle que l'hydrure de sodium, dans un solvant tel que le N,N-diméthylformamide et à une température comprise entre la température ambiante et la température de reflux du solvant. De même, par réaction avec un composé de formule $R_{21}COHal$ ou $R_{22}SO_2Hal$ dans laquelle Hal représente un atome d'halogène, en présence d'une base telle que la pyridine ou la triéthylamine, dans un solvant tel que le dichlorométhane ou le N,N-diméthylformamide, à une température comprise entre la température ambiante et la température de reflux du solvant, on prépare les composés de formule (I) dans laquelle

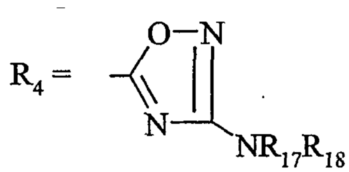

dans lequel $R_{18}$ = $-COR_{21}$ ou $-SO_2R_{22}$.

De façon particulière on peut également préparer un composé de formule (I) dans laquelle

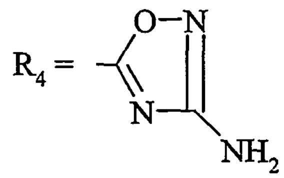

en suivant les différentes étapes du procédé décrit dans le SCHEMA I ci-après.

SCHEMA I

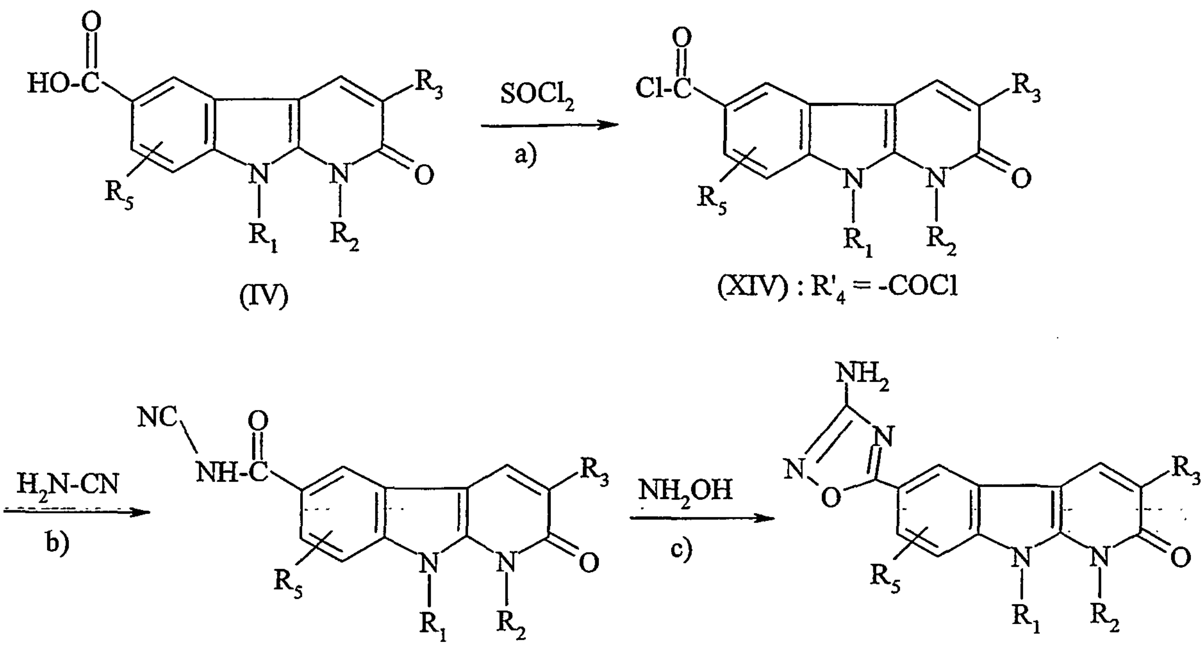


(IV)

(XIV) : $R'_4$ = -COCl

(XIV) : $R'_4$ = -CONH-CN

(I)

A l'étape a) du SCHEMA I, on fait réagir un acide de formule (IV) avec le chlorure de thionyle dans un solvant tel que le tétrahydrofurane à une température comprise entre 0˚C et la température ambiante pour obtenir le chlorure d'acide correspondant.

A l'étape b) la réaction du chlorure d'acide avec le cyanamide sans solvant ou dans un solvant tel que le THF à une température comprise entre la température ambiante et la température de reflux du solvant ou la température de fusion du cyanamide permet d'obtenir le dérivé N-cyanocarboxamide correspondant qui par réaction, à l'étape c) avec l'hydroxylamine dans un solvant tel que la pyridine, à une température comprise entre la température ambiante et 120˚C permet d'obtenir le composé de formule (I) attendu.

Conformément à l'invention, on peut préparer les composés de formule (I) dans laquelle

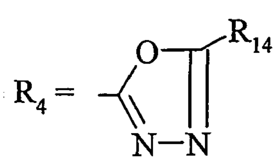

selon un procédé qui est caractérisé en ce que :
on cyclise un composé de formule :

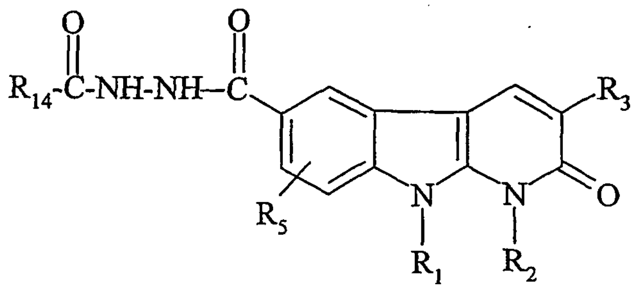

(IX)

dans laquelle $R_1$, $R_2$, $R_3$, $R_5$ et $R_{14}$ sont tels que définis pour un composé de formule (I).

La réaction de cyclisation s'effectue de façon générale en présence d'une quantité catalytique d'un acide tel que l'acide toluène-4-sulfonique, dans un solvant tel que le toluène, à une température comprise entre la température ambiante et la température de reflux du solvant et en éliminant l'eau formée par azéotropie.

Selon une variante du procédé, on effectue la cyclisation par réaction du composé de formule (IX) avec le chlorure de toluène-4-sulfonyle, en présence d'une base telle que la triéthylamine, dans un solvant tel que le dichlorométhane et à une température comprise entre la température ambiante et la température de reflux du solvant.

Selon une autre variante du procédé on effectue la cyclisation en présence d'oxychlorure de phosphore selon le procédé décrit dans J. Qrg. Chem. USSR, 1989, 25 (5), 935-940.

De façon particulière on peut préparer un composé de formule (I) dans laquelle

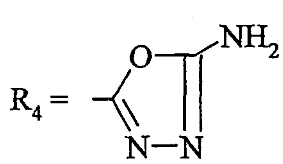

par réaction d'un composé de formule :

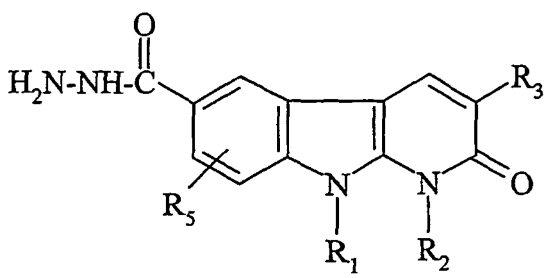

(X)

dans laquelle $R_1$, $R_2$, $R_3$ et $R_5$ sont tels que définis pour un composé de formule (I), avec du bromure de cyanogène dans un solvant tel que le méthanol, à une température comprise entre la température ambiante et la température de reflux du solvant, suivi d'une hydrolyse en milieu basique.

De façon particulière aussi, on peut préparer un composé de formule (I) dans laquelle

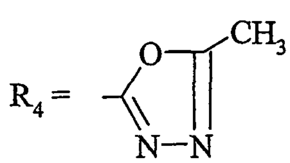

par réaction d'un composé de formule (X) avec
l'orthoacétate de triéthyle, en présence d'une quantité catalytique d'un acide tel que l'acide toluène-4-sulfonique, à une température comprise entre la température ambiante et 150°C.

Selon une variante du procédé, à condition que $R_1$ et/ou $R_2 \neq H$, on peut préparer un composé de formule (I) dans laquelle

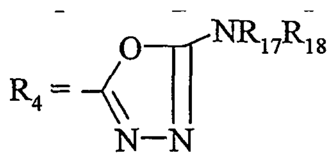

dans lequel $R_{17}$ et/ou $R_{18}$ représentent un $(C_1\text{-}C_4)$alkyle, ou $R_{18}$ peut de plus représenter $-COR_{21}$ ou $-SO_2R_{22}$ à partir d'un composé de formule (I) dans laquelle

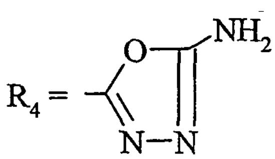

selon les méthodes précédemment décrites.

Conformément à l'invention, on peut préparer les composés de formule (I) dans laquelle

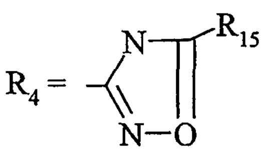

selon un procédé qui est caractérisé en ce que :
on fait réagir un composé de formule :

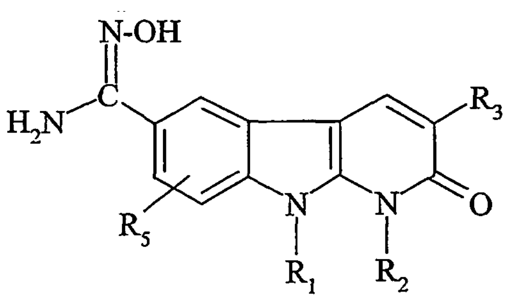

(XI)

dans laquelle $R_1$, $R_2$, $R_3$ et $R_5$ sont tels que définis pour un composé de formule (I), avec :

a) -soit le chlorure de trichloroacétyle, en présence d'une base, pour obtenir un composé de formule :

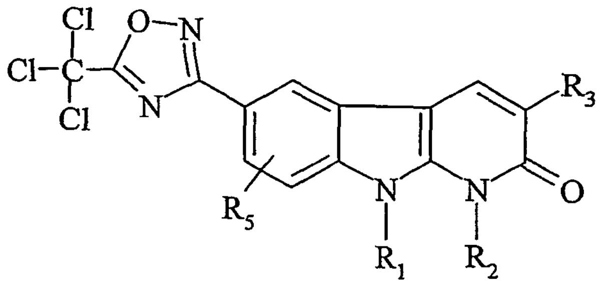

(XII)

et on fait réagir le composé de formule (XII) ainsi obtenu avec une amine de formule $HNR_{19}R_{20}$, lorsqu'on doit préparer un composé de formule (I) dans laquelle $R_{15} = NR_{19}R_{20}$ ;
b) - soit un anhydride de formule $(R_{15}CO)_2O$, lorsqu'on doit préparer un composé de formule (I) dans laquelle $R_{15}$ = $(C_1\text{-}C_4)$alkyle ;
c) - soit un dérivé de l'acide oxalique de formule

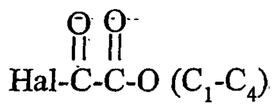

alkyle dans laquelle Hal représente un atome d'halogène, lorsqu'on doit préparer un composé de formule (I) dans laquelle $R_{15}$ = COO$(C_1\text{-}C_4)$alkyle.

A l'étape a), la réaction s'effectue en présence d'une base telle que la pyridine ou la N-méthylpyrrolidin-2-one, dans un solvant tel que le dioxane et à une température comprise entre la température ambiante et la température de reflux du solvant. Puis la réaction du composé (XII) avec l'amine s'effectue dans un solvant tel que le tétrahydrofurane ou le N,N-diméthylformamide et à une température comprise entre - 78°C et la température ambiante.

A l'étape b) la réaction s'effectue en présence d'un acide tel que l'acide acétique à une température comprise entre la température ambiante et la température de reflux du mélange réactionnel.

A l'étape c) la réaction s'effectue en présence d'une base telle que la pyridine, dans un solvant tel que le 1,2-dichloroéthane et à une température comprise entre 0°C et la température de reflux du solvant.

Un composé de formule (I) dans laquelle $R_1$ et/ou $R_2$ représentent un groupe $(C_1\text{-}C_4)$alkyle peut également être préparé par réaction d'un composé de formule (I) dans laquelle $R_1$ et/ou $R_2$ représentent un atome d'hydrogène avec un halogénure de $(C_1\text{-}C_4)$alkyle, en présence d'une base telle que l'hydrure de sodium, dans un solvant tel que le N,N-diméthylformamide et à une température comprise entre la température ambiante et la température de reflux du solvant.

Un composé de formule (I) dans laquelle $R_1$ représente un groupe $-(CH_2)_mOH$ peut également être préparé par réaction d'un composé de formule (I) dans laquelle $R_1$ = H avec un composé de formule $X\text{-}(CH_2)_mO\text{-}Gp$ dans laquellle X est un groupe partant tel que défini précédemment et Gp est un groupe O-protecteur, en présence d'une base telle que l'hydrure de sodium, dans un solvant tel que le N,N-diméthylformamide et à une température comprise entre 0°C et la température de reflux du solvant puis traiter le composé ainsi obtenu pour enlever le groupe O-protecteur selon des méthodes connues.

Un composé de formule (I) dans laquelle $R_1$ représente un groupe $-(CH_2)_mCN$ peut également être préparé par réaction d'un composé de formule (I) $Hal\text{-}(CH_2)_mCN$ dans laquelle Hal représente un atome d'halogène, en présence d'une base telle qu'un carbonate de métal alcalin comme le carbonate de potassium, dans un solvant tel que le N,N-diméthylformamide et à une température comprise entre la température ambiante et la température de reflux du solvant.

Un composé de formule (I) dans laquelle $R_1$ représente un groupe $-(CH_2)_mNR_9R_{10}$ peut également être préparé par réaction d'un composé de formule (I) dans laquelle $R_1$ = H avec un composé de formule $X\text{-}(CH_2)_mNR_9R_{10}$ dans laquelle X représente un groupe partant tel que défini précédemment, en présence d'une base telle qu'un carbonate de métal alcalin comme le carbonate de potassium, dans un solvant tel que le N,N-diméthylfonnalnide et à une température comprise entre 0°C et la température de reflux du solvant.

Les composés de formule (I) ainsi obtenus peuvent être ultérieurement séparés du milieu réactionnel et purifiés selon les méthodes classiques, par exemple par cristallisation ou chromatographie.

Les composés de formule (I) ainsi obtenus sont isolés sous forme de base libre ou de sel, selon les techniques classiques.

Les composés de formule (II) et (IV) peuvent être préparés selon la méthode décrite dans le brevet français n° 97 08409 ou dans la demande internationale WO 2004/041817.

Selon cette méthode, on fait réagir un 2-aminoindole de formule :

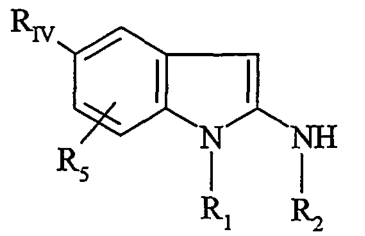

(VI)

dans laquelle $R_1$, $R_2$ et $R_5$ sont tels que définis pour un composé de formule (I) et $R_{IV}$ représente un groupe $-COR_{11}$ ou $(C_1-C_4)$alcoxycarbonyle, avec un ester de formule :

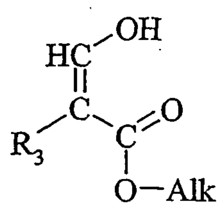

(VII)

dans laquelle $R_3$ est tel que défini pour un composé de formule (I), et Alk représente un alkyle en $C_1$-$C_4$.

La réaction est effectuée dans un solvant polaire et de préférence basique, par exemple dans la pyridine, à une température comprise entre la température ambiante et la température de reflux du solvant.

On peut également préparer un composé de formule (II) ou (IV) par un autre procédé en faisant réagir un 2-aminoindole de formule :

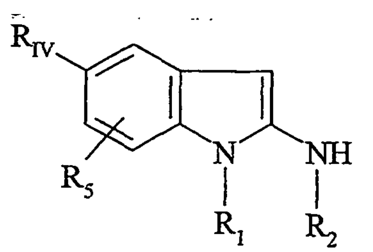

(VI)

dans laquelle $R_1$, $R_2$, $R_{IV}$ et $R_5$ sont tels que définis ci-dessus avec un ester de formule :

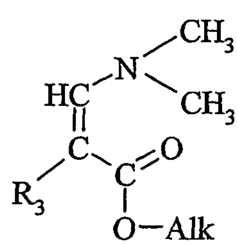

(VIII)

dans laquelle $R_3$ est tel que défini pour un composé de formule (I) et Alk représente un alkyle en $C_1$-$C_4$.

La réaction est effectuée dans un solvant protique et polaire, de préférence en milieu acide, à une température comprise entre la température ambiante et la température de reflux du solvant.

La préparation du composé de formule (VIII) est effectuée au moyen du diméthoxy-N,N-diméthylméthanamine d'après une méthode similaire à celle décrite dans J. Org. Chem., 1982, 47, 2846-2851 ou au moyen du réactif de Bredereck (*tert*butoxybis(diméthylamino)méthane) selon Liebigs Ann. Chem., 1980, 3, 344-357.

D'une manière générale, on peut également préparer un composé de formule :

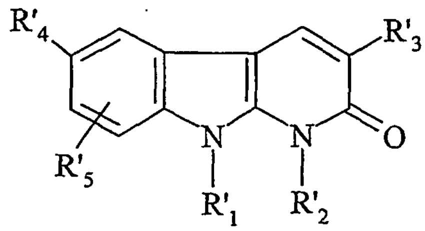

(XIV)

dans laquelle les substituants $R'_1$, $R'_2$, $R'_3$, $R'_4$, $R'_5$ sont des précurseurs des substituants $R_1$, $R_2$, $R_3$, $R_4$, $R_5$ tels que définis pour un composé de formule (I), puis, en utilisant des méthodes connues de l'homme de l'art, transformer ces substituants pour obtenir les substituants $R_1$, $R_2$, $R_3$, $R_4$, $R_5$ désirés pour le composé de formule (I).

Les composés de formule (XIV) dans laquelle $R'_4$ est un groupe cyano peuvent être transformés en composés de formule (II) dans laquelle $R_{11}$ = H par action du Nickel de Raney en présence d'hypophosphite de sodium.

Un composé de formule (II) dans laquelle $R_1$ représente un $(C_1-C_4)$alkyle peut également être préparé par réaction d'un composé de formule (XIV) dans laquelle $R'_4$ = H avec un composé de formule $R_{11}COCl$, en présence de $AlCl_3$, dans un solvant tel que le dichlorométhane et à une température comprise entre 0°C et la température ambiante.

Les composés de formule (III) sont connus ou se préparent selon des méthodes connues.

Les composés de formule (V) sont connus ou se préparent selon des méthodes connues.

Les composés de formule (VII) sont connus ou se préparent selon les méthodes décrites dans WO 2004/041817.

Les composés de formule (IX) se préparent par réaction d'un composé de formule:

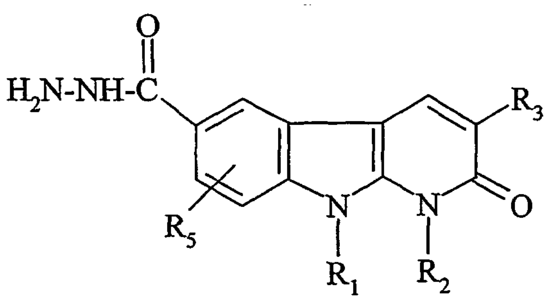

(X)

dans laquelle $R_1$, $R_2$, $R_3$ et $R_5$ sont tels que définis pour un composé de formule (I), avec un acide ou un dérivé fonctionnel de cet acide de formule :

$HOOC-R_{14}$

dans laquelle $R_{14}$ est tel que défini pour un composé de formule (I), selon les méthodes classiques d'acylation.

Les composés de formule (X) se préparent à partir des composés de formule :

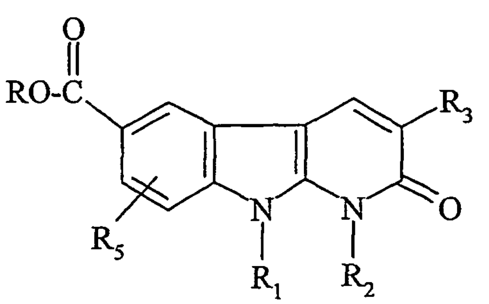

(IV)

dans laquelle $R_1$, $R_2$, $R_3$ et $R_5$ sont tels que définis pour un composé de formule (I) et R représente un atome d'hydrogène ou un $(C_1-C_4)$alkyle.

Lorsque R = H, on fait réagir l'acide de formule (TV) lui-même ou un dérivé fonctionnel de cet acide avec un

composé de formule :

$H_2N-NH-Pg$ (XIII)

dans laquelle Pg représente un groupe N-protecteur tel que le *tert*-butyloxycarbonyle, selon les méthodes classiques du couplage peptidique et le composé intermédiaire obtenu est déprotégé selon les méthodes classiques.

Lorsque R = ($C_1$-$C_4$)alkyle, on fait réagir l'ester de formule (IV) avec l'hydrazine, dans un solvant tel que l'éthanol et à une température comprise entre la température ambiante et la température de reflux du solvant.

Les composés de formule (XI) se préparent par réaction d'un composé de formule:

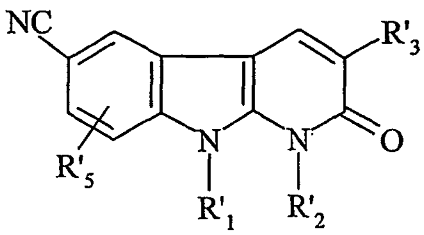

(XIV) : $R'_4$ = CN

dans laquelle $R'_1$, $R'_2$, $R'_3$ et $R'_5$ sont tels que définis précédemment avec l'hydroxylamine, en présence d'une base telle que le triéthylamine, dans un solvant tel que l'éthanol et à une température comprise entre la température ambiante et la température de reflux du solvant.

Les composés de formule (XIV : $R'_4$ = CN) sont connus et se préparent selon les méthodes décrites dans WO 2004/041817.

L'invention, selon un autre de ses aspects, a également pour objet les composés de formule (IX), (X), (XI) et (XII). Ces composés sont utiles comme intermédiaires de synthèse des composés de formule (I).

Ainsi, l'invention a pour objet des composés de formule :

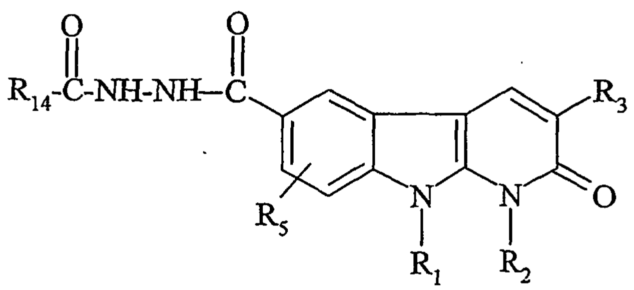

(IX)

dans laquelle :

- $R_1$ représente un atome d'hydrogène ; un groupe ($C_1$-$C_4$)alkyle ; un groupe $-(CH_2)_mOH$ ; un groupe $-(CH_2)_mCN$ ; un groupe $-(CH_2)_mNR_9R_{10}$ ;
- $R_2$ représente un atome d'hydrogène ou un groupe ($C_1$-$C_4$)alkyle ;
- $R_3$ représente un phényle substitué par $R_6$, $R_7$, $R_8$ ;
- $R_5$ représente un atome d'hydrogène ou un groupe ($C_1$-$C_4$)alkyle ;
- $R_6$, $R_7$ et $R_8$ représentent chacun indépendamment l'un de l'autre un atome d'hydrogène ; un atome d'halogène ; un groupe ($C_1$-$C_4$)alkyle ; un groupe ($C_1$-$C_4$)alcoxy ; un hydroxy ; un cyano ; un groupe $-(CH_2)_nNR_9R_{10}$ ; un groupe $-O-(CH_2)_mNR_9R_{10}$ ;
- $R_9$ et $R_{10}$ représentent chacun indépendamment l'un de l'autre un atome d'hydrogène ou un groupe ($C_1$-$C_4$)alkyle ;
- ou bien $R_9$ et $R_{10}$ ensemble avec l'atome d'azote auquel ils sont liés constituent un radical hétérocyclique choisi parmi la pyrrolidin-1-yle, la pipéridin-1-yle, la morpholin-4-yle ou la pipérazin-1-yle non substitué ou substitué en position -4- par un ($C_1$-$C_4$)alkyle ;
- $R_{14}$ représente un atome d'hydrogène ; un groupe ($C_1$-$C_4$)alkyle ; un groupe $-NR_{17}R_{18}$
- $R_{17}$ et $R_{18}$ représentent chacun indépendamment un atome d'hydrogène ou un groupe ($C_1$-$C_4$)alkyle ; $R_{18}$ peut de plus représenter un groupe $-COR_{21}$ ; un groupe $-SO_2R_{22}$ ;
- $R_{21}$ représente un groupe ($C_1$-$C_4$)alkyle ; un groupe ($C_3$-$C_6$)cycloalkyle ; un groupe $-(CH_2)_mNR_9R_{10}$ ;

- $R_{22}$ représente un groupe $(C_1-C_4)$alkyle ;
- m est 1, 2 ou 3 ;
- n est 0, 1, 2 ou 3.

L'invention a aussi pour objet des composés de formule :

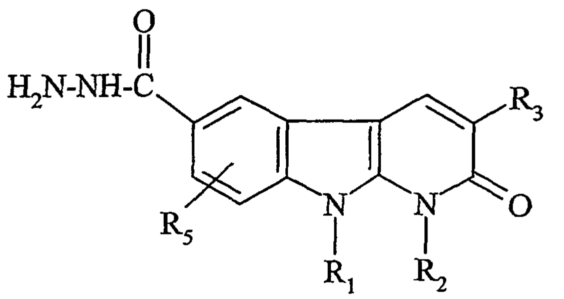

(X)

dans laquelle :

- $R_1$ représente un atome d'hydrogène ; un groupe $(C_1-C_4)$alkyle ; un groupe $-(CH_2)_mOH$ ; un groupe $-(CH_2)_mCN$ ; un groupe $-(CH_2)_mNR_9R_{10}$;
- $R_2$ représente un atome d'hydrogène ou un groupe $(C_1-C_4)$alkyle ;
- $R_3$ représente un phényle substitué par $R_6$, $R_7$, $R_8$;
- $R_5$ représenté un atome d'hydrogène ou un groupe $(C_1-C_4)$alkyle ;
- $R_6$, $R_7$ et $R_8$ représentent chacun indépendamment l'un de l'autre un atome d'hydrogène ; un atome d'halogène ; un groupe $(C_1-C_4)$alkyle ; un groupe $(C_1-C_4)$alcoxy ; un hydroxy ; un cyano ; un groupe $-(CH_2)_nNR_9R_{10}$ ; un groupe $-O-(CH_2)_mNR_9R_{10}$;
- $R_9$ et $R_{10}$ représentent chacun indépendamment l'un de l'autre un atome d'hydrogène ou un groupe $(C_1-C_4)$alkyle ;
- ou bien $R_9$ et $R_{10}$ ensemble avec l'atome d'azote auquel ils sont liés constituent un radical hétérocyclique choisi parmi la pyrrolidin-1-yle, la pipéridin-1-yle, la morpholin-4-yle ou la pipérazin-1-yle non substitué ou substitué en position -4- par un $(C_1-C_4)$alkyle;
- m est 1, 2 ou 3 ;
- n est 0, 1, 2 ou 3.

L'invention a aussi pour objet des composés de formule :

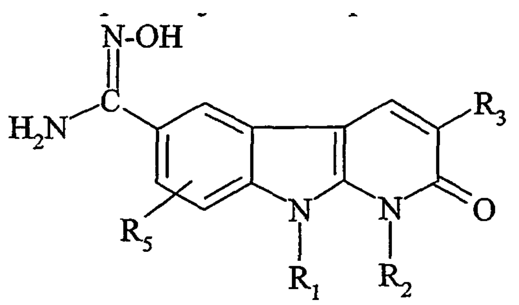

(XI)

dans laquelle :

- $R_1$ représente un atome d'hydrogène ; un groupe $(C_1-C_4)$alkyle ; un groupe $-(CH_2)_mOH$ ; un groupe $-(CH_2)_mCN$ ; un groupe $-(CH_2)_mNR_9R_{10}$;
- $R_2$ représente un atome d'hydrogène ou un groupe $(C_1-C_4)$alkyle ;
- $R_3$ représente un phényle substitué par $R_6$, $R_7$, $R_8$ ;
- $R_5$ représente un atome d'hydrogène ou un groupe $(C_1-C_4)$alkyle ;
- $R_6$, $R_7$ et $R_8$ représentent chacun indépendamment l'un de l'autre un atome d'hydrogène ; un atome d'halogène ; un groupe $(C_1-C_4)$alkyle ; un groupe $(C_1-C_4)$alcoxy ; un hydroxy ; un cyano ; un groupe $-(CH_2)_nNR_9R_{10}$ ; un groupe

$-O-(CH_2)_mNR_9R_{10}$;

- $R_9$ et $R_{10}$ représentent chacun indépendamment l'un de l'autre un atome d'hydrogène ou un groupe $(C_1-C_4)$alkyle ;
- ou bien $R_9$ et $R_{10}$ ensemble avec l'atome d'azote auquel ils sont liés constituent un radical hétérocyclique choisi parmi la pyrrolidin-1-yle, la pipéridin-1-yle, la morpholin-4-yle ou la pipérazin-1-yle non substitué ou substitué en position -4- par un $(C_1-C_4)$alkyle ;
- m est 1,2 ou 3;
- n est 0, 1, 2 ou 3.

L'invention a aussi pour objet des composés de formule :

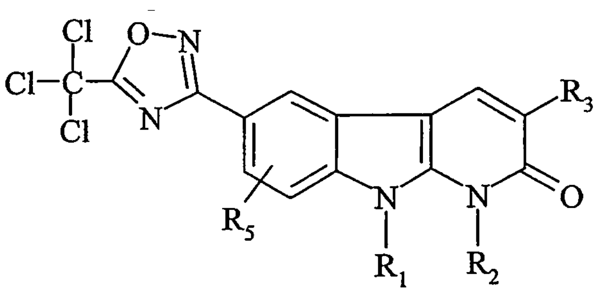

(XII)

dans laquelle :

- $R_1$ représente un atome d'hydrogène ; un groupe $(C_1-C_4)$akyle ; un groupe $-(CH_2)_mOH$ ; un groupe $-(CH_2)_mCN$ ; un groupe $-(CH_2)_mNR_9R_{10}$ ;
- $R_2$ représente un atome d'hydrogène ou un groupe $(C_1-C_4)$alkyle ;
- $R_3$ représente un phényle substitué par $R_6$, $R_7$, $R_8$ ;
- $R_5$ représente un atome d'hydrogène ou un groupe $(C_1-C_4)$alkyle ;
- $R_6$, $R_7$ et $R_8$ représentent chacun indépendamment l'un de l'autre un atome d'hydrogène ; un atome d'halogène ; un groupe $(C_1-C_4)$alkyle ; un groupe $(C_1-C_4)$alcoxy ; un hydroxy ; un cyano ; un groupe $-(CH_2)_nNR_9R_{10}$ ; un groupe $-O-(CH_2)_mNR_9R_{10}$;
- $R_9$ et $R_{10}$ représentent chacun indépendamment l'un de l'autre un atome d'hydrogène ou un groupe $(C_1-C_4)$alkyle ;
- ou bien $R_9$ et $R_{10}$ ensemble avec l'atome d'azote auquel ils sont liés constituent un radical hétérocyclique choisi parmi la pyrrolidin-1-yle, la pipéridin-1-yle, la morpholin-4-yle ou la pipérazin-1-yle non substitué ou substitué en position -4- par un $(C_1-C_4)$alkyle ;
- m est 1, 2 ou 3;
- n est 0, 1, 2 ou 3.

Sauf indication contraire, les spectres de résonance magnétique nucléaire ($RMN^1H$) du proton sont enregistrés dans DMSO-$d_6$, la référence est placée dans le DMSO-$d_6$ qui se situe à 2,50 ppm du tétraméthylsilane.

Les signaux observés en RMN sont exprimés ainsi : s: singulet ; se : singulet élargi ; d : doublet ; d.d : doublet dédoublé ; t : triplet ; td : triplet dédoublé ; q : quadruplet ; m : massif ; mt : multiplet.

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

Dans les préparations et exemples qui vont suivre, les abréviations suivantes sont utilisées :

DIPEA : diisopropyléthyamine
TEA : triéthylamine
DMA : diméthylacétamide
DMF : diméthylformamide
EP : éther de pétrole
DMFDMA : diméthylformamide diméthylacétal
NMP : N-Me pyrrolidin-2-one
LAH : hydrure de lithium et d'aluminium
THF : tétrahydrofurane
Ether : éther diéthylique

DCM : dichlorométhane
CDI : carbonyldiimidazole
AcOEt : acétate d'éthyle
AcOH : acide acétique
dppp : 1,3-tris(diphénylphosphino)propane
iPrOH : alcool isopropylique
réactif de Bredereck : *tert*butoxybis(diméthylamino)méthane
DDQ : 2,3-dichloro-5,6-dicyano-1,4-benzoquinone
PyBOP : (benzotriazol-1-yloxy)tripyrrolidinophosphoniumhexafluorophosphate
TA : température ambiante
F : point de fusion

**Préparation des composés de formule (VI).**

Les composés de formule (VI) peuvent exister sous 2 formes tautomères :

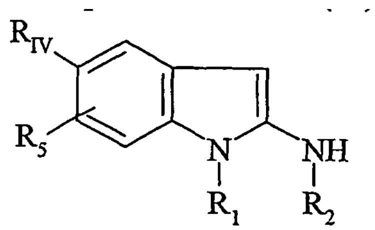

ou

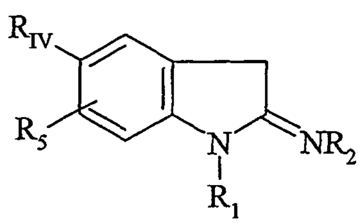

Préparation 1.1

N-Méthyl-5-bromo-1*H*-indole-2-amine, chlorhydrate

A) N'-(4-Bromophényl)-N-formylhydrazine.

On dissout 10 g de chlorohydrate de 4-bromophénylhydrazine dans 30 ml d'eau ; on ajoute 6,2 g de $K_2CO_3$ et 36 ml de formate de méthyle puis on chauffe à reflux pendant 1 heure puis à TA pendant 12 heures. Le précipité formé est filtré puis lavé par un mélange isopropanol/EP (50/50 ; v/v). On obtient 10,5 g du composé attendu.
RMN $CDCl_3$ (300 MHz) : 6,73-6,77 ppm : m : 2H ; 7,34-7,41 ppm : m : 2H ; 8,33 ppm : m : 1H.

B) N-Méthyl-N'-(4-bromophényl)acétohydrazine.

On chauffe à reflux une solution de 80 ml de LAH dans du THF, on ajoute 10,5 g du composé obtenu à l'étape A, en suspension dans 60 ml de THF puis on chauffe à reflux pendant 15 heures. On refroidit le milieu réactionnel puis on ajoute, goutte à goutte, 12 ml d'eau et 9 ml de NaOH 1N. On filtre sur Célite® pour éliminer l'insoluble puis on lave par AcOEt et évapore à sec le milieu réactionnel. On reprend par 80 ml d'AcOH et on ajoute 17 g de $K_2CO_3$ dans 80 ml d'eau puis 4 ml d'anhydride acétique et on laisse 1 heure sous agitation à TA. On décante puis on sèche la phase organique sur $MgSO_4$ et évapore à sec. On ajoute de l'éther de pétrole puis on filtre les cristaux formés et on obtient 9 g du composé attendu.
RMN $CDCl_3$ (300 MHz) : 2,15 ppm : s: 3H ; 3,13 ppm : s : 3H ; 6,57-6,62 ppm : m : 2H ; 7,32-7,40 ppm : m : 2H.

C) N-Méthyl-5-bromo-1*H*-indole-2-amine, chlorhydrate.

On dissout 9 g du composé de l'étape précédente dans 50 ml de $POCl_3$ et on chauffe à 80˚C pendant 2 heures. Après retour à TA, on ajoute de l'éther, le précipité formé est filtré puis lavé à l'éther. On obtient 8,2 g du composé attendu.
RMN $CDCl_3$ (300 MHz) : 4,31 ppm : s : 3H ; 7,14-7,87 ppm : m : 4H ; 10,70 ppm : m : 1H ; 12,62 ppm : s : 1H.

Préparation 1.2

1-Méthyl-2-(méthylamino)-1*H*-indole-5-carboxylate de méthyle.

A) 4-(2-Acétylhydrazino)benzoate de méthyle.

On dissout 5,5 g de 4-hydrazinobenzoate de méthyle dans 38,2 ml d'AcOH contenant 2,4 g d'acétate de sodium, on chauffe 18 heures à 80˚C. On essore le minéral puis on évapore et on reprend par un minimum d'$Et_2O$. On essore pour obtenir 7,97 g du composé attendu.

B) 4-(2-Acétyl-1,2-diméthylhydrazino)benzoate de méthyle.

On met en suspension 2,95 g de NaH à 95 % dans 90 ml de DMF et on ajoute goutte à goutte 8,135 g du composé de l'étape précédente en solution dans le minimum de DMF, puis après quelques minutes on ajoute goutte à goutte 9,75 ml d'iodure de méthyle. On agite 1 heure à TA. On verse le milieu sur une solution saturée de $NH_4Cl$ et on extrait par AcOEt. La phase organique est lavée par NaCl, séchée, évaporée pour donner 5,4 g du composé attendu.

C) 1-Méthyl-2-(méthylamino)-1*H*-indole-5-carboxylate de méthyle.

On mélange 5,4 g du composé de l'étape précédente et 62 ml d'oxychlorure de phosphore et on chauffe 2 heures et demie à 80˚C. On évapore le milieu et on reprend par AcOEt. Le solide formé est essoré, lavé par AcOEt, séché pour donner 4 g du composé attendu.
RMN MeOD (250 MHz) : 3,2 ppm : s : 3H ; 3,6 ppm : s : 3H ; 3,9 ppm : s : 3H ; 7,3-7,4 ppm : m : 2H ; 8,1-8,2 ppm : m : 2H.

Préparation 1.3

2-(Méthylamino)-1*H*-indole-5-carboxylate de méthyle.

A) 4-(2-Acétyl-2-méthylhydrazino)benzoate de méthyle.

On dissout 10 g du composé de la Préparation 1.2, étape A dans 60 ml de DMF anhydre et on refroidit à 0˚C. On ajoute par petites fractions 1,9 g de NaH à 60 % et on laisse sous agitation jusqu'à l'arrêt du dégagement gazeux. On ajoute 4,5 ml de $CH_3I$ et on agite 20 minutes à 0˚C. On verse le milieu réactionnel sur une solution saturée de $NH_4Cl$ puis on extrait par AcOEt. On lave la phase organique sur une solution saturée de NaCl, sèche sur $MgSO_4$ puis on purifie par chromatographie sur silice en éluant par AcOEt/cyclohexane (50/50 ; v/v) puis (75/25 ; v/v). On obtient 6,2 g du composé attendu.
RMN DMSO (300MHz) : 2,15 ppm : s : 3H ; 3,17 ppm : s : 3H ; 3,89 ppm : s : 3H ; 6,11 ppm : s : 1H ; 6,71 ppm : d : 2H ; 7,98 ppm : d : 2H.

B) 2-(Méthylamino)-1*H*-indole-5-carboxylate de méthyle.

On dissout 5,3 g du composé de l'étape précédente dans 30 ml de $POCl_3$ et on chauffe 2 heures à 80˚C. Après refroidissement du milieu réactionnel, on ajoute de l'éther et on agite. On filtre le précipité formé puis on le lave à l'éther pour obtenir 3,2 g du composé attendu.
RMN DMSO (300MHz) : 3,08 ppm : s : 3H ; 3,85 ppm : s : 3H ; 4,25 ppm : s : 2H ; 6,29 ppm : d : 1H ; 8 ppm : m : 2H ; 10,78 ppm : s : 1H ; 12,64 ppm : s : 1H.

Préparation 1.4

1-Méthyl-2-(méthylamino)-1*H*-indole-5-carbonitrile.

A) N'-(4-Cyanophényl)acétohydrazide.

A une solution de 5 g de chlorhydrate de 4-cyanophénylhydrazine dans 40 ml d'acide acétique on ajoute 2,7 g d'acétate de sodium puis chauffe à 80˚C pendant 20 heures. On concentre sous vide le mélange réactionnel, reprend le résidu à l'eau, extrait à l'AcOEt, lave la phase organique par une solution saturée de NaCl, sèche sur $MgSO_4$ et évapore le solvant sous vide. On triture le résidu dans un mélange EP/AcOEt (90/10 ; v/v) et essore le précipité formé. On obtient 4,8 g du composé attendu.
RMN$^1$H : $CDCl_3$ (300 MHz) : δ (ppm) : 1,92 : s : 3H ; 6,75 : d : 2H ; 7,53 : d : 2H ; 8,25 : s : 1H ; 9,76 : s : 1H.

B) N'-(4-Cyanophényl)-N,N-diméthylacétohydrazide.

A une suspension de 2,8 g de NaH à 60 % dans l'huile dans 50 ml de DMF on ajoute, en goutte à goutte et à TA, une solution de 4,8 g du composé obtenu à l'étape précédente dans 20 ml de DMF et laisse sous agitation jusqu'à la fin du dégagement gazeux. On ajoute ensuite 6,8 ml d'iodure de méthyle puis laisse sous agitation pendant 2 heures à TA. On verse le mélange réactionnel sur une solution saturée de $NH_4Cl$, extrait à l'AcOEt, lave la phase organique par une solution saturée de NaCl, sèche sur $MgSO_4$ et évapore le solvant sous vide. On reprend le résidu dans l'EP et essore le précipité formé. On obtient 4,5 g du composé attendu.
RMN$^1$H : $CDCl_3$ (300 MHz) : δ (ppm) : 2,04 : s : 3H ; 3,03 : s : 3H ; 3,21 : s : 3H ; 6,70 : d : 3H ; 7,57 : d : 2H.

C) 1-Méthyl-2-(méthylamino)-1*H*-indole-5-carbonitrile.

On chauffe à 75˚C pendant 1 heure 30 minutes une solution de 4,0 g du composé obtenu à l'étape précédente dans 30 ml de $POCl_3$. Après refroidissement à TA on essore le précipité formé et le lave à l'éther. On obtient 3,4 g du composé attendu.

Préparation 1.5

N,1-Diméthyl-1*H*-indol-2-amine.

On prépare ce composé selon les modes opératoires décrits dans WO 2004/041817, F = 249˚C.

**Préparations des composés de formule (VII) ou (VIII).**

**Préparation 2.1**

2-(3-Bromophényl)-3-hydroxy-2-propénoate d'éthyle (VII).

A) 3-Bromophénylacétate d'éthyle.

On dissout 5 g d'acide 3-bromophénylacétique dans 80 ml d'éthanol, on ajoute 3 ml d'$H_2SO_4$ concentré puis on chauffe à reflux pendant 2 heures. On évapore l'éthanol, neutralise par une solution saturée de $K_2CO_3$ puis on extrait par AcOEt et sèche sur $MgSO_4$. On obtient 5,2 g du composé attendu sous forme liquide.
RMN $CDCl_3$ (300 MHz) : 1,18 ppm : t : 3H ; 3,50 ppm : s : 2H ; 4,08 ppm : q : 2H ; 7,09-7,37 ppm : m : 4H.

B) 2-(3-Bromophényl)-3-hydroxy-2-propénoate d'éthyle.

On dissout 5,2 g du composé de l'étape précédente dans 70 ml de formiate d'éthyle et on ajoute par petites fractions 1,7 g de NaH à 60 %. On laisse sous agitation à TA pendant 5 heures. On verse sur 100 ml d'HCl 1N puis on extrait par AcOEt, sèche sur $MgSO_4$ puis évapore à sec. On obtient 5,8 g du composé attendu sous forme d'huile.
RMN $CDCl_3$ (300 MHz) : 1,9 ppm : t : 3H ; 4,20 ppm : q : 2H ; 7,11-7,42 ppm : m : 5H ; 12,06 ppm : d : 1H.

Préparation 2.2

2-(2,4-Diméthylphényl)-3-diméthylaminoacrylate d'éthyle (VIII).

A) 1-Bromométhyl-2,4-diméthylbenzène.

On dissout 5 g de (2,4-diméthylphényl)méthanol dans 100 ml d'éther. On refroidit le milieu réactionnel à 0°C. On ajoute, goutte à goutte, 5,2 ml de tribromure de phosphore. On agite à température ambiante pendant une nuit. On verse le milieu réactionnel sur de la glace et on extrait à l'AcOEt. On lave la phase organique avec une solution de NaCl saturée. On sèche la phase organique sur $MgSO_4$, filtre et évapore à sec. On obtient 7,3 g du composé attendu.

RMN $CDCl_3$ (300 MHz) : 2,28 ppm : s : 3H ; 2,35 ppm : s : 3H ; 4,48 ppm : s : 2H ; 6,90-7,22 ppm : m : 3H.

B) (2,4-Diméthylphényl)acétonitrile.

On dissout 7,3 g du composé de l'étape précédente dans 120 ml d'éthanol et 30 ml d'eau. On ajoute 4,7 g de cyanure de potassium. On chauffe à reflux pendant 5 heures. On évapore l'éthanol et reprend le résidu dans l'eau. On extrait à l'AcOEt puis on lave la phase organique avec une solution saturée de NaCl. On sèche la phase organique sur $MgSO_4$, on filtre et on évapore à sec. On obtient 5,3 g du composé attendu.

RMN $CDCl_3$ (300 MHz) : 2,37 ppm : s : 6H ; 3,68 ppm : s : 2H ; 7,00-7,32 ppm : m : 3H.

C) (2,4-Diméthylphényl)acétate d'éthyle.

On dissout 5,3 g du composé 2.2, étape B dans 120 ml d'éthanol et 7 ml d'$H_2SO_4$. On chauffe à reflux pendant 10 jours. On évapore l'éthanol et on reprend le résidu avec une solution de $K_2CO_3$ saturée. On extrait à l'AcOEt, on lave la phase organique avec une solution saturée de NaCl. On sèche la phase organique sur $MgSO_4$, on filtre et on évapore à sec. On obtient 6,0 g d'huile jaune.

RMN $CDCl_3$ (300 MHz) : 1,25 ppm : t : 3H ; 2,27 ppm : 2s : 6H ; 3,56 ppm : s : 2H ; 4,14 ppm : q : 2H ; 6,91-7,27 ppm : m : 3H.

D) 2-(2,4-Diméthylphényl)-3-diméthylaminoacrylate d'éthyle.

On dissout 6,0 g du composé de l'étape précédente dans 9 ml de réactif de Bredereck. On chauffe à 100°C pendant 15 heures. On évapore à sec. On purifie sur colonne de silice éluée avec un mélange AcOEt/cyclohexane (10/90 ; v/v). On obtient 6,7 g d'huile jaune.

RMN $CDCl_3$ (300 MHz) : 1,21 ppm : t : 3H ; 2,16 ppm : s : 3H ; 2,29 ppm : s : 3H ; 2,60 ppm : s : 6H ; 4,09 ppm : q : 2H ; 6,90-7,57 ppm : m : 4H.

En procédant selon les Préparations décrites ci-dessus, on obtient les intermédiaires de formule (VII) ou (VIII) rassemblés dans le tableau ci-après :

**TABLEAU 1**

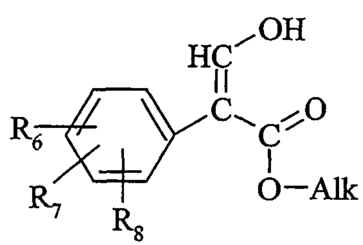 (VII) ou 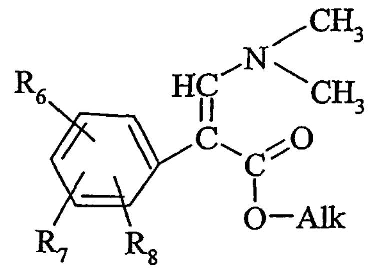 (VIII)

| Préparations | $R_6$, $R_7$, $R_8$ | Intermédiaire (VII) ou (VIII) | Caractérisation |
|---|---|---|---|
| 2.3 | 2,4-diCl | (VII):Alk=Et<br>(VIII):Alk=Me | RMN $CDCl_3$ (300 MHz) : 1,25 ppm : t : 3H ; 4,25 ppm : q : 2H ; 7,13-7,44 ppm : m : 4H ;<br>12,03 ppm : m : 1H. F = 73°C |
| 2.4 | 2,4-diOMe | (VIII):Alk=Me | RMN DMSO (300 MHz) : 2,62 ppm : s : 6H ; 3,44 ppm : s : 3H ; 3,69 ppm : s : 3H ; 3,75 ppm : s : 3H ; 6,40-6,48 ppm : m : 2H ; 6,87 ppm : d : 1H ; 7,41 ppm : s : 1H.. |
| 2.5 | H | (VII) : Alk = Me | Décrit dans J. Am. Chem. Soc., 1974, 96, 2127-2129 |
| 2.6 | 3-Br | (VIII) : Alk = Me | RMN DMSO (300 MHz) : 2,66 ppm : s : 6H ; 3,50 ppm : s : 3H ; 7,10-7,52 : m : 5H. |
| 2.7 | 2-Cl | (VII) : Alk = Et | RMN$^1$H : $CDCl_3$ (300 MHz) : δ (ppm) : 1,24 : t : 3H ; 4,25 : q : 2H ; 7,15-7,50 : m : 4H ; 12,0 : d : 1H. |
| 2.8 | 4-Cl | (VIII) : Alk = Me | RMN$^1$H : $CDCl_3$ (300 MHz) : δ (ppm) : 2,57 : s : 6H ; 3,51 : s : 3H ; 6,98-7,44 : m : 5H. |
| 2.9 | 3-Br | (VIII) : Alk = Et | |
| 2.10 | 4-Br | (VIII) : Alk = Et | F = 97°C |
| 2.11 | 3-F | (VIII) : Alk = Me | RMN$^1$H : $CDCl_3$ (300 MHz) : δ (ppm) : 2,66 : s : 6H ; 3,50 : s : 3H ; 6,90-7,31 : m : 4H ; 7,52: s: 1H. |
| 2.12 | 4-F | (VIII) : Alk = Et | |
| 2.13 | 3-Me | (VIII) : Alk = Me | RMN$^1$H : $CDCl_3$ (300 MHz) : δ (ppm) : 2,33 : s : 3H ; 2,66 : s : 6H ; 3,61 : s : 3H ; 7,04-7,25 : m : 4H ; 7,54 : s : 1H. |

(suite)

| Préparations | $R_6$, $R_7$, $R_8$ | Intermédiaire (VII) ou (VIII) | Caractérisation |
|---|---|---|---|
| 2.14 | 3-OMe | (VII) : Alk = Et | RMN[1]H : $CDCl_3$ (300 MHz) : δ (ppm) : 1,35 : t : 3H ; 3,85 : s : 3H ; 4,34 : q : 2H ; 6,7-7,0 : m : 2H ; 7,2-7,4 : m : 3H ; 12,18 : d : 1H. |
| 2.15 | 4-OMe | (VII) : Alk = Et | RMN[1]H : $CDCl_3$ (300 MHz) : δ (ppm) : 1,25 : t : 3H ; 3,86 : s : 3H ; 4,33 : q : 2H ; 6,92 : d : 2H ; 7,23 : d : 2H ; 7,28 : d : 1H ; 12,08 : d : 1H. |
| 2.16 | 3-CN | (VIII) : Alk = Et | |
| 2.17 | 4-CN | (VIII) : Alk = Et | |
| 2.18 | 3,5-diF | (VIII): Alk=Me | RMN[1]H : DMSO-$d_6$ (300 MHz) : δ (ppm) : 2,70 : s : 6H ; 3,52 : s : 3H ; 6,79-7,08 : m : 3H ; 7,53 : s : 1H. |
| 2.19 | 2-OMe | (VII) : Alk = Et | RMN[1]H: $CDCl_3$ (300 MHz) : δ (ppm) : 1,24 : t : 3H ; 3,78 : s : 3H ; 4,22 : q : 2H ; 6,85-7,00 : m : 2H ; 7,10-7,40 : m: 2H ;11,91 : d : 1H. |

**Préparation 2.20**

3-(Diméthylamino)-2-[4-(2-morpholin-4-yléthoxy)phényl]acrylate d'éthyle.

A) [4-(2-Morpholin-4-yléthoxy)phényl]acétate d'éthyle.

A une solution de 1 g de (4-hydroxyphényl)acétate d'éthyle dans 20 ml d'EtOH on ajoute 2,5 g de $K_2CO_3$, 1,68 g de chlorhydrate de 4-(2-chloroéthyl)morpholine et 0,01 g d'iodure de tétrabutylammonium puis chauffe à reflux pendant 5 heures. Après refroidissement à TA, on filtre le $K_2CO_3$, le lave à l'EtOH et concentre sous vide le filtrat. On reprend le résidu dans l'eau, extrait à l'AcOEt, lave la phase organique à l'eau, sèche sur $MgSO_4$ et évapore le solvant sous vide. On obtient 1,5 g du composé attendu sous forme d'huile incolore.
RMN$^1$H : DMSO-$d_6$ (300 MHz) : $\delta$ (ppm) : 1,17 : t : 3H ; 2,44 : m : 4H ; 2,67 : t : 2H ; 3,53-3,59 : m : 6H ; 4,02-4,09 : m : 4H ; 6,88 : d : 2H ; 7,15 : d : 2H.

B) 3-(Diméthylamino)-2-[4-(2-morpholin-4-yléthoxy)phényl]acrylate d'éthyle.

On chauffe à 100°C pendant une nuit un mélange de 1,5 g du composé obtenu à l'étape précédente dans 1 ml de réactif de Bredereck. Après refroidissement à TA, on ajoute de l'EtOH et concentre sous vide. On obtient 1,9 g du composé attendu sous forme d'huile orange.

**Préparations des composés de formule (XIV), (X) et (XI).**

Préparation 3.1

3-(2,4-Diméthoxyphényl)-1-méthyl-2-oxo-2,9-dihydro-1*H*-pyrido[2,3-b]indol-6-carbaldéhyde.

A) 6-Bromo-3-(2,4-diméthoxyphényl)-1-méthyl-1,9-dihydropyrido[2,3-b]indol-2-one.

On dissout 4,0 g de composé de la Préparation 1.1 dans 30 ml d'acide acétique. On chauffe à 100°C pendant 15 minutes puis on rajoute 3,6 g du composé de la Préparation 2.4. On chauffe à reflux pendant 2 heures 30 minutes. On refroidit le milieu réactionnel puis on filtre le précipité formé. On obtient 3,3 g du composé attendu.
RMN DMSO (300 MHz) : 3,58 ppm : s : 3H ; 3,65 ppm : s : 3H ; 3,80 ppm : s : 3H ; 6,53 ppm : dd : 1H ; 6,60 ppm : s : 1H ; 7,10 ppm : d : 1H ; 7,29 ppm : d : 1H ; 7,43 ppm : d : 1H ; 8,04 ppm : s : 2H ; 12,21 ppm : s : 1H.

B) 3-(2,4-Diméthoxyphényl)-1-méthyl-2-oxo-2,9-dihydro-1*H*-pyrido[2,3-b]indol-6-carbonitrile.

On dissout 3,0 g du composé de l'étape précédente dans 70 ml de NMP. On rajoute 1,3 g de CuCN puis on chauffe à 200°C pendant 24 heures. On verse le milieu réactionnel sur de l'eau, on extrait le produit à l'AcOEt. On purifie par chromatographie sur colonne de silice en éluant à l'AcOEt pur puis par AcOEt/MeOH (98/2 ; v/v) On obtient 0,82 g de poudre beige.
RMN DMSO (300 MHz) : 3,66 ppm : s : 3H ; 3,70 ppm : s : 3H ; 3,80 ppm : s : 3H ; 6,55 ppm : dd : 1H ; 6,62 ppm : d : 1H ; 7,13 ppm : d : 1H ; 7,55-7,62 ppm : m : 2H ; 8,10 ppm : s : 1H ; 8,37 ppm : s : 1H ; 12,53 ppm : s : 1H.

C) 3-(2,4-Diméthoxyphényl)-1-méthyl-2-oxo-2,9-dihydro-1*H*-pyrido[2,3-b]indol-6-carbaldéhyde.

On dissout 0,4 g du composé de l'étape précédente dans 30 ml de pyridine, 10 ml d'acide acétique et 6 ml d'eau. On ajoute 2,6 g de $NaH_2PO_2$ et 2,0 g de Nickel de Raney. On chauffe à 65°C pendant 40 heures. On filtre le catalyseur à chaud sur Célite, on lave avec du méthanol et on évapore à sec, on reprend le résidu dans de l'AcOEt puis on lave avec une solution de NaCl saturée. On sèche sur $MgSO_4$ puis on purifie par chromatographie sur colonne de silice en éluant par de l'AcOEt puis AcOEt/MeOH (98/2 ; v/v). On obtient 160 mg du composé attendu.
RMN DMSO (300 MHz) : 3,67 ppm : s : 3H ; 3,70 ppm : s : 3H ; 3,80 ppm : s : 3H ; 6,55 ppm : d : 1H ; 6,62 ppm : s : 1H ; 7,16 ppm : d : 1H ; 7,61 ppm : d : 1H ; 7,75 ppm : d : 1H ; 8,13 ppm : s : 1H ; 8,43 ppm : s : 1H ; 10,00 ppm : s : 1H ; 12,47 ppm : s : 1H.

Préparation 3.2

3-(2,4-Diméthoxyphényl)-1,9-diméthyl-2-oxo-2,9-dihydro-1*H*-pyrido[2,3-b] indole-6-carboxylate d'éthyle.

A) Acide 3-(2,4-diméthoxyphényl)-1-méthyl-2-oxo-2,9-dihydro-1*H*-pyrido[2,3-b] indole-6-carboxylique.

On dissout 5,0 g de KOH dans 15 ml d'$H_2O$ puis on ajoute 2,2 g du composé de la Préparation 3.1, étape B et 1,1 ml d'une solution $H_2O_2$ à 35 % dans l'eau. On chauffe à 130°C pendant 2 jours. On refroidit le milieu réactionnel puis on ajoute de l'eau. On extrait à l'AcOEt puis on acidifie la phase aqueuse avec une solution de HCl 1N. On filtre le précipité formé, on le lave avec de l'eau puis on le reprend avec un mélange AcOEt/MeOH 50/50. On sèche sur $MgSO_4$ puis on évapore à sec. On obtient 1,1 g du composé attendu sous forme de poudre.

RMN DMSO (300 MHz) : 3,67 ppm : s : 3H ; 3,70 ppm : s : 3H ; 3,80 ppm : s : 3H; 6,54 ppm: d: 1H; 6,61 ppm: s: 1H; 7,19 ppm : d: 1H; 7,41 ppm: d: 1H; 7,90-7,96 ppm : m : 2H ; 8,42 ppm : s : 1H.

B) 3-(2,4-Diméthoxyphényl)-1-méthyl-2-oxo-2,9-dihydro-1H-pyrido[2,3-b] indole-6-carboxylate d'éthyle.

On dissout 0,4 g du composé de l'étape précédente dans 30 ml d'éthanol et 0,5 ml d'$H_2SO_4$ concentré. On chauffe à reflux pendant 5 heures. On évapore l'éthanol, on neutralise avec une solution de $K_2CO_3$ saturée puis on extrait à l'AcOEt. On purifie par chromatographie sur colonne de silice éluée avec de l'AcOEt. On obtient 0,21 g du composé attendu sous forme de poudre.

RMN DMSO (300 MHz) : 1,36 ppm : t : 3H ; 3,67 ppm : s : 3H ; 3,70 ppm : s : 3H ; 3,80 ppm : s : 3H ; 4,32 ppm : q : 2H ; 6,52-6,56 ppm : dd : 1H ; 6,61 ppm : d : 1H ; 7,16 ppm : d : 1H ; 7,54 ppm : d : 1H ; 8,15 ppm : s : 1H ; 8,49 ppm : s : 1H ; 8,83 ppm : d : 1H ; 12,33 ppm : s : 1H.

C) 3-(2,4-Diméthoxyphényl)-1,9-diméthyl-2-oxo-2,9-dihydro-1*H*-pyrido[2,3-b] indole-6-carboxylate d'éthyle.

On met en suspension 15 mg de NaH à 60% dans 5 ml de DMF. On rajoute 0,1 g du composé de la Préparation 3.2, étape B puis à la fin du dégagement gazeux, 0,03 ml de $CH_3I$. On agite à TA pendant 1 heure. On verse le milieu réactionnel sur de l'eau, on filtre le précipité blanc formé, lave avec de l'eau puis on reprend le précipité dans de l'AcOEt et un peu de méthanol. On sèche sur $MgSO_4$ puis on évapore à sec. On reprend le précipité dans un mélange AcOEt/EP (10/90 ; v/v) et on filtre. On obtient 0,1 g du composé attendu.

RMN DMSO (300 MHz) : 1,35 ppm : t : 3H ; 3,70 ppm : s : 3H ; 3,81 ppm : s : 3H ; 3,94 ppm : s : 3H ; 3,99 ppm : s : 3H ; 4,32 ppm : q : 2H ; 6,54-6,57 ppm : dd : 1H ; 6,62 ppm : d : 1H ; 7,16 ppm : d : 1H ; 7,68 ppm : d : 1H ; 7,85-7,89 ppm : dd : 1H ; 8, 18 ppm : s : 1H ; 8,49 ppm : s : 1H.

Préparation 3.3

3-(2,4-Diméthylphényl)-1-méthyl-2-oxo-2,9-dihydro-1*H*-pyrido[2,3-b]indole-6-carboxylate de méthyle.

On dissout 1,3 g du composé de la Préparation 1.3 dans 10 ml d'AcOH glacial, on chauffe à 110°C puis on ajoute 1,18 g du composé de la Préparation 2.2 et on maintient le chauffage à 110°C pendant une nuit. On extrait par AcOEt, on sèche la phase organique sur $MgSO_4$, on filtre et évapore à sec. On chromatographie sur silice en éluant par un mélange AcOEt/cyclohexane (75/25 ; v/v). On obtient 100 mg du composé attendu.

RMN DMSO (300 MHz) : 2,14 ppm : s : 3H ; 2,31 ppm : s : 3H ; 3,70 ppm : s : 3H ; 3,86 ppm : s : 3H ; 7,04 ppm : m : 3H ; 7,55 ppm : d : 1H ; 7,85 ppm : dd : 1H ; 8,18 ppm : s : 1H ; 8,53 ppm : s : 1H ; 12,40 ppm : s : 1H.

En opérant comme décrit dans les Préparations 3 ci-dessus, on prépare les intermédiaires de formule (II') ou (IV) rassemblés dans le tableau ci-après.

TABLEAU 2

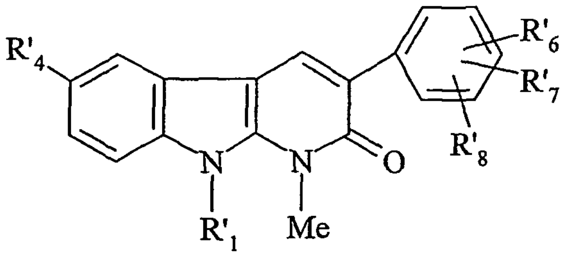

(XIV)

| Préparations | R'$_1$ | R'$_6$, R'$_7$, R'$_8$ | R'$_4$ | Caractérisation |
|---|---|---|---|---|
| 3.4 | H | H | -CHO | F = 240°C |
| 3.5 | H | 2,4-diCl | -COOMe | DMSO (300 MHz) : 3,69 ppm : s: 3H ; 3,86 ppm : s : 3H ; 7,41-7,48 ppm : m : 2H ; 7,54 ppm : d : 1H ; 7,66 ppm : s : 1H ; 7,85 ppm : d : 1H ; 8,33 ppm : s : 1H ; 8,54 ppm : s : 1H ; 12,48 ppm : s : 1H. |
| 3.6 | Me | 2,4-diMe | -COOMe | F = 257°C |
| 3.7 | Me | H | -COOMe | F = 128°C |
| 3.8 | Me | 2-Me, 5-F | -COOMe | DMSO-d$_6$ (300MHz) : δ (ppm) :2,15:s:3H;3,87:s: 3H; 4,03 : s : 3H ; 4,20 : s : 3H ; 7,02-7,11 : m : 2H ; 7,28 : t : 1H ; 7,73 : d : 1H ; 7,90 : dd : 1H ; 8,35 : s : 1H ; 8,58 : d : 1H. |
| 3.9 | Me | 3-F, 4-Me | -COOMe | DMSO-d$_6$ (300MHz) : δ (ppm) : 2,27 : s : 3H ; 3,88 : s : 3H ; 4,02 : s : 3H ; 4,18 : s : 3H ; 7,29 : t : 1H ; 7,60-7,72 : m : 3H ; 7,89 : dd : 1H ; 8,66 : d : 1H ; 8,70 : s : 1H. |
| 3.10 | H | H | -COOEt | DMSO-d$_6$ (200MHz) : δ (ppm) : 1,45 : t : 3H; 3,6 : s : 3H ; 3,8 : s : 3H ; 4,4 : q : 2H ; 7,3-7,5 : m : 3H ; 7,6 : d : 1H ; 7,8-8,0 : m : 3H ; 8,6 : s : 1H ; 8,7 : s : 1H. |

(suite)

| Préparations | $R'_1$ | $R'_6$, $R'_7$, $R'_8$ | $R'_4$ | Caractérisation |
|---|---|---|---|---|
| 3.11 | H | 3-Me, 4-F | -COOMe | DMSO-$d_6$ (300MHz) : δ (ppm) : 2,35 : s : 3H ; 3,77 : s : 3H ; 3,93 : s : 3H ; 7,20 : t : 1H ; 7,60 : d : 1H ; 7,70-7,77 : m : 2H ; 7,91 : d : 1H ; 8,60 : s : 1H ; 8,66 : s : 1H ; 12,49 : s : 1H. |
| 3.12 | Me | 2-Cl | -COOMe | DMSO-$d_6$ (300MHz) : δ (ppm) : 3,84 : s : 3H ; 4,00 : s : 3H ; 4,19 : s: 3H ; 7,38 : se : 3H ; 7,50 : se : 1H ; 7,71 : d : 1H ; 7,87 : d : 1H ; 8,35 : s : 1H ; 8,55 : s : 1H. |
| 3.13 | Me | 4-Cl | -COOMe | DMSO-$d_6$ (300MHz) : δ (ppm) : 3,87 : s : 3H ; 3,97 : s : 3H ; 4,12 : s: 3H ; 7,42 : d : 2H ; 7,63 : d : 1H ; 7,84 : d : 3H ; 8,57 : s : 1H ; 8,59 : s : 1H. |
| 3.14 | Me | 3-Br | -COOMe | DMSO-$d_6$ (300MHz) : δ (ppm) : 3,88 : s : 3H ; 4,01 : s : 3H ; 4,17 : s: 3H ; 7,37 : t : 1H ; 7,47 : m : 1H ; 7,70 : d : 1H ; 7,81-7,95 : m : 2H ; 8,08 : d : 1H ; 8,65 : d : 1H ; 8,72 : s : 1H. |
| 3.15 | Me | 4-Br | -COOMe | F = 227°C |
| 3.16 | Me | 3-F | -COOMe | DMSO-$d_6$ (300MHz) : δ (ppm) : 3,88 : s : 3H ; 4,03 : s : 3H ; 4,18 : s: 3H; 7,08-7,14 : m : 1H ; 7,40-7,47 : m : 1H ; 7,70-7,75 : m : 3H ; 7,90 : dd : 1H ; 8,66 : d : 1H ; 8,73 : s : 1H. |
| 3.17 | Me | 4-F | -COOMe | DMSO-$d_6$ (300MHz) : δ (ppm) : 3,88 : s : 3H ; 4,02 : s : 3H ; 4,17 : s : 3H ; 7,22 : t : 2H ; 7,70 : d : 1H ; 7,80-7,95 : m : 3H ; 8,62 : t : 2H. |
| 3.18 | Me | 3-Me | -COOMe | DMSO-$d_6$ (300MHz) : δ (ppm) : 2,36 : s : 3H ; 3,86 : s : 3H ; 3,97 : s : 3H ; 4,12 : s : 3H ; 7,09 : d : 1H ; 7,27 : t : 1H ; 7,58-7,65 : m : 3H ; 8,86 : d : 1H ; 8,51 : s : 1H ; 8,57 : s : 1H. |

(suite)

| Préparations | $R'_1$ | $R'_6$, $R'_7$, $R'_8$ | $R'_4$ | Caractérisation |
|---|---|---|---|---|
| 3.19 | Me | 3-OMe | -COOMe | DMSO-$d_6$ (300MHz) : δ (ppm) : 3,81 : s : 3H ; 3,87 : s : 3H ; 4,01 : s : 3H ; 4,17 : s : 3H ; 6,88 : d : 1H ; 7,30 : t : 1H ; 7,40 : m : 2H ; 7,68 : d : 1H ; 7,88 : d : 1H ; 8,61-8,64 : m : 2H. |
| 3.20 | Me | 4-OMe | -COOMe | DMSO-$d_6$ (300MHz) : δ (ppm) : 3,79 : s : 3H ; 3,88 : s : 3H ; 4,02 : s : 3H ; 4,17 : s : 3H ; 6,95 : d : 2H ; 7,68-7,76 : m : 3H ; 7,88 : dd : 1H ; 8,53 : s : 1H ; 8,62 : s : 1H. |
| 3.21 | Me | 3-CN | -COOMe | DMSO-$d_6$ (300MHz) : δ (ppm) : 3,96 : s : 3H ; 4,10 : s : 3H ; 4,17: s: 3H; 7,41: d: 1H; 7,53 : m : 1H ; 7,59 : m : 1H ; 7,98 : d : 1H ; 8,07 : m : 2H ; 8,18 : s : 1H ; 8,50 : s : 1H. |
| 3.22 | Me | 4-CN | -COOMe | F = 263°C |
| 3.23 | Me | 4- $OCH_2CH_2$- 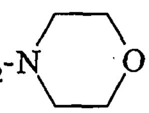 | -COOMe | DMSO-$d_6$ (300MHz) : δ (ppm) : 2,6 : s : 4H ; 2,72 : t : 2H ; 3,59 : m : 4H ; 3,93 : s : 3H ; 4,01 : s : 3H ; 4,15 : m : 5H ; 6,96 : d : 2H ; 7,68-7,73 : m : 3H ; 7,88 : d : 1H ; 8,51 : s : 1H ; 8,61 : s : 1H. |
| 3.24 | Me | 3,5-diF | -COOMe | DMSO-$d_6$ (300MHz) : δ (ppm) : 3,89 : s : 3H ; 4,03 : s : 3H ; 4,19 : s : 3H; 7,08-7,15 : m: 1H ; 7,69-7,74 : m : 3H ; 7,92 : dd : 1H ; 8,69 : s : 1H ; 8,88 : s : 1H. |
| 3.25 | Me | 2-OMe | -COOMe | DMSO-$d_6$ (300MHz) : δ (ppm) : 3,71 : s : 3H ; 3,86 : s : 3H ; 3,99:s:3H;4,18:s: 3H; 6,97 : t : 1H ; 7,03 : d : 1H ; 7,24:d:1H;7,31:t:1H; 7,70 : d : 1H; 7,86 : d : 1H ; 8,24 : s : 1H ; 8,52 : s : 1H. |

**Préparation 3.26**

3-(2,4-Dichlorophényl)-1,9-diméthyl-2-oxo-2,9-dihydro-1*H*-pyrido[2,3-b]indole-6-carboxylate de méthyle.

On chauffe pendant 2 heures à 100°C un mélange de 25,47 g du composé obtenu à la Préparation 1.2 et 27,4 g du composé obtenu à la Préparation 2.3 (VIII) dans 100 ml d'acide acétique. On verse le mélange réactionnel chaud

dans 500 ml d'un mélange eau/glace, essore le précipité formé, le lave à l'eau puis à l'éther et le sèche. On obtient 22 g du composé attendu.

**Préparation 3.27**

3-(2,4-Dichlorophényl)-1,9-diméthyl-2-oxo-2,9-dihydro-1*H*-pyrido[2,3-b]indole-6-carbaldéhyde.

A) 3-(2,4-Dichlorophényl)-1,9-diméihyl-2-oxo-2,9-dihydro-1*H*-pyrido[2,3-b]indole-6-carbonitrile.

On chauffe à 100°C pendant 15 minutes un mélange de 0,4 g du composé obtenu à la Préparation 1.4 dans 4 ml d'acide acétique puis ajoute 0,43 g du composé obtenu à la Préparation 2.3 (VIII) et chauffe à 100°C pendant 3 heures. Après refroidissement à TA, on ajoute de l'eau au mélange réactionnel, essore le précipité beige formé et le lave à l'eau. On reprend le précipité dans du MeOH et concentre sous vide le solvant. On obtient 0,5 g du composé attendu.
RMN$^1$H : DMSO-$d_6$ (300 MHz) : δ (ppm) : 4,02 : s : 3H ; 4,21 : s : 3H ; 7,42-7,51 : m : 2H ; 7,66-7,70 : m : 2H ; 7,83 : d : 1H ; 8,31 : s : 1H ; 8,42 : s : 1H.

B) 3-(2,4-Dichlorophényl)-1,9-diméthyl-2-oxo-2,9-dihydro-1*H*-pyrido[2,3-b]indole-6-carbaldéhyde.

A une solution de 0,45 g du composé obtenu à l'étape précédente dans 30 ml de pyridine, 10 ml d'acide acétique et 6 ml d'eau, on ajoute 3,4 g de $NaH_2PO_2$ et 1,7 ml de nickel de Raney® et chauffe à 65°C pendant 36 heures. On filtre le catalyseur, lave au MeOH et concentre sous vide le filtrat. On reprend le résidu à l'eau, extrait à l'AcOEt, lave la phase organique par une solution saturée de NaCl, sèche sur $MgSO_4$ et évapore le solvant sous vide. On reprend le résidu dans un mélange AcOEt/EP (50/50 ; v/v) et essore le précipité formé. On obtient 0,2 g du composé attendu.

**Préparation 3.28**

3-(2,4-Diméthoxyphényl)-1,9-diméthyl-2-oxo-2,9-dihydro-1*H*-pyrido[2,3-b]indole-6-carboxylate de méthyle.

On chauffe à 110°C pendant 10 minutes une solution de 1,83 g du composé obtenu à la Préparation 1.2 dans 9 ml d'acide acétique, puis ajoute 2 g du composé obtenu à la Préparation 2.4 (VIII) et chauffe à 110°C pendant 12 heures. Après refroidissement à TA, on ajoute de l'eau, essore le précipité formé, le lave à l'eau puis par un mélange EP/propan-2-ol (50/50 ; v/v) et le sèche sous vide. On obtient 2,76 g du composé attendu.
RMN$^1$H : DMSO-$d_6$ (300MHz) : δ (ppm) : 3,71 : s : 3H ; 3,81 : s : 3H ; 3,86 : s : 3H ; 3,99 : s : 3H ; 4,19 : s : 3H ;6,56 : dd : 1H ; 6,62 : d : 1H ; 7,16 : d : 1H ; 7,70 : d : 1H;7,87:dd:1H;8,19:s:1H;8,51:d:1H.

**Préparation 3.29**

3-[4-[[bis(4-Méthoxybenzyl)amino]méthyl]phényl]-1,9-diméthyl-2-oxo-2,9-dihydro-1*H*-pyrido[2,3-b]indole-6-carboxylate de méthyle.

A) 3-[4-(Aminométhyl)phényl]-1,9-diméthyl-2-oxo-2,9-dihydro-1*H*-pyrido[2,3-b] indole-6-carboxylate de méthyle.

On hydrogène à 30°C et sous 50 bars de pression un mélange de 1 g du composé de la Préparation 3.22, 0,3 g de nickel de Raney®, 5 ml de NaOH concentrée dans 50 ml de MeOH. On filtre le catalyseur, amène le filtrat à pH = 7 par ajout d'HCl concentré et concentre sous vide. On reprend le résidu à l'eau, essore le précipité formé et le sèche. On reprend le produit obtenu dans 10 ml de MeOH, ajoute, goutte à goutte, 0,565 g de chlorure de thionyle et laisse 2 heures sous agitation à TA. On concentre sous vide, sèche et utilise le composé attendu tel quel.

B) 3-[4-[[bis(4-Méthoxybenzyl)amino]méthyl]phényl]-1,9-diméthyl-2-oxo-2,9-dihydro-1*H*-pyrido[2,3-b]indole-6-carboxylate de méthyle.

On chauffe pendant 18 heures à 80°C un mélange du composé obtenu à l'étape précédente, 1,48 g de $K_2CO_3$ et 0,841 g de chlorure de 4-méthoxybenzyle dans 10 ml de DMF. On verse le mélange réactionnel dans l'eau, essore le précipité formé et le sèche. On obtient 0,5 g du composé attendu.

**Préparation 3.30**

1,9-Diméthyl-3-[4-(morpholin-4-ylméthyl)phényl]-2-oxo-2,9-dihydro-1*H*-pyrido[2,3-b]indole-6-carboxylate de méthyle.

A) 3-(4-Formylphényl)-1,9-diméthyl-2-oxo-2,9-dihydro-1*H*-pyrido[2,3-b] indole-6-carboxylate de méthyle

On chauffe à 65˚C pendant 18 heures un mélange de 1,87 g du composé de la Préparation 3.22, 7,53 g d'hypophosphite de sodium et 7 g de nickel de Raney® dans 190 ml d'un mélange de pyridine/AcOH/eau (30/10/6 ; v/v/v). On essore le catalyseur et concentre sous vide le filtrat. On reprend le résidu à l'eau, essore le précipité formé et le sèche. On obtient 1,6 g du composé attendu, F = 230˚C.

B) 1,9-Diméthyl-3-[4-(morpholin-4-ylméthyl)phényl]-2-oxo-2,9-dihydro-1*H*-pyrido [2,3-b]indole-6-carboxylate de méthyle.

A un mélange de 0,374 g du composé obtenu à l'étape précédente, 0,174 ml de morpholine et 0,255 g de triacétoxyborohydrure de sodium dans 5 ml de DMF, on ajoute, 1 g de zéolithes 4Å et laisse 18 heures sous agitation à TA. On filtre le mélange réactionnel et concentre sous vide le filtrat. On reprend le résidu à l'eau, essore le précipité formé, le lave à l'EtOH et le sèche. On obtient 0,29 g du composé attendu, F = 175˚C.

**Préparation 3.31**

6-Acétyl-3-(2,4-dichlorophényl)-1,9-diméthyl-1,9-dihydro-2*H*-pyrido[2,3-b] indol-2-one.

A) 3-(2,4-Dichlorophényl)-1,9-diméthyl-1,9-dihydro-2*H*-pyrido[2,3-b]indol-2-one.

On prépare ce composé selon les modes opératoires décrits dans WO 2004/041817, Composé N˚ 28, F = 241˚C.

B) 6-Acétyl-3-(2,4-dichlorophényl)-1,9-diméthyl-1,9-dihydro-2*H*-pyrido[2,3-b] indol-2-one.

On refroidit à 0˚C une solution de 0,5 g du composé obtenu à l'étape précédente dans 20 ml de DCM anhydre, ajoute 0,75 g de chlorure d'aluminium et 0,2 ml de chlorure d'acétyle et laisse 3 heures sous agitation à TA. On verse le mélange réactionnel sur de la glace, extrait au DCM, lave la phase organique par une solution saturée de NaCl, sèche sur $Na_2SO_4$ et évapore le solvant sous vide. On obtient 0,6 g du composé attendu.

RMN$^1$H : DMSO-$d_6$ (300 MHz) : δ (ppm) : 2,63 : s : 3H ; 4,02 : s : 3H ; 4,20 : s : 3H ; 7,43-7,50 : m : 2H ; 7,69-7,73 : m : 2H ; 7,89 : dd : 1H ; 8,38 : s : 1H ; 8,62 : s : 1H.

**Préparation 3.32**

1,9-Diméthyl-2-oxo-3-phényl-2,9-dihydro-1*H*-pyrido[2,3-b]indole-6-carbohydrazide.

A) Acide 1,9-diméthyl-2-oxo-3-phényl-2,9-dihydro-1*H*-pyrido[2,3-b]indole-6-carboxylique.

A une solution de 1,6 g du composé de la Préparation 3.7 dans 75 ml de MeOH et 18 ml d'eau, on ajoute 0,8 g de LiOH, $H_2O$ puis chauffe à reflux pendant 3 heures. On concentre le MeOH sous vide, acidifie à pH = 1 par ajout d'HCl 1N et essore le précipité formé. On reprend le précipité dans du MeOH, évapore le solvant sous vide, triture le résidu dans un mélange EP/AcOEt (90/10 ; v/v) et essore. On obtient 1,5 g du composé attendu.

RMN$^1$H : DMSO-$d_6$ (300 MHz) : δ (ppm) : 4,01 : s : 3H ; 4,16 : s : 3H ; 7,28-7,42 : m : 3H ; 7,62 : d : 1H ; 7,77-7,80 : m : 2H ; 7,89 : dd : 1H ; 8,50-8,57 : m : 2H.

B) 2-[[1,9-Diméthyl-2-oxo-3-phényl-2,9-dihydro-1*H*-pyrido[2,3-b]indol-6-yl]carbonyl]hydrazinecarboxylate de *tert*-butyle.

A une solution de 1,5 g du compose obtenu à l'étape précédente dans 40 ml de DCM et 10 ml de DMF on ajoute 0,66 g d'hydrazinecarboxylate de *tert*-butyle, 2,5 g de PyBOP et 0,9 ml de DIPEA puis laisse 4 heures sous agitation à TA. On verse le mélange réactionnel sur une solution saturée de $NH_4Cl$, extrait au DCM, sèche la phase organique sur $MgSO_4$ et évapore le solvant sous vide. On chromatographie le résidu sur gel de silice en éluant à l'AcOEt puis par un mélange AcOEt/MeOH (95/5 ; v/v). On reprend le produit obtenu dans un mélange AcOEtlcyclohexane (75/25 ; v/v) et essore le précipité formé. On obtient 1,9 g du composé attendu.

RMN$^1$H : DMSO-$d_6$ (300 MHz) : δ (ppm) : 1,39 : s : 9H ; 4,06 : s : 3H ; 4,17 : s : 3H ; 7,29-7,42 : m : 3H ; 7,67 : d : 1H ; 7,76-7,82 : m : 3H ; 8,40 : s : 1H ; 8,51 : s : 1H ; 8,91 : s : 1H ; 10,11 : s : 1H.

C) 1,9-Diméthyl-2-oxo-3-phényl-2,9-dihydro-1*H*-pyrido[2,3-b]indole-6-carbohydrazide.

On chauffe à 80°C pendant 2 heures un mélange de 1,9 g du composé obtenu à l'étape précédente dans 100 ml de MeOH et 10 ml d'HCl 6N. On concentre le mélange réactionnel sous vide, reprend le résidu dans du MeOH, amène à pH = 7 par ajout de triéthylamine et concentre sous vide. On chromatographie le résidu sur gel de silice en éluant à l'AcOEt puis par le mélange AcOEt/MeOH/$NH_4OH$ à 28 % (90/10/1 ; v/v/v). On obtient le composé attendu.
RMN$^1$H : DMSO-$d_6$ (300 MHz) : δ (ppm) : 4,00 : s : 3H ; 4,14 : s : 3H ; 7,26-7,76 : m : 7H ; 8,39 : s : 1H ; 8,47 : s : 1H ; 9,68 : s : 1H.

**Préparation 3.33**

3-(2,4-Dichlorophényl)-1,9-diméthyl-2-oxo-2,9-dihydro-1*H*-pyrido[2,3-b]indole-6-carbohydrazide.

A) 2-[[3-(2,4-Dichlorophényl)-1,9-diméthyl-2-oxo-2,9-dihydro-1*H*-pyrido[2,3-b] indol-6-yl]carbonyl]hydrazinecarboxylate de *tert*-butyle.

On prépare ce composé selon le mode opératoire décrit à l'étape B de la Préparation 3.32 à partir du composé obtenu à l'étape A de la variante de l'EXEMPLE 5. On obtient 0,33 g du composé attendu.
RMN$^1$H : DMSO-$d_6$ (300 MHz) : δ (ppm) : 1,44 : s : 9H ; 4,03 : s : 3H ; 4,26 : s : 3H ; 7,43-7,50 : m : 2H ; 7,68-7,72 : m : 2H ; 7,80-7,82 : m : 1H ; 8,23 : s : 1H ; 8,43 : s : 1H ; 8,86 : s : 1H ; 10,08 : s : 1H.

B) 3-(2,4-Dichlorophényl)-1,9-diméthyl-2-oxo-2,9-dihydro-1*H*-pyrido[2,3-b]indole-6-carbohydrazide.

On prépare ce composé selon le mode opératoire décrit à l'étape C de la Préparation 3.32 à partir du composé de l'étape précédente.
RMN1H : DMSO-$d_6$ (300 MHz) : δ (ppm) : 4,02 : s : 3H ; 4,18 : s : 3H ; 7,42-7,50 : m : 2H ; 7,65-7,79 : m : 3H ; 8,21 : s : 1H ; 8,38 : d : 1H ; 9,64 : s : 1H.

**Préparation 3.34**

3-(2,4-Diméthoxyphényl)-1-méthyl-2-oxo-2,9-dihydro-1*H*-pyrido[2,3-b]indole-6-carbohydrazide.

A) Acide 3-(2,4-diméthoxyphényl)-1-méthyl-2-oxo-2,9-dihydro-1*H*-pyrido[2,3-b] indole-6-carboxylique.

A une suspension de 0,9 g du composé obtenu à l'étape B de la Préparation 3.1 dans 6 ml d'eau, on ajoute 2 g de KOH puis 0,5 ml d'$H_2O_2$ à 35 % et chauffe à 130°C pendant 2 jours. Après refroidissement à TA, on alcanilise par ajout de NaOH concentrée, lave la phase aqueuse à l'AcOEt, acidifie la phase aqueuse par ajout d'HCl 1N. Essore le précipité formé et le lave à l'eau. On reprend le précipité au MeOH et évapore le solvant sous vide. On reprend le précipité dans un mélange propan-2-ol/EP (50/50 ; v/v), triture et essore le précipité. On obtient 0,9 g du composé attendu.
RMN$^1$H : DMSO-$d_6$ (300 MHz) : δ (ppm) : 3,69-3,80 : m : 9H ; 6,53-6,61 : m : 2H ; 7,20 : d : 1H ; 7,35 : d : 1H ; 7,80 : d : 1H ; 7,94 : s : 1H ; 8,30 : s : 1H.

B) 2-[[3-(2,4-Diméthoxyphényl)1-méthyl-2-oxo-2,9-dihydro-1*H*-pyrido[2,3-b]indol-6-yl]carbonyl]hydrazinecarboxylate de *tert*-butyle.

On prépare ce composé selon le mode opératoire décrit à l'étape B de la Préparation 3.32 à partir du composé de l'étape précédente.
RMN$^1$H : DMSO-$d_6$ (300 MHz) : δ (ppm) : 1,43 : s : 9H ; 3,68 : s : 3H ; 3,71 : s : 3H ; 3,81 : s : 3H ; 6,54-6,63 : m : 2H ; 7,18 : d : 1H; 7,51 : d : 1H ; 7,69-7,74 : m : 1H ; 7,98 : s : 1H ; 8,27 : s : 1H ; 8,84 : se : 1H ; 10,00 : se : 1H ; 12,23 : se : 1H.

C) 3-(2,4-Diméthoxyphényl)-1-méihyl-2-oxo-2,9-dihydro-1*H*-pyrido[2,3-b]indole-6-carbohydrazide.

On laisse une nuit sous agitation à TA un mélange de 0,39 g du composé obtenu à l'étape précédente dans 15 ml de MeOH et 1 ml d'HCl 6N. On concentre sous vide le mélange réactionnel, reprend le résidu dans la triéthylamine

et la pyridine jusqu'à dissolution et chromatographie sur gel de silice en éluant par le mélange AcOEt/MeOH/$NH_4OH$ à 28 % (90/10/1 ; v/v/v). On obtient 0,2 g du composé attendu.

**Préparation 3.35**

3-(2,4-Dichlorophényl)-N'-hydroxy-1,9-diméthyl-2-oxo-2,9-dihydro-1*H*-pyrido[2,3-b] indole-6-carboximidamide.

A une suspension de 1 g du composé de la Préparation 3.27 étape A dans 20 ml d'EtOH anhydre, on ajoute 0,91 g de chlorhydrate d'hydroxylamine et 1,82 ml de triéthylamine et chauffe à reflux pendant 3 jours. Après refroidissement à TA, on ajoute au mélange réactionnel du MeOH et de la pyridine et chromatographie sur gel de silice en éluant par le mélange AcOEt/MeOH/triéthylamine (90/10/2 ; v/v/v). On reprend le produit obtenu dans l'eau, triture, essore le précipité formé et le lave avec un mélange propan-2-ol/EP (50/50 ; v/v). On obtient 0,7 g du composé attendu.
RMN$^1$H : DMSO-$d_6$ (300 MHz) : δ (ppm) : 4,02 : s : 3H ; 4,20 : s : 3H ; 7,41-7,50 : m : 2H ; 7,65-7,68 : m : 2H ; 7,82 : d : 1H ; 8,30 : s : 1H ; 8,41 : s : 1H.

**Préparations des composés de formule (V).**

**Préparation 4.1**

N"-Hydroxy-N,N-bis(4-méthoxybenzyl)guanidine (V).

A) bis-(4-Méthoxybenzyl)cyanamide.

A une solution de 1 g de cyanamide dans 50 ml de DMF on ajoute 13,1 g de $K_2CO_3$ et 6,5 ml de chlorure de 4-méthoxybenzyle, puis chauffe à 80°C pendant 20 heures. Après refroidissement à TA, on verse le mélange réactionnel dans l'eau, extrait à l'AcOEt, sèche la phase organique sur $MgSO_4$ et évapore le solvant sous vide. On chromatographie le résidu sur gel de silice en éluant par le mélange AcOEt/cyclohexane (75/25 ; v/v). On obtient 6,5 g du composé attendu sous forme d'huile.
RMN $CDCl_3$ (300 MHz) : δ (ppm) : 3,73 : s : 6H ; 3,95 : s: 4H ; 6,82 : d : 4H ; 7,16 : d : 4H.

B) N"-Hydroxy-N,N-bis(4-méthoxybenzyl)guanidine (V).

A une solution de 6,5 g du compose obtenu à l'étape précédente dans 40 ml d'EtOH on ajoute 3,2 g de chlorhydrate d'hydroxylamine et 6,4 ml de triéthylamine puis laisse 5 heures sous agitation à TA. On concentre le mélange réactionnel sous vide, reprend le résidu à l'eau, extrait à l'AcOEt, sèche sur $MgSO_4$ et évapore le solvant sous vide. On obtient 3,6 g du composé attendu.
RMN $CDCl_3$ (300 MHz) : δ (ppm) : 3,80 : s : 6H ; 4,24 : s: 4H ; 6,84 : d : 4H ; 7,16 : d : 4H.

**Préparation 4.2**

N'-Hydroxy-2-phénoxyéthanimidamide.

On chauffe à reflux pendant 3 heures un mélange de 1 g de phénoxyacétonitrile, 2,4 g de $K_2CO_3$ et 1,2 g de chlorhydrate d'hydroxylamine dans 60 ml d'EtOH et 10 ml d'eau. On concentre sous vide le mélange réactionnel, reprend le résidu à l'eau et extrait à l'AcOEt. On extrait la phase organique par une solution d'HCl 1N, alcalinise la phase aqueuse par ajout d'une solution de NaOH 1N, extrait à l'AcOEt, sèche sur $MgSO_4$ et évapore le solvant sous vide. On obtient 0,9 g du composé attendu sous forme d'huile incolore qui cristallise.
RMN$^1$H : $CDCl_3$ (300 MHz) : δ (ppm) : 4,56 : s: 2H ; 4,90 : s : 2H ; 6,95-7,02 : m : 3H ; 7,26-7,32 : m : 2H.

**EXEMPLE 1 :** Composé N° 7 3-(2,4-Diméthoxyphényl)-1-méthyl-2-oxo-2,9-dihydro-1*H*-pyrido[2,3-b]indole-6-carbaldéhyde-O-éthyloxime.

On dissout 0,3 g du composé de la Préparation 3.1 dans 20 ml d'éthanol, on ajoute 0,4 g de chlorhydrate de O-éthylhydroxylamine dissous dans 1 ml d'eau et on laisse 2 heures sous agitation à TA. On évapore l'éthanol, reprend par de l'eau puis extrait par AcOEt. On chromatographie sur silice en éluant par AcOEt puis AcOEt/MeOH (98/2 ; v/v). On obtient 70 mg du composé attendu.
RMN$^1$H : DMSO (300 MHz) : 1,26 ppm : t : 3H ; 3,66 ppm : s : 3H ; 3,70 ppm : s : 3H ; 3,80 ppm : s : 3H ; 4,13 ppm : q : 2H ; 6,52-6,56 ppm : dd : 1H ; 6,60 ppm : d : 1H ; 7,16 ppm : d : 1H ; 7,48 ppm : s : 2H ; 8 ppm : s : 2H ; 8,26

ppm : s : 1H ; 12,14 ppm : s : 1H.

**EXEMPLE 2 :** Composé N° 8

3-(2,4-Diméthoxyphényl)-1-méthyl-6-(3-phényl-1-[1,2,4]oxadiazol-5-yl)-1,9-dihydro-2H-pyrido[2,3-b]indol-2-one.

On dissout 0,5 g du composé de la Préparation 3.2, étape A dans 10 ml de DMF, on ajoute 0,29 g de CDI puis on agite pendant 30 minutes à TA. On ajoute 0,22 g de N'-hydroxybenzènecarboximidamide dissous dans 4 ml de DMF, on agite à TA pendant 6 heures puis on rajoute 0,26 g de CDI et on chauffe à 115°C pendant 18 heures. On ajoute de l'eau, extrait à l'éther puis sèche sur $MgSO_4$ et chromatographie sur silice en éluant par AcOEt puis AcOEt/MeOH (98/2 ; v/v). Le résidu obtenu est trituré dans un mélange AcOEtlcyclohexane (50/50 ; v/v) puis filtré. On obtient 0,15 g du composé attendu.

RMN$^1$H : DMSO (300 MHz) : 3,69 ppm : s : 3H ; 3,72 ppm : s : 3H ; 3,81 ppm : s : 3H ; 6,55-6,59 ppm : dd : 1H ; 6,63 ppm : d : 1H ; 7,18 ppm : d : 1H ; 7,55-7,62 ppm : m : 3H ; 7,68 ppm : d : 1H ; 8,01-8,05 ppm : dd : 1H ; 8,10-8,16 ppm : m : 2H ; 8,25 ppm : s : 1H ; 8,73 ppm : d : 1H ; 12,48 ppm : s : 1H.

**EXEMPLE 3 :** Composé N° 3

3-(2,4-Dichlorophényl)-1-méthyl-6-(3-méthyl-[1,2,4]oxadiazol-5-yl)-1,9-dihydro-2H-pyrido[2,3-b]indol-2-one.

On met en suspension 0,12 g de NaH à 60 % dans 12 ml de THF anhydre, on ajoute 0,32 g de tamis moléculaire puis 0,22 g de N'-hydroxyéthanimidamide et on chauffe à 70°C pendant 1 heure et demie. On ajoute 0,4 g du composé de la Préparation 3.5 puis on chauffe à 80°C pendant 2 heures. On ajoute de l'eau, extrait par AcOEt puis on chromatographie sur silice en éluant par AcOEt/cyclohexane (60/40 ; v/v) puis AcOEt pur. On obtient 120 mg du composé attendu.

RMN$^1$H : DMSO (300 MHz) : 2,47 ppm : s : 3H ; 3,77 ppm : s : 3H ; 7,49-7,56 ppm : m : 2H ; 7,71-7,75 ppm : m : 2H ; 8,01 ppm : d : 1H ; 8,45 ppm : s : 1H ; 8,73 ppm : s : 1H ; 12,64 ppm : s : 1H.

**EXEMPLE 4 :** Composé N° 2

3-(2,4-Dichlorophényl)-1,9-diméthyl-6-(3-méthyl-[1,2,4]oxadiazol-5-yl)-1,9-dihydro-2H-pyrido[2,3-b]indol-2-one.

On met en suspension 7 mg de NaH à 60 % dans 2 ml de DMF et on ajoute 50 mg du composé de l'Exemple 3 puis 0,02 ml de $CH_3I$ puis on laisse sous agitation à TA pendant 2 heures. On verse le mélange réactionel sur de l'eau puis on filtre le précipité formé. On le lave à l'eau puis avec un mélange AcOEt/cyclohexane (50/50 ; v/v) pour obtenir 30 mg du composé attendu.

RMN$^1$H: DMSO (300 MHz) : 2,27 ppm : s : 3H ; 4,03 ppm : s : 3H ; 4,22 ppm : s : 3H ; 7,43-7,50 ppm : m : 2H ; 7,69 ppm : s : 1H ; 7,84 ppm : d : 1H ; 7,98 ppm : d : 1H ; 8,44 ppm : s : 1H ; 8,69 ppm : s : 1H.

**EXEMPLE 5** : Composé N° 14

6-(3-Amino-1,2,4-oxadiazol-5-yl)-3-(2,4-dichlorophényl)-1,9-diméthyl-1,9-dihydro-2*H*-pyrido[2,3-b]indol-2-one.

A un mélange de 3 g de tamis moléculaire dans 15 ml d'éthanol on ajoute 0,26 g de sodium et laisse 15 minutes sous agitation à TA. On ajoute ensuite 0,9 g de N''-hydroxyguanidine hémisulfate hémihydrate et laisse 1 heure sous agitation à TA. On ajoute enfin 0,4 g du composé obtenu à la Préparation 3.26 et chauffe à reflux pendant 4 jours. Après refroidissement à TA, on ajoute de l'eau au mélange réactionnel, extrait à l'AcOEt, sèche la phase organique sur $MgSO_4$ et évapore le solvant sous vide. On chromatographie le résidu sur gel de silice en éluant par le mélange AcOEt/cyclohexane (75/25 ; v/v). On obtient 0,02 g du produit attendu.

RMN$^1$H : DMSO-$d_6$ (300 MHz) : δ (ppm) : 4,04 : s : 3H ; 4,22 : s : 3H ; 6,32 : s : 2H ; 7,43-7,51 : m : 2H ; 7,70 : d : 1H ; 7,81 : d : 1H ; 7,90 : dd : 1H ; 8,42 : s : 1H ; 8,5 : d : 1H.

On peut également préparer le composé N°14 en suivant les modes opératoires décrits dans les étapes A, B, C et D ci-après.

A) Acide 3-(2,4-dichlorophényl)-1,9-diméthyl-2-oxo-2,9-dihydro-1H-pyrido[2,3-b] indole-6-carboxylique.

A un mélange de 9,5 g du composé obtenu à la Préparation 3.26 dans 100 ml de MeOH on ajoute une solution de 5,14 g de KOH en pastilles dans 90 ml d'eau et chauffe à reflux pendant 18 heures. Après refroidissement à TA puis au bain de glace, on acidifie le mélange réactionnel à pH = 1 par ajout d'une solution d'HCl 5N, en diluant à l'eau pour

permettre l'agitation. On essore le précipité formé, le lave à l'eau et le sèche. On obtient 9,1 g du composé attendu.

B) Chlorure de 3-(2,4-dichlorophényl)-1,9-diméthyl-2-oxo-2,9-dihydro-1*H*-pyrido [2,3-b]indole-6-carbonyle.

On refroidit au bain de glace une suspension de 9,1 g du composé obtenu à l'étape précédente dans 100 ml de THF anhydre, ajoute, goutte à goutte, une solution de 45 ml de chlorure de thionyle dans 45 ml de THF et laisse 15 minutes sous agitation puis concentre sous vide à TA On reprend le résidu par 40 ml de DCM et évapore le solvant sous vide. On obtient le composé attendu que l'on utilise tel quel.

C) N-Cyano-3-(2,4-dichlorophényl)-1,9-diméthyl-2-oxo-2,9-dihydro-1*H*-pyrido [2,3-b]indole-6-carboxamide.

A une solution du composé obtenu à l'étape précédente dans 100 ml de THF anhydre on ajoute une solution de 10 g de cyanamide dans 50 ml de THF et chauffe à 60°C sous atmosphère d'argon. Après 30 minutes, on ajoute 100 ml de THF et concentre sous vide la suspension obtenue. On reprend le résidu dans 200 ml d'EtOH, laisse une nuit sous agitation à TA, essore le précipité formé, le lave à l'EtOH et le sèche sous vide. On reprend le produit obtenu dans l'eau, essore le précipité et le sèche. On obtient 8,3 g du composé attendu.

D) 6-(3-Amino-1,2,4-oxadiazol-5-yl)-3-(2,4-dichlorophényl)-1,9-diméthyl-1,9-dihydro-2*H*-pyrido[2,3-b]indol-2-one.

On chauffe à 120°C pendant 15 minutes un mélange de 3 g du composé obtenu à l'étape précédente et 0,9 g de chlorhydrate d'hydroxylamine dans 60 ml de pyridine. On ajoute ensuite 30 g d'alumine 60 basique et on concentre sous vide jusqu'à siccité. On chromatographie le résidu sur alumine 60 basique en éluant à l'AcOEt puis par le gradient du mélange AcOEt/MeOH jusqu'à (97/3 ; v/v). On obtient 1,2 g du composé attendu.

**EXEMPLE 6** : Composé N° 20

3-(2,4-Dichlorophényl)-1,9-diméthyl-2-oxo-2,9-dihydro-1*H*-pyrido[2,3-b]indole-6-carbaldéhyde oxime.

A une solution de 0,2 g du composé obtenu à la Préparation 3.27 dans 15 ml d'EtOH à 96 % on ajoute 0,18 g de chlorhydrate d'hydroxylamine et laisse 3 heures sous agitation à TA. On concentre sous vide le solvant, reprend le résidu à l'eau et essore le précipité formé. On dissout le précipité dans un mélange MeOH/pyridine et chromatographie cette solution sur gel de silice en éluant par le mélange DCM/MeOH (98/2 ; v/v). On obtient 0,14 g du composé attendu, F = 287-290°C.

RMN$^1$H : DMSO-$d_6$ (300 MHz) : $\delta$ (ppm) : 4,01 : s : 3H ; 4,17 : s : 3H ; 5,76 : s : 1H ; 7,42-7,49 : m : 2H ; 7,53-7,57 : dd : 1H ; 7,63-7,69 : m : 2H ; 8,06 : d : 1H ; 8,19-8,23 : m : 2H.

**EXEMPLE 7 :** Composé N° 21

3-(2,4-Dichlorophényl)-1,9-diméthyl-2-oxo-2,9-dihydro-1*H*-pyrido[2,3-b]indole-6-carbaldéhyde O-éthyloxime.

A une solution de 0,2 g du composé obtenu à la Préparation 3.27 dans 15 ml d'EtOH on ajoute 0,25 g de chlorhydrate de O-éthylhydroxylamine et laisse 20 heures sous agitation à TA. On concentre sous vide le mélange réactionnel, reprend le résidu à l'eau et essore le précipité formé. On reprend le précipité dans un mélange AcOEt/MeOH (50/50 ; v/v) et chromatographie cette solution sur gel de silice en éluant par le mélange AcOEt/cyclohexane (75/25 ; v/v). On reprend le produit obtenu dans un mélange AcOEt/cyclohexane (50/50 ; v/v) et essore le précipité formé. On obtient 0,1 g du composé attendu.

RMN$^1$H : DMSO-$d_6$ (300 MHz) : $\delta$ (ppm) : 1,27 : t : 3H ; 4,02 : s : 3H ; 4,12-4,22 : m : 5H ; 7,42-8,48 : m : 8H.

**EXEMPLE 8 :** Composé N° 22

3-(2,4-Dichlorophényl)-1,9-diméthyl-2-oxo-2,9-dihydro-1*H*-pyrido[2,3-b]indole-6-carbaldéhyde O-isobutyloxime.

A une solution de 0,14 g du composé N° 20 dans 4 ml de DMF on ajoute 0,017 g d'hydrure de sodium à 60 % dans l'huile et laisse 10 minutes sous agitation à TA. On ajoute ensuite 0,076 ml de bromure d'isobutyle et laisse une nuit sous agitation à TA. On verse le mélange réactionnel sur une solution saturée de $NH_4Cl$, extrait à l'AcOEt, sèche la phase organique sur $MgSO_4$ et évapore le solvant sous vide. On chromatographie le résidu sur gel de silice en éluant par le mélange AcOEt/cyclohexane (75/25 ; v/v). On obtient 0,035 g du composé attendu, F = 100-110°C.

RMN$^1$H : DMSO-$d_6$ (300 MHz) : $\delta$ (ppm) : 0,92 : s : 3H ; 0,94 : s : 3H ; 2,02 : m : 1H ; 3,88 : d : 2H ; 4,01 : s :

3H ; 4,17 : s : 3H ; 7,42-7,50 : m : 2H ; 7,54-7,57 : dd : 1H ; 7,65-7,69 : m : 2H ; 8,10 : d : 1H ; 8,26 : s : 1H ; 8,30 : s : 1H.

**EXEMPLE 9 :** Composé N° 23

Acide [[[[3-(2,4-dichlorophényl)-1,9-diméthyl-2-oxo-2,9-dihydro-1*H*-pyrido[2,3-b]indol-6-yl]méthylène]amino]oxy]acétique.

A une solution de 0,3 g du composé obtenu à la Préparation 3.27 dans 25 ml d'EtOH à 96 % on ajoute 0,426 g d'hémichlorhydrate de O-(carboxyméthyl)hydroxylamine et laisse une nuit sous agitation à TA. On concentre sous vide le mélange réactionnel, reprend le résidu dans l'eau, triture et essore le précipité formé. On reprend le précipité dans une solution de NaOH 2N, lave la phase aqueuse à l'AcOEt, acidifie la phase aqueuse à pH = 1 par ajout d'une solution d'HCl 6N, essore le précipité formé et extrait le filtrat à l'AcOEt. On réunit le précipité et la phase organique et évapore sous vide le solvant. On reprend le résidu dans du propan-2-ol chaud, essore à chaud le précipité formé et le lave à l'éther. On obtient 0,135 g du composé attendu.

RMN$^1$H : DMSO-$d_6$ (300 MHz) : δ (ppm) : 4,01 : s : 3H ; 4,18 : s : 3H ; 4,64 : s : 2H ; 7,42-7,49 : m : 2H ; 7,55 : dd : 1H ; 7,67-7,69 : m : 2H ; 8,12 : d : 1H ; 8,27 : s : 1H ; 8,37 : s : 1H ; 12,8 : se : 1H.

**EXEMPLE 10** : Composé N° 26

6-(3-Amino-1,2,4-oxodiazol-5-yl)-3-(2,4-diméthoxyphényl)-1,9-diméthyl-1,9-dihydro-2*H*-pyrido[2,3-b]indol-2-one.

A) 6-[3-[bis(4-méthoxybenzyl)amino]-1,2,4-oxodiazol-5-yl]-3-(2,4-diméthoxyphényl)-1,9-diméthyl-1,9-dihydro-2*H*-pyrido[2,3-b]indol-2-one.

A un mélange de 6,3 g de tamis moléculaire dans 63 ml d'EtOH anhydre on ajoute 0,78 g de sodium puis, après dissolution, on ajoute 5,6 g du composé obtenu à la Préparation 4.1 et laisse 2 heures sous agitation à TA et sous atmosphère d'argon. On ajoute, ensuite, 1,2 g du composé obtenu à la Préparation 3.28 et chauffe à reflux pendant une nuit. Après refroidissement à TA, on ajoute du DCM et de l'AcOEt au mélange réactionnel, filtre le tamis moléculaire, le lave au DCM, lave le filtrat par une solution d'HCl 1N, par une solution de NaOH 2N, sèche la phase organique sur $MgSO_4$ et évapore le solvant sous vide. On chromatographie le résidu sur gel de silice en éluant par le mélange DCM/MeOH (97/3 ; v/v). On reprend le produit obtenu au MeOH, triture, essore le précipité formé, le lave au MeOH et le sèche sous vide. On obtient 0,596 g du composé attendu.

RMN$^1$H : DMSO-$d_6$ (300 MHz) : δ (ppm) : 3,71 : m : 9H ; 3,79 : s : 3H ; 3,99 : s : 3H;4,20:s:3H;4,48:s:4H;6,56:m:1H;6,61:s:1H;6,90:d:4H;7,14:d : 1H ; 7,23 : d : 4H ; 7,79 : d : 1H ; 7,91 : d : 1H ; 8,24 : s : 1H ; 8,59 : s : 1H.

B) 6-(3-Amino-1,2,4-oxodiazol-5-yl)-3-(2,4-diméthoxyphényl)-1,9-diméthyl-1,9-dihydro-2*H*-pyrido[2,3-b]indol-2-one.

A une solution de 0,593 g du composé obtenu à l'étape précédente dans 10 ml de DCM et 2 ml d'eau on ajoute 1 g de DDQ et chauffe à reflux pendant une nuit. Après refroidissement à TA, on essore le précipité formé, le lave au DCM puis à l'eau. On dissout le précipité dans du MeOH et chromatographie sur gel de silice en éluant à l'AcOEt puis par le mélange AcOEt/MeOH (90/10 ; v/v). On reprend le produit obtenu dans du MeOH, essore le précipité formé et le lave au MeOH. On obtient 0,094 g du composé attendu, F =320-322°C.

RMN$^1$H : DMSO-$d_6$ (300 MHz) : δ (ppm) : 3,76 : s : 3H ; 3,86 : s : 3H ; 4,05 : s : 3H ; 4,25 : s : 3H ; 6,39 : s : 2H ; 6,62 : dd : 1H ; 6,68 : d : 1H ; 7,22 : d : 1H ; 7,84 : d : 1H ; 7,93 : dd : 1H ; 8,26 : s : 1H ; 8,57 : s : 1H.

**EXEMPLE 11 :** Composé N° 39

3-[4-(Aminométhyl)phényl]-6-(3-amino-1,2,4-oxadiazol-5-yl)-1,9-diméthyl-1,9-dihydro-2*H*-pyrido[2,3-b] indol-2-one.

A) 3-[4-([bis(4-méthoxybenzyl)amino]méthyl]phényl]-6-[3-[bis(4-méthoxybenzyl) amino]-1,2,4-oxadiazol-5-yl]-1,9-diméthyl-1,9-dihydro-2*H*-pyrido[2,3-b] indol-2-one.

On prépare ce composé selon le mode opératoire décrit à l'étape A de l'Exemple 10 à partir du composé de la Préparation 3.29 et du composé de la Préparation 4.1.

B) 3-[4-(Aminométhyl)phényl]-6-(3-amino-1,2,4-oxadiazol-5-yl)-1,9-diméthyl-1,9-dihydro-2*H*-pyrido[2,3-b] indol-2-one.

On prépare ce composé selon le mode opératoire décrit à l'étape B de l'Exemple 10. On obtient 0,2 g du

composé attendu.

RMN$^1$H : DMSO-$d_6$/TFA (200 MHz) : δ (ppm) : 3,9 : s : 2H ; 4,0 : s : 3H ; 4,2 : s : 3H ; 7,5 : d : 2H ; 7,8-8,0 : m : 4H ; 8,65 : d : 2H.

**EXEMPLE 12 :** Composé N° 41

3-(2,4-Dichlorophényl)-6-[3-(diméthylamino)-1,2,4-oxadiazol-5-yl]-1,9-diméthyl-1,9-dihydro-2*H*-pyrido[2,3-b]indol-2-one.

A une solution de 0,09 g du composé N° 14 dans 3 ml de DMF on ajoute 0,025 g d'hydrure de sodium à 60 % dans l'huile puis 0,04 ml d'iodure de méthyle et laisse 2 heures sous agitation à TA. On ajoute de l'eau au mélange réactionnel, essore le précipité formé, le lave à l'eau puis par un mélange propan-2-ol/EP (50/50 ; v/v). On obtient 0,07 g du composé attendu, F = 320-324°C.

RMN$^1$H : pyridine-$d_5$ (300 MHz) : δ (ppm) : 2,94 : s : 6H ; 3,80 : s : 3H ; 3,82 : s : 3H ; 7,32 : dd : 1H ; 7,47-7,52 : m : 2H ; 7,58 : d : 1H ; 7,95 : s : 1H ; 8,09 : dd : 1H ; 8,66 : d : 1H.

**EXEMPLE 13** : Composé N° 42

N-[5-[3-(2,4-Dichlorophényl)-1,9-diméthyl-2-oxo-2,9-dihydro-1*H*-pyrido[2,3-b] indol-6-yl]-1,2,4-oxadiazol-3-yl]cyclopropane carboxamide.

A une solution de 0,1 g du composé N° 14 dans 5 ml de pyridine on ajoute 0,4 ml de chlorure de cyclopropanecarbonyle et chauffe à 50°C pendant 2 heures. Après refroidissement à TA on extrait à l'AcOEt, lave la phase organique à l'eau, sèche sur $Na_2SO_4$ et évapore le solvant sous vide. On chromatographie le résidu sur gel de silice en éluant par le mélange AcOEt/MeOH (90/10 ; v/v). On reprend le produit obtenu dans un mélange AcOEt/cyclohexane (50/50 ; v/v), triture et essore le précipité formé. On obtient 0,065 g du composé attendu, F = 292-295°C.

RMN$^1$H : DMSO-$d_6$ (300 MHz) : δ (ppm) : 0,86 : m : 4H ; 1,99 : m : 1H ; 4,03 : s : 3H ; 4,23 : s : 3H ; 7,43-7,51 : m : 2H ; 7,70 : s : 1H ; 7,86 : d : 1H ; 7,96 : dd : 1H ; 8,45 : s : 1H ; 8,66 : s : 1H ; 11,48 : s : 1H.

**EXEMPLE 14** : Composé N° 44

N-[5-[3-(2,4-Dichlorophényl)-1,9-diméthyl-2-oxo-2,9-dihydro-1*H*-pyrido[2,3-b] indol-6-yl]-1,2,4-oxadiazol-3-yl]méthane sulfonamide.

A une solution de 0,1 g du composé N° 14 dans 10 ml de pyridine et 1 ml de DCM on ajoute 0,08 ml de chlorure de méthanesulfonyle et 0,025 g de DMAP puis laisse 96 heures sous agitation à TA. On verse le mélange réactionnel dans l'eau, extrait à l'AcOEt, lave la phase organique par une solution saturée de NaCl, sèche sur $Na_2SO_4$ et évapore le solvant sous vide. On chromatographie le résidu sur gel de silice en éluant par le mélange DCM/MeOH (95/5 ; v/v). On obtient 0,025 g du composé attendu, F = 263-266°C.

RMN$^1$H : DMSO-$d_6$ (300 MHz) : δ (ppm) : 3,22 : s : 3H ; 4,03 : s : 3H ; 4,23 : s : 3H ; 7,43-7,51 : m : 2H ; 7,69 : d : 1H ; 7,84 : d : 1H ; 7,95 : d : 1H ; 8,47 : s : 1H ; 8,64 : s : 1H ; 11,82 : s : 1H.

**EXEMPLE 15** : Composé N° 49

[3-(2,4-Dichlorophényl)-1-méthyl-6-(3-méthyl-1,2,4-oxadiazol-5-yl)-2-oxo-1,2-dihydro-9*H*-pyrido[2,3-b] indol-9-yl]acétonitrile.

On chauffe à 80°C pendant 4 jours un mélange de 0,3 g du composé N° 3, 0,195 g de $K_2CO_3$ et 0,1 ml de bromoacétonitrile dans 20 ml de DMF. Après refroidissement à TA, on verse le mélange réactionnel dans une solution saturée de $NH_4Cl$, extrait à l'AcOEt, lave la phase organique par une solution saturée de NaCl, sèche sur $MgSO_4$ et évapore le solvant sous vide. On chromatographie le résidu sur gel de silice en éluant par le mélange AcOEt/cyclohexane (75/25 ; v/v). On triture le produit obtenu dans du cyclohexane et essore le précipité formé. On obtient 0,09 g du composé obtenu.

RMN$^1$H : DMSO-$d_6$ (300 MHz) : δ (ppm) : 2,43 : s : 3H ; 4,02 : s : 3H ; 6,01 : s : 2H ; 7,45-7,53 : m : 2H ; 7,71 : d : 1H ; 8,00 : d : 1H ; 8,09 : dd : 1H ; 8,52 : s : 1H ; 8,76:d:1H.

**EXEMPLE 16 :** Composé N° 50

3-(2,4-Dichlorophényl)-1-méthyl-6-(3-méthyl-1,2,4-oxodiazol-5-yl)-9-(2-morpholin-4-yléthyl)-1,9-dihydro-2*H*-pyrido [2,3-b]indol-2-one.

On chauffe à 80°C pendant 4 jours un mélange de 0,3 g du composé N° 3, 0,39 g de $K_2CO_3$ et 0,263 g de chlorhydrate de 2-chloroéthylmorpholine dans 15 ml de DMF. Après refroidissement à TA, on verse le mélange réactionnel-dans une solution saturée de NaCl, essore le précipité formé et le lave à l'eau. On reprend le précipité dans du MeOH et concentre sous vide le solvant. On triture le résidu dans un mélange propan-2-ol/EP (50/50 ; v/v) et essore le précipité formé. On obtient 0,3 g du composé attendu.

RMN$^1$H : DMSO-$d_6$ (300 MHz) : δ (ppm) : 2,35-2,42 : m : 7H ; 2,73 : t : 2H ; 3,42-3,44 : m : 4H ; 4,01 : s : 3H ; 4,75-4,79 : m : 2H ; 7,43-7,51 : m : 2H ; 7,69 : d : 1H ; 7,87 : d : 1H ; 8;00 : dd : 1H ; 8,46 : s : 1H ; 8,70 : d : 1H.

**EXEMPLE 17 :** Composé N° 53

3-(2,4-Dichlorophényl)-6-(N-hydroxyéthanimidoyl)-1,9-diméthyl-1,9-dihydro-2*H*-pyrido[2,3-b]indol-2-one.

A une solution de 0,28 g du composé de la Préparation 3.31 dans 30 ml d'EtOH, on ajoute 0,15 g de chlorhydrate d'hydroxylamine puis 0,3 g de $K_2CO_3$ et chauffe à reflux pendant 3 heures. Après refroidissement à TA, on ajoute de l'eau et essore le précipité formé. On dissout le précipité dans un mélange DCM/MeOH et chromatographie sur gel de silice en éluant par le mélange DCM/MeOH (98/02 ; v/v) On obtient 0,13 g du composé attendu.

RMN$^1$H : DMSO-$d_6$ (300 MHz) : δ (ppm) : 2,23 : s : 3H ; 4,02 : s : 3H ; 4,17 : s : 3H ; 7,43-7,50 : m : 2H ; 7,59-7,70 : m : 3H ; 8,17 : s : 1H ; 8,30 : s : 1H ; 11,06: s : 1H.

**EXEMPLE 18** : Composé N° 55

6-(5-Amino-1,3,4-oxadiazol-2-yl)-1,9-diméthyl-3-phényl-1,9-dihydro-2*H*-pyrido [2,3-b]indol-2-one.

A une solution de 1,5 g du composé de la Préparation 3.32 dans 30 ml de MeOH anhydre on ajoute 0,46 g de bromure de cyanogène et chauffe à reflux pendant 2 heures 30 minutes. On verse le mélange réactionnel dans l'eau, alcalinise par ajout d'une solution de $NH_4OH$ à 28 % et essore le précipité formé. On reprend le précipité dans un mélange MeOH/AcOEt/pyridine, filtre l'insoluble et chromatographie le filtrat sur gel de silice en éluant par le mélange AcOEt/MeOH (90/10 ; v/v). On obtient 0,5 g du composé attendu, F = 308-309°C.

RMN$^1$H : DMSO-$d_6$ (300 MHz) : δ (ppm) : 4,03 : s : 3H ; 4,18 : s : 3H ; 7,13 : s : 2H ; 7,29-7,43 : m : 3H ; 7,72-7,80 : m : 4H ; 7,40 : s : 1H ; 8,54: m : 1H.

**EXEMPLE 19** : Composé N° 58 3-(2,4-Dichlorophényl)-1,9-diméthyl-6-(5-méthyl-1,3,4-oxadiazol-2-yl)-1,9-dihydro-2*H*-pyrido[2,3-b]indol-2-one.

A une suspension de 0,2 g du composé obtenu à la Préparation 3.33 et 2 ml d'orthoacétate de triéthyle, on ajoute une quantité catalytique d'acide para-toluènesulfonique et chauffe à 140°C pendant une nuit. Après refroidissement à TA, on essore le précipité formé et le lave à l'eau. On reprend le précipité dans un mélange propan-2-ol/EP (50/50 ; v/v), triture et essore. On obtient 0,13 g du composé attendu, F = 311-316°C.

RMN$^1$H : DMSO-$d_6$ (300 MHz) : δ (ppm) : 2,58 : s : 3H ; 4,03 : s : 3H ; 4,22 : s : 3H ; 7,43-7,51 : m : 2H ; 7,69 : se : 1H ; 7,81-7,91 : m : 2H ; 8,44 : s : 1H ; 8,55: se : 1H.

**EXEMPLE 20** : Composé N° 59

3-(2,4-Dichlorophényl)-1,9-diméthyl-6-(5-méthyl-1,2,4-oxodiazol-3-yl)-1,9-dihydro-2*H*-pyrido[2,3-b]indol-2-one.

On chauffe à reflux pendant 2 heures un mélange de 0,4 g du composé de la Préparation 3.35, 10 ml d'anhydride acétique et 1 ml d'acide acétique. Après refroidissement à TA, on essore le précipité formé et le lave avec un mélange AcOEt/MeOH (50/50 ; v/v). On obtient 0,24 g du composé attendu, F = 306-307°C.

RMN$^1$H : DMSO-$d_6$ (300 MHz) : δ (ppm) : 2,67 : s : 3H ; 4,02 : s : 3H ; 4,20 : s : 3H ; 7,43-7,51 : m : 2H ; 7,69 : d : 1H ; 7,78 : d : 1H ; 7,90 : dd : 1H ; 8,41: s : 1H ; 8,55 : s : 1H.

**EXEMPLE 21** : Composé N° 60

6-(5-Amino-1,2,4-oxodiazol-3-yl)-3-(2,4-dichlorophényl)-1,9-diméthyl-1,9-dihydro-2*H*-pyrido[2,3-b]indol-2-one.

A) 3-(2,4-Dichlorophényl)-1,9-diméthyl-6-[5-(trichlorométhyl)-1,2,4-oxodiazol-3-yl)-1,9-dihydro-2*H*-pyrido[2,3-b]indol-2-one.

A une solution de 0,2 g du composé de la Préparation 3.35.. dans 5 ml de dioxane anhydre et 0,6 ml de NMP on ajoute 0,107 ml de chlorure de trichloroacétyle puis 0,082 ml de pyridine et laisse 1 heure sous agitation à TA. On verse le mélange réactionnel dans l'eau, essore le précipité formé, le lave à l'eau puis au toluène et à l'éther. On obtient 0,19 g du composé attendu.

B) 6-(5-Amino-1,2,4-oxodiazol-3-yl)-3-(2,4-dichlorophényl)-1,9-diméthyl-1,9-dihydro-2*H*-pyrido[2,3-b]indol-2-one.

On refroidit à -78°C 5 ml de THF anhydre, fait barboter de l'ammoniac pendant 5 minutes puis ajoute une solution de 0,19 g du composé obtenu à l'étape précédente dans 5 ml de THF et laisse 2 heures sous agitation en laissant remonter la température à TA. On concentre le mélange réactionnel sous vide, reprend le résidu à l'eau, triture le produit obtenu, essore le précipité formé, le lave avec un mélange propan-2-ol/EP (50/50 ; v/v) puis à l'éther. On obtient 0,08 g du composé attendu, F = 272-275°C.

RMN$^1$H : DMSO-$d_6$ (300 MHz) : δ (ppm) : 4,03 : s : 3H ; 4,21 : s : 3H ; 7,43-7,51 : m : 2H ; 7,67-7,70 : m : 2H ; 7,82-7,86 : m : 1H ; 8,26: s : 1H ; 8,37 : s : 1H ; 12,90 : se : 1H.

**EXEMPLE 22 :** Composé N° 66

3-[3-(2,4-Dichlorophényl)-1,9-diméthyl-2-oxo-2,9-dihydro-1*H*-pyrido[2,3-b]indol-6-yl]-1,2,4-oxodiazole-5-carboxylate d'éthyle.

On refroidit à 0°C une solution de 0,17 g du composé de la Préparation 3.35 dans 2 ml de 1,2-dichloroéthane et 0,1 ml de pyridine, ajoute 0,09 ml de chlorure d'éthyloxalyle et laisse 30 minutes sous agitation en laissant remonter la température à TA. On chauffe ensuite à 80°C pendant 2 heures. Après refroidissement à TA, on essore le précipité formé. On dissout le précipité dans du DCM et du MeOH et chromatographie sur gel de silice en éluant par le mélange AcOEt/MeOH (95/05 ; v/v). On obtient 0,14 g du composé attendu.

Le tableau qui suit illustre les structures chimiques et les propriétés physiques de quelques exemples de composés selon l'invention. Dans ce tableau, Me représente méthyle.

**TABLEAU 3**

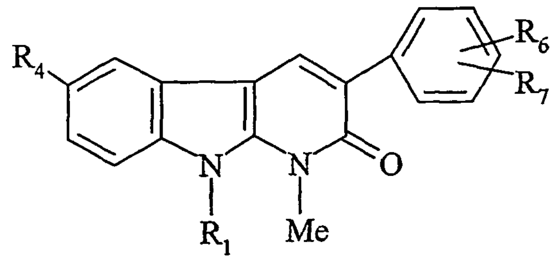

(I) : $R_8 = H$
$R_5 = H$

| Composés N° | $R_1$ | $R_6$, $R_7$ | $R_4$ | Caractérisation |
|---|---|---|---|---|
| 1 | H | H | -CH=N-OH | F = 255°C |
| 2 | Me | 2,4-diCl | 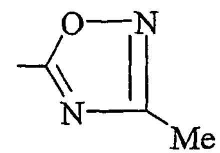 | F = 286°C |
| 3 | H | 2,4-diCl | 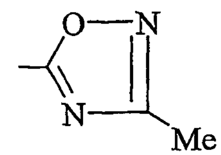 | F = 279°C |
| 4 | Me | 2,4-diMe | 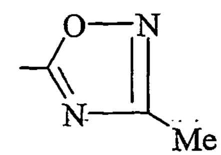 | RMN |
| 5 | Me | 2,4-diOMe | 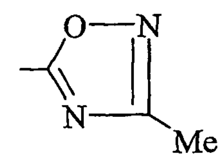 | F = 241°C |

(suite)

| Composés N° | $R_1$ | $R_6$, $R_7$ | $R_4$ | Caractérisation |
|---|---|---|---|---|
| 6 | H | 2,4-diMe | 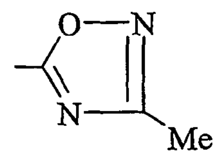 | F = 340°C |
| 7 | H | 2,4-diOMe | -CH=N-OEt | F = 295°C |
| 8 | H | 2,4-diOMe | 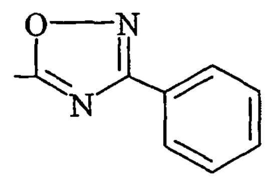 | RMN |
| 9 | Me | H | 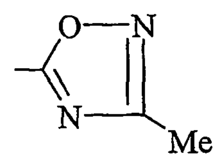 | F = 233°C |
| 10 | Me | 2-Me,5-F | 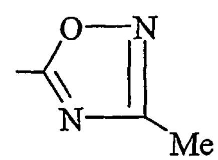 | F = 280°C |
| 11 | Me | 3-F,4-Me | 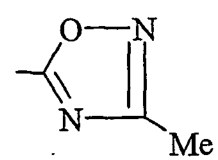 | F = 273°C |

(suite)

| Composés N° | $R_1$ | $R_6$, $R_7$ | $R_4$ | Caractérisation |
|---|---|---|---|---|
| 12 | H | H | 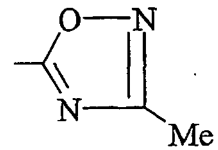 | RMN |
| 13 | H | 2,4-diCl | 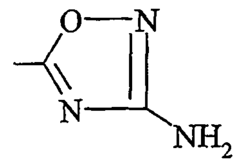 | F = 330°C |
| 14 | Me | 2,4-diCl | 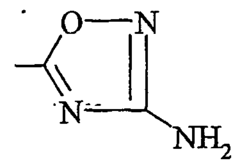 | F = 293°C |
| 15 | Me | 3-Me,4-F | 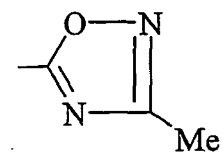 | F = 130°C |
| 16 | H | 3-Me,4-F | 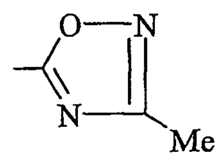 | F = 325°C |
| 17 | H | 3-F,4-Me | 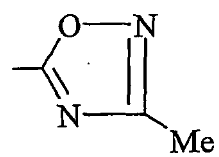 | F = 320°C |

(suite)

| Composés N° | $R_1$ | $R_6$, $R_7$ | $R_4$ | Caractérisation |
|---|---|---|---|---|
| 18 | H | 2-Me,5-F | 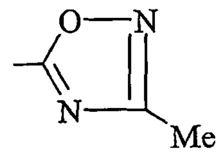 | RMN |
| 19 | Me | 2,4-diCl | 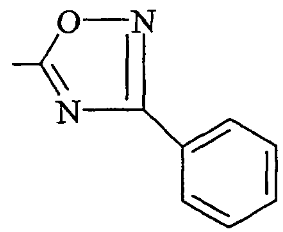 | F = 319°C |
| 20 | Me | 2,4-diCl | -CH=N-OH | F = 287-290°C |
| 21 | Me | 2,4-diCl | -CH=N-O-Et | |
| 22 | Me | 2,4-diCl | $-CH=N-O-CH_2CH(CH_3)_2$ | F = 100-110°C |
| | | | | |
| 23 | Me | 2,4-diCl | $-CH=N-O-CH_2-COOH$ | |
| 24 | Me | 2,4-diCl | $-CH=N-O-CH_2-CO_2Et$ | F = 160°C |
| 25 | Me | 2,4-diCl | 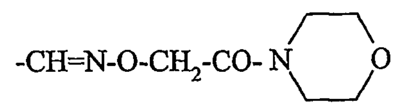 | RMN |
| 26 | Me | - 2,4-diOMe | 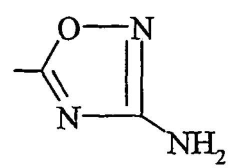 | F = 320-322°C |

(suite)

| Composés N° | $R_1$ | $R_6$, $R_7$ | $R_4$ | Caractérisation |
|---|---|---|---|---|
| 27 | Me | 2,4-diMe | 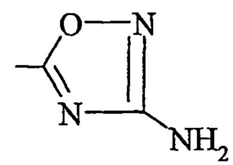 | F = 334°C |
| 28 | Me | 2-Cl | 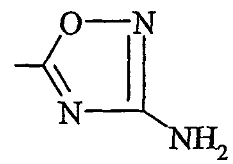 | F=301°C |
| 29 | Me | 4-Cl | 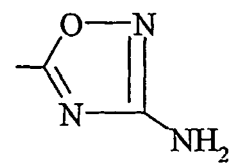 | F = 308-310°C |
| 30 | Me | 3-Br | 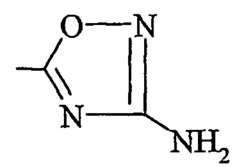 | F = 290°C |
| 31 | Me | 4-Br | 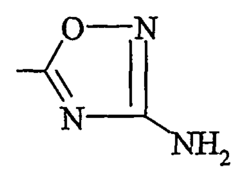 | RMN |

(suite)

| Composés N° | $R_1$ | $R_6$, $R_7$ | $R_4$ | Caractérisation |
|---|---|---|---|---|
| 32 | Me | 3-F | 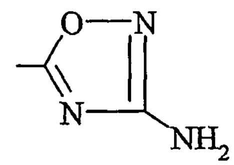 | F = 309°C |
| 33 | Me | 4-F | 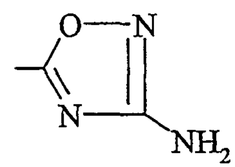 | F = 307-308°C |
| 34 | Me | 3-Me | 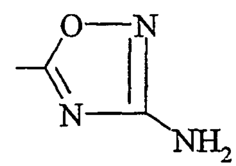 | F = 295-296°C |
| 35 | Me | 3-OMe | 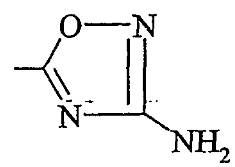 | F = 274-278°C |
| 36 | Me | 4-OMe | 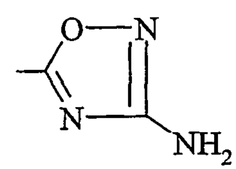 | F = 182-185°C |
| 37 | Me | 3-CN | 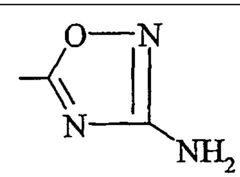 | F = 290-291°C |

(suite)

| Composés N° | $R_1$ | $R_6$, $R_7$ | $R_4$ | Caractérisation |
|---|---|---|---|---|
| 38 | Me | 4-CN | 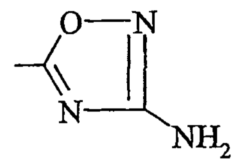 | RMN |
| 39 | Me | 4-$CH_2NH_2$ | 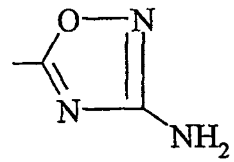 | RMN |
| 40 | Me | 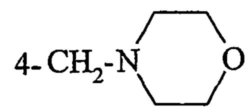 | 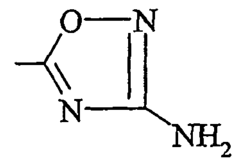 | RMN |
| 41 | Me | 2,4-diCl | 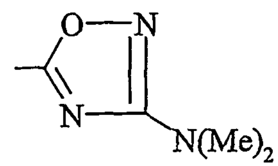 | F = 320-324°C |
| 42 | Me | 2,4-diCl | 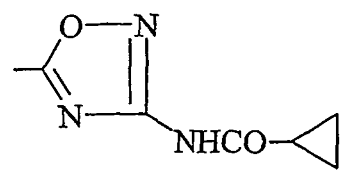 | F = 292-295°C |
| 43 | Me | 2,4-diCl | 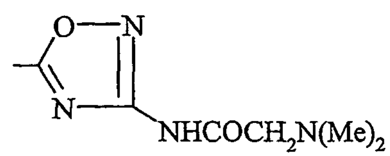 | F = 260°C |

(suite)

| Composés N° | $R_1$ | $R_6$, $R_7$ | $R_4$ | Caractérisation |
|---|---|---|---|---|
| 44 | Me | 2,4-diCl | 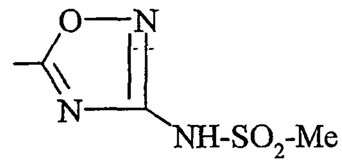 | F = 263-266°C |
| 45 | Me | 3-F | 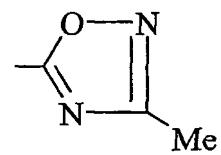 | RMN |
| 46 | Me | 4-$OCH_2CH_2$- 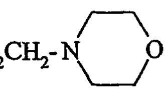 | 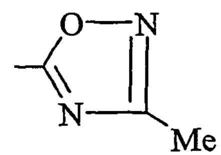 | RMN |
| 47 | Et | 2,4-diCl | 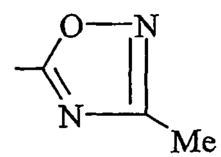 | RMN |
| 48 | Me | 3,5-diF | 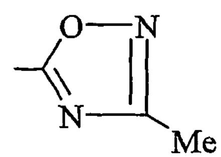 | RMN |

(suite)

| Composés N° | $R_1$ | $R_6$, $R_7$ | $R_4$ | Caractérisation |
|---|---|---|---|---|
| 49 | $-CH_2$ CN | 2,4-diCl | 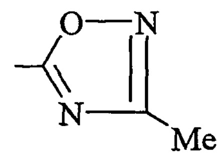 | |
| 50 | 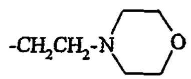 | 2,4-diCl | 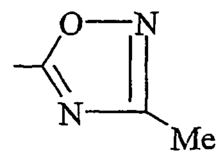 | |
| 51 | Me | 2,4-diCl | 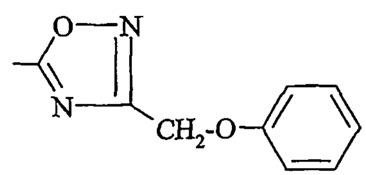 | RMN |
| 52 | Me | 2-OMe | 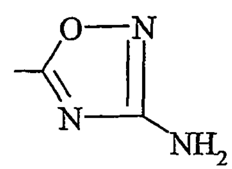 | F = 271-272°C |
| 53 | Me | 2,4-diCl | 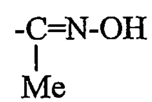 | |
| 54 | Me | 2,4-diCl | 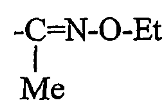 | RMN |

(suite)

| Composés N° | $R_1$ | $R_6$, $R_7$ | $R_4$ | Caractérisation |
|---|---|---|---|---|
| 55 | Me | H | 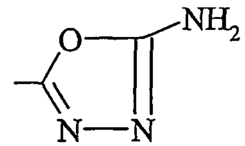 | F = 308-309°C |
| 56 | Me | 2,4-diCl | 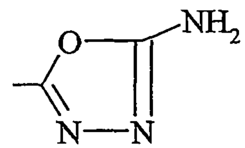 | F = 304-305°C |
| 57 | H | 2,4-diOMe | 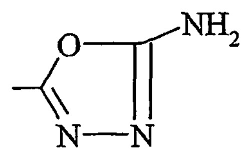 | RMN |
| 58 | Me | 2,4-diCl | 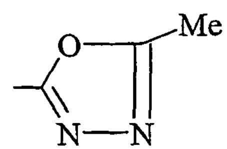 | F = 311-316°C |
| 59 | Me | 2,4-diCl | 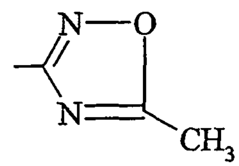 | F = 306-307°C |
| 60 | Me | 2,4-diCl | 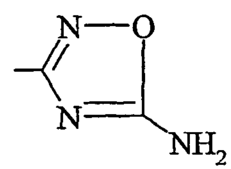 | F = 272-275°C déc |

(suite)

| Composés N° | $R_1$ | $R_6$, $R_7$ | $R_4$ | Caractérisation |
|---|---|---|---|---|
| 61 | Me | 2,4-diCl | 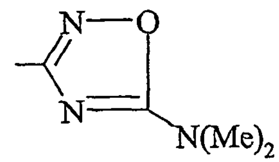 | F = 287-289°C |
| 62 | Me | 2,4-diCl | 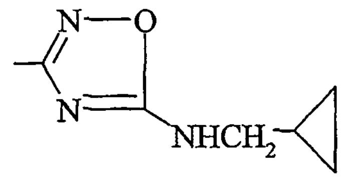 | F = 279-283°C |
| 63 | Me | 2,4-diCl | 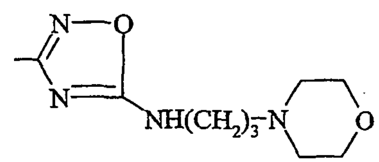 | F = 228-230°C |
| 64 | Me | 2,4-diCl | 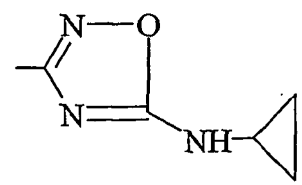 | F = 272-276°C |
| 65 SSR 119557 | Me | 2,4-diCl | 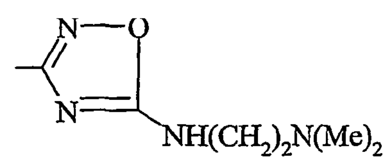 | F = 145-150°C |

(suite)

| Composés N° | $R_1$ | $R_6$, $R_7$ | $R_4$ | Caractérisation |
|---|---|---|---|---|
| 66 | Me | 2,4-diCl | 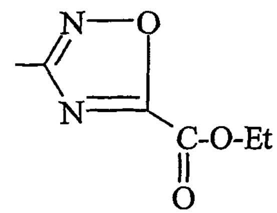 | RMN |
| 67 | Me | 3-Cl | 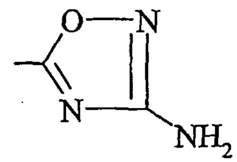 | RMN |

RMN$^1$H : DMSO-$d_6$ (300 MHz) : Composé N° 4 : δ (ppm) : 2,15 : s : 3H ; 2,32 : s : 3H ; 2,41 : s : 3H ; 4,02 : s : 3H ; 4,22 : s : 3H ; 7,01-7,1 : m : 3H ; 7,82 : d : 1H ; 7,96 : dd : 1H ; 8,28 : s : 1H ; 8,68 : s : 1H.

RMN$^1$H : DMSO-$d_6$ (300 MHz) : Composé N° 8 : δ (ppm) : 3,69 : s : 3H ; 3,72 : s : 3H ; 3,81 : s : 3H ; 6,55-6,59 : dd : 1H ; 6,63 : d : 1H ; 7,18 : d : 1H ; 7,55-7,62 : m : 3H ; 7,68 : d : 1H ; 8,01-8,05 : dd : 1H ; 8,10-8,16 : m : 2H ; 8,25 : s : 1H ; 8,73 : d : 1H ; 12,48 : s : 1H.

RMN$^1$H : DMSO-$d_6$ (300 MHz) : Composé N° 12 : δ (ppm): 2,48 : s : 3H ; 3,79 : s : 3H ; 7,37 : d : 1H ; 7,44-7,49 : m : 2H ; 7,69 : d : 1H ; 7,72-7,84 : m : 2H ; 7,99 : d : 1H ; 8,67 : s : 1H ; 8,81 : s : 1H ; 12,6 : se : 1H.

RMN$^1$H : DMSO-$d_6$ (300 MHz) : Composé N° 18 : δ (ppm): 2,17 : s : 3H ; 2,40 : s : 3H ; 3,71 : s : 3H ; 7-7,25 : m : 2H ; 7,6 : d : 1H ; 7,25 : d : 1H ; 7,9 : d : 1H ; 8,75 : s : 1H ; 8,65 : s : 1H.

RMN$^1$H : DMSO-$d_6$ (300 MHz) : Composé N° 25 : δ (ppm): 3,44 : se : 4H ; 3,56 : se : 4H ; 4,01 : s : 3H ; 4,17 : s : 3H ; 4,84 : s : 2H ; 7,42-7,49 : m : 2H ; 7,55 : dd : 1H ; 7,66-7,69 : m : 2H ; 8,10 : d : 1H ; 8,26 : s : 1H ; 8,38 : s : 1H.

RMN$^1$H : DMSO-$d_6$ (200 MHz) : Composé N° 31 : δ (ppm): ; 4,0 : s : 3H ; 4,2 : s : 3H ; 6,3 : s : 2H ; 7,5 : d : 2H; 7,7-8,0 : m : 4H ; 8,65 : d : 2H.

RMN$^1$H : DMSO-$d_6$ (300 MHz) : Composé N° 32 : δ (ppm): 4,04 : s : 3H ; 4,20 : s : 3H ; 6,33 : s : 2H ; 7,12 : t : 1H ; 7,44 : q : 1H ; 7,70 : m : 2H ; 7,80 : d : 1H ; 7,90 : d : 1H ; 8,69 : s : 1H ; 8,77 : s : 1H.

RMN$^1$H : DMSO-$d_6$ (200 MHz) : Composé N° 38 : δ (ppm): ; 4,05 : s : 3H ; 4,15 : s : 3H ; 6,3 : s : 2H ; 7,4 : d : 2H ; 7,75-8,0 : m : 4H ; 8,6 : d : 2H.

RMN$^1$H : DMSO-$d_6$/TFA(200 MHz) : Composé N° 40 : δ (ppm): ; 3,95 : s : 2H ; 3,55-3,7 : m : 8H ; 4,1 : s : 3H ; 4,2 : s : 3H ; 7,55 : d : 2H ; 7,8-7,95 : m : 4H ; 8,6 : d : 2H.

RMN$^1$H : DMSO-$d_6$ (300 MHz) : Composé N° 43 : δ (ppm): 2,30 : s : 6H ; 3,23 : s : 2H ; 4,05 : s : 3H ; 4,25 : s : 3H ; 7,47-7,50 : m : 2H ; 7,72 : s : 1H ; 7,89 : d : 1H ; 7,99 : d : 1H ; 8,39 : s : 1H ; 8,70 :s : 1H ; 10,90 : s : 1H.

RMN$^1$H : DMSO-$d_6$ (300 MHz) : Composé N° 45 : δ (ppm): 2,42 : s : 3H ; 4,04 : s : 3H ; 4,21 : s : 3H ; 7,09-7,15 : m : 1H ; 7,41-7,48 : q : 1H ; 7,71-7,75 : m : 2H ; 7,83 : d : 1H ; 7,97-8,00 : dd : 1H ; 8,06 : s : 2H.

RMN$^1$H : MeOD-$d_4$ (300 MHz) : Composé N° 46 : 5 (ppm): 2,43 : s : 3H ; 2,63 : t : 4H ; 2,84 : t : 2H ; 3,74 : t : 4H ; 4,07 : s : 3H ; 4,19 : t : 5H ; 7,00 : d : 2H ; 7,63 : d : 3H ; 8,00 : d : 1H ; 8,22 : s : 1H ; 8,47 : s : 1H.

RMN$^1$H : DMSO-$d_6$ (300 MHz) : Composé N° 47 : δ (ppm): 1,47 : t : 3H ; 2,42 : s : 3H ; 3,99 : s : 3H ; 4,72 : q : 2H ; 7,43-7,51 : m : 2H ; 7,69 : d : 1H ; 7,86 : d : 1H ; 8,01 : d : 1H ; 8,40 : s : 1H ; 8,71 : s : 1H.

RMN$^1$H : DMSO-$d_6$ (300 MHz) : Composé N° 48 : δ (ppm): 2,42 : s : 3H ; 4,03: s : 3H ; 4,20 : s : 3H ; 7,09-7,16 : m : 1H ; 7,67-7,72 : m : 2H ; 7,82 : d : 1H ; 7,97-8,00 : dd : 1H ; 8,81 : s : 1H ; 8,92 : s : 1H.

RMN$^1$H : DMSO-$d_6$ (300 MHz) : Composé N° 51 : δ (ppm): 4,04: s : 3H ; 4,24 : s : 3H ; 5,35 : s : 2H ; 7,01 : t : 1H ; 7,09 : d : 2H ; 7,34 : t : 2H ; 7,43-7,52 : m : 2H ; 7,70 : s : 1H ; 7,87 : d : 1H ; 8,03 : dd : 1H ; 8,49 : s : 1H ; 8,77 : s : 1H.

RMN$^1$H : DMSO-$d_6$ (300 MHz) : Composé N° 54 : δ (ppm): 1,28 : t : 3H ; 2,26 : s : 3H ; 4,02: s : 3H ; 4,15-4,22 : m : 5H ; 7,42-7,47 : m : 2H ; 7,60-7,70 : m : 3H ; 8,20 : s : 1H ; 8,32 : s : 1H.

RMN$^1$H : DMSO-$d_6$ (300 MHz) : Composé N° 57 : δ (ppm): 3,67 : s : 3H ; 3,71 : s : 3H ; 3,80: s : 3H ; 6,54-6,57 : dd : 1H ; 6,62 : d : 1H ; 7,09 : s : 2H ; 7,17 : d : 1H ; 7,58 : d : 1H ; 7,65-7,69 : dd : 1H ; 8,12 : s : 1H ; 8,21 : s : 1H ; 12,24 : s : 1H.

RMN$^1$H : DMSO-d6 (300 MHz) : Composé N° 66 : δ (ppm): 1,37 : t : 3H ; 4,02: s : 3H ; 4,21 : s : 3H ; 4,47 : q : 2H ; 7,44-7,47 : m : 2H ; 7,69 : d : 1H ; 7,82 : d : 1H ; 7,96 : dd : 1H ; 8,48 : s : 1H ; 8,64 : s : 1H.

RMN$^1$H : DMSO-$d_6$ (300 MHz) : Composé N° 67 : δ (ppm): 4,0 : s : 3H ; 4,2: s : 3H ; 6,33 : s : 2H ; 7,45-7,9 : m : 6H ; 8,6 : s : 1H ; 8,7 : s : 1H.

Les composés de formule (I) selon la présente invention ont été testés *in vitro* sur une lignée cellulaire humaine de cancer du sein : la lignée MDA-MB-231 disponible auprès de l'American Type Culture Collection (référence HTB26).

L'évaluation de l'effet antiprolifératif est effectuée selon J.M. Derocq et al., FEBS Letters, 1998, 425, 419-425 : on mesure le taux d'incorporation de la [3H]thymidine dans l'ADN des cellules traitées, après 96 heures d'incubation d'un composé de formule (I). La concentration inhibitrice 50 ($CI_{50}$) est définie comme la concentration qui inhibe la prolifération cellulaire de 50 %.

Les composés selon l'invention présentent une $CI_{50}$ généralement inférieure à 10 $\mu$M sur la lignée MDA-MB-231.

Les composés de formule (I) ont également été testés sur une autre lignée cellulaire humaine de cancer du sein, dite lignée multi-résistante MDR, (de l'anglais multi-drug-resistant) et appelée MDA-$A_1$. Cette lignée est décrite par E. Collomb, C. Dussert et P.M. Martin dans Cytometry, 1991, 12(1), 15-25.

Le terme "multi-résistant" qui qualifie cette lignée, signifie que ladite lignée est peu sensible d'une manière générale aux drogues de chimiothérapie communément utilisée et en particulier aux antimitotiques d'origine naturelle tels que le paclitaxel, la vincristine, la vinblastine.

Les composés selon l'invention présentent une $CI_{50}$ généralement inférieure à 10 $\mu$M sur la lignée multi-résistante MDA-$A_1$.

Ainsi, selon la présente invention, il apparaît que les composés de formule (I) inhibent la prolifération des cellules tumorales y compris celle des cellules présentant une multi-résistance. Il apparaît donc que les composés selon l'invention ont une activité anticancéreuse.

Ainsi selon un autre de ses aspects, l'invention a pour objet des médicaments qui comprennent un composé de formule (I), ou un sel d'addition de ce dernier à un acide pharmaceutiquement acceptable ou encore un hydrate ou un solvat du composé de formule (I).

Ces médicaments trouvent leur emploi en thérapeutique, notamment dans le traitement ou la prévention des maladies causées ou exacerbées par la prolifération des cellules tumorales.

Comme inhibiteur de la prolifération des cellules tumorales, ces composés sont utiles dans le traitement de tous types de néplasmes, de toutes origines, qu'ils soient solides ou non, bénins ou malins, primaires ou métastasiques, carcinomes, sarcomes, adénomes, adénocarcinomes, en particulier : cancer du sein ; cancer du poumon ; cancer de l'intestin grêle, cancer du colon et du recium ; cancer des voies respiratoires, de l'oropharynx et de fhypopharynx ; cancer de l'oesophage; cancer du foie, cancer de l'estomac, cancer des canaux biliaires, cancer de la vésicule biliaire, cancer du pancréas; cancers des voies urinaires y compris rein, urothelium et vessie; cancers du tractus génital féminin y compris cancer de l'utérus, du col de l'utérus, des ovaires, chloriocarcinome et trophoblastome; cancers du tractus génital masculin y compris cancer de la prostate, des vésicules séminales, des testicules, tumeurs des cellules germinales; cancers des glandes endocrines y compris cancer de la thyroïde, de l'hypophyse, des glandes surrénales ; cancers de la peau y compris hémangiomes, mélanomes, sarcomes, incluant le sarcome de Kaposi ; tumeurs du cerveau, des nerfs, des yeux, des méninges, incluant astrocytomes, gliomes, glioblastomes, rétinoblastomes, neurinomes, neuroblastomes, schwannomes, méningiomes ; tumeurs venant de tumeurs malignes hématopoïétiques incluant leucémies, chloromes, plasmacytomes, mycosis fongoïde, lymphome ou leucémie des cellules T, lymphome non hodgkinien, hémopathies malignes, myélomes.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

Les composés de formule (I) ci-dessus peuvent être utilisés à des doses journalières de 0,002 à 2000 mg par kilogramme de poids corporel du mammifère à traiter, de préférence à des doses journalières de 0,1 à 300 mg/kg. Chez l'être humain, la dose peut varier de préférence de 0,02 à 10000 mg par jour, plus particulièrement de 1 à 3000 mg

selon l'âge du sujet à traiter ou le type de traitement : prophylactique ou curatif.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

Selon la présente invention, le ou les composés de formule (I) peuvent être administrés en association avec un (ou plusieurs) principe(s) actif(s) anticancéreux, en particulier des composés antitumoraux tels que les agents alkylants tels que les alkylsulfonates (busulfan), la dacarbazine, la procarbazine, les moutardes azotées (chlorméthine, melphalan, chlorambucil), cyclophosphamide, ifosfamide; les nitrosourées tels que la carmustine, la lomustine, la sémustine, la streptozocine; les alcaloïdes antinéoplasiques tels que la vincristine, la vinblastine ; les taxanes tel que le paclitaxel ou le taxotère ; les antibiotiques antinéoplasiques tels que l'actinomycine; les agents intercalants, les antimétabolites antinéoplasiques, les antagonistes des folates, le méthotrexate ; les inhibiteurs de la synthèse des purines; les analogues de la purine tels que mercaptopurine, 6-thioguanine; les inhibiteurs de la synthèse des pyrimidines, les inhibiteurs d'aromatase, la capécitabine, les analogues de la pyrimidine tels que fluorouracil, gemcitabine, cytarabine et cytosine arabinoside ; le bréquinar ; les inhibiteurs de topoisomérases tels que la camptothécine ou l'étoposide; les agonistes et antagonistes hormonaux anticancéreux incluant le tamoxifène ; les inhibiteurs de kinase, l'imatinib ; les inhibiteurs de facteurs de croissance ; les antiinflammatoires tels que le pentosane polysulfate, les corticostéroïdes, la prednisone, la dexamethasone, les antracyclines incluant la doxorubicine, la bléomycine, la mitomycine et la méthramycine ; les complexes métalliques anticancéreux, les complexes du platine, le cisplatine, le carboplatine, l'oxaliplatine ; l'interféron alpha, le triphénylthiophosphoramide, l'altrétamine ; les agents antiangiogéniques tel que le bévacizumab ; les inhibiteurs du protéasome tel que le bortézomib ; la thalidomide ; les adjuvants d'immunothérapie ; les vaccins.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

**Revendications**

1. Composé répondant à la formule :

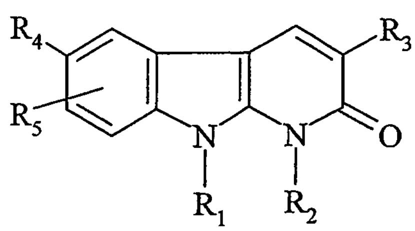

(I)

dans laquelle :

- $R_1$ représente un atome d'hydrogène ; un groupe $(C_1-C_4)$alkyle ; un groupe $-(CH_2)_mOH$; un groupe $-(CH_2)_mCN$ ; un groupe $-(CH_2)_mNR_9R_{10}$;
- $R_2$ représente un atome d'hydrogène ou un groupe $(C_1-C_4)$alkyle;
- $R_3$ représente un phényle substitué par $R_6$, $R_7$, $R_8$ ;
- $R_4$ représente :

. un groupe

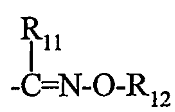 ;

. un radical hétérocyclique choisi parmi :

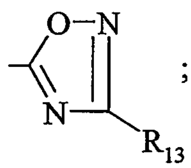 ; 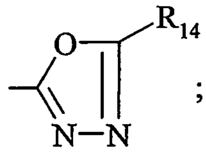 ; 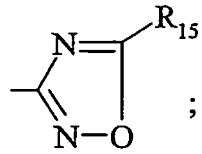 ;

- $R_5$ représente un atome d'hydrogène ou un groupe $(C_1-C_4)$alkyle ;
- $R_6$, $R_7$ et $R_8$ représentent chacun indépendamment l'un de l'autre un atome d'hydrogène ; un atome d'halogène ; un groupe $(C_1-C_4)$alkyle ; un groupe $(C_1-C_4)$alcoxy ; un hydroxy ; un cyano ; un groupe $-(CH_2)_nNR_9R_{10}$ ; un groupe $-O-(CH_2)_mNR_9R_{10}$ ;
- $R_9$ et $R_{10}$ représentent chacun indépendamment l'un de l'autre un atome d'hydrogène ou un groupe $(C_1-C_4)$ alkyle ;
- ou bien $R_9$ et $R_{10}$ ensemble avec l'atome d'azote auquel ils sont liés constituent un radical hétérocyclique choisi parmi la pyrrolidin-1-yle, la pipéridin-1-yle, la morpholin-4-yle ou la pipérazin-1-yle non substitué ou substitué en position -4- par un $(C_1-C_4)$alkyle ;
- $R_{11}$ représente un atome d'hydrogène ou un groupe $(C_1-C_4)$alkyle ;
- $R_{12}$ représente un atome d'hydrogène ; un groupe $(C_1-C_4)$alkyle ; un groupe $-(CH_2)_m-CO-R_{16}$ ;
- $R_{13}$ représente un atome d'hydrogène ; un groupe $(C_1-C_4)$akyle ; un phényle ; un groupe $-NR_{17}R_{18}$ ; un groupe

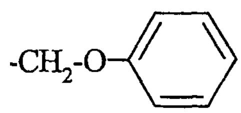 ;

- $R_{14}$ représente un atome d'hydrogène ; un groupe $(C_1-C_4)$alkyle ; un groupe $-NR_{17}R_{18}$ ;
- $R_{15}$ représente un atome d'hydrogène ; un groupe $(C_1-C_4)$alkyle ; un groupe $-NR_{19}R_{20}$ ; un groupe -COO $(C_1-C_4)$alkyle ;
- $R_{16}$ représente un hydroxy ; un $(C_1-C_4)$alcoxy ; un groupe $-NR_9R_{10}$ ;
- $R_{17}$ et $R_{18}$ représentent chacun indépendamment un atome d'hydrogène ou un groupe $(C_1-C_4)$alkyle ; $R_{18}$ peut de plus représenter un groupe $-COR_{21}$ ; un groupe $-SO_2R_{22}$ ;
- $R_{19}$ et $R_{20}$ représentent chacun indépendamment un atome d'hydrogène ou un groupe $(C_1-C_4)$alkyle ; $R_{20}$ peut de plus représenter un groupe $(C_3-C_6)$cycloalkyle, un groupe $(C_3-C_6)$cycloalkylméthyle, un groupe $-(CH_2)_mNR_9R_{10}$;
- $R_{21}$ représente un groupe $(C_1-C_4)$alkyle ; un groupe $(C_3-C_6)$cycloalkyle ; un groupe $-(CH_2)_mNR_9R_{10}$;
- $R_{22}$ représente un groupe $(C_1-C_4)$alkyle ;
- m est 1, 2 ou 3 ;
- n est 0, 1, 2 ou 3.

à l'état de base ou de sel d'addition à un acide, ainsi qu'à l'état d'hydrate ou de solvat.

**2.** Composé selon la revendication 1, **caractérisé en ce que** :

- $R_1$ représente un atome d'hydrogène, un méthyle, un éthyle, un cyanométhyle, un 2-morpholin-4-yléthyle ;
- $R_2$ représente un méthyle ;
- $R_3$ représente un phényle, un 3-bromophényle, un 4-bromophényle, un 2-chlorophényle, un 3-chlorophényle, un 4-chlorophényle, un 3-fluorophényle, un 4-fluorophényle, un 3-méthylphényle, un 2-méthoxyphényle, un 3-méthoxyphényle, un 4-méthoxyphényle, un 3-cyanophényle, un 4-cyanophényle, un 2,4-dichlorophényle, un 3,5-difluorophényle, un 2,4-diméthylphényle, un 2,4-diméthoxyphényle, un 2-méthyl-5-fluorophényle, un 3-fluoro-4-méthylphényle, un 3-méthyl-4-fluorophényle, un 4-(aminométhyl)phényle, un 4-(morpholin-4-ylméthyl) phényle, un 4-(2-morpholin-4-yléthoxy)phényle ;
- $R_4$ représente :

. un groupe (hydroxyimino)méthyle, un groupe N-hydroxyéthanimidoyle, un groupe (éthoxyimino)méthyle, un groupe N-éthoxyéthanimidoyle, un groupe (isobutoxyimino)méthyle, un groupe [(carboxyméthoxy)imino] méthyle, un groupe [(2-éthoxy-2-oxoéthoxy)imino]méthyle, un groupe [(2-morpholin-4-yl-2-oxoéthoxy)imino]méthyle ;
. un 3-méthyl-1,2,4-oxadiazol-5-yle, un 3-phényl-1,2,4-oxadiazol-5-yle, un 3-amino-1,2,4-oxadiazol-5-yle, un 3-(diméthylamino)-1,2,4-oxadiazol-5-yle, un 3-[(cyclopropylcarbonyl)amino]-1,2,4-oxadiazol-5-yle, un 3-[(N,N-diméthyl glycyl)amino]-1,2,4-oxadiazol-5-yle, un 3-[(méthylsulfonyl)amino]-1,2,4-oxadiazol-5-yle, un 3-(phénoxyméthyl)-1,2,4-oxadiazol-5-yle ;
. un 5-méthyl-1,3,4-oxadiazol-2-yle, un 5-amino-1,3,4-oxadiazol-2-yle ;
. un 5-méthyl-1,2,4-oxadiazol-3-yle, un 5-amino-1,2,4-oxadiazol-3-yle, un 5-(diméthylamino)-1,2,4-oxadiazol-3-yle, un 5-(cyclopropylamino)-1,2,4-oxadiazol-3-yle, un 5-[(cyclopropylméthyl)amino]-1,2,4-oxadiazol-3-yle, un 5-[(3-morpholin-4-ylpropyl)amino]-1,2,4-oxadiazol-3-yle, un 5-[[2-(diméthyl amino)éthyl]amino]-1,2,4-oxadiazol-3-yle, un 5-(éthoxycarbonyl)-1,2,4-oxadiazol-3-yle ;

- $R_5$ représente un atome d'hydrogène ; à l'état de base ou de sel d'addition à un acide, ainsi qu'à l'état d'hydrate ou de solvat.

**3.** Composé de formule (I), selon la revendication 1 choisi parmi :

- 6-(3-Amino-1,2,4-oxadiazol-5-yl)-3-(2,4-dichlorophényl)-1,9-diméthyl-1,9-dihydro-2*H*-pyrido[2,3-b]indol-2-one.
- 3-(2,4-Dichlorophényl)-1,9-diméthyl-2-oxo-2,9-dihydro-1*H*-pyrido[2,3-b]indole-6-carbaldéhyde oxime ;
- 3-(2,4-Dichlorophényl)-1,9-diméthyl-2-oxo-2,9-dihydro-1*H*-pyrido[2,3-b]indole-6-carbaldéhyde O-éthyloxime ;
- 5-[3-(4-Chlorophényl)-1,9-diméthyl-2,9-dihydro-1*H*-pyrido[2,3-b]indole-6-yl]-1,2,4-oxadiazol-3-amine ;
- 5-[3-(3-Fluorophényl)-1,9-diméthyl-2,9-dihydro-1*H*-pyrido[2,3-b]indole-6-yl]-1,2,4-oxadiazol-3-amine ;
- 5-[1,9-diméthyl-3-(3-méthylphényl)-2,9-dihydro-1*H*-pyrido[2,3-b]indole-6-yl]-1,2,4-oxadiazol-3-amine ;
- 3-[4-(Aminométhyl)phényl]-6-(3-amino-1,2,4-oxadiazol-5-yl)-1,9-diméthyl-1,9-dihydro-2*H*-pyrido[2,3-b] indol-2-one ;
- 5-[1,9-diméthyl-3-[4-(morpholin-4-ylméthyl)phényl]-2,9-dihydro-1*H*-pyrido[2,3-b]indole-6-yl]-1,2,4-oxadiazol-3-amine ;
- 5-[3-(2,4-Dichlorophényl)-1,9-diméthyl-2,9-dihydro-1*H*-pyrido[2,3-b]indole-6-yl]-1,3,4-oxadiazol-2-amine;
- N'-[3-[3-(2,4-Dichlorophényl)-1,9-diméthyl-2,9-dihydro-1*H*-pyrido[2,3-b]indole-6-yl]-1,2,4-oxadiazol-5-yl]-N, N-diméthyléthane-1,2-diamine ;

à l'état de base ou de sel d'addition à un acide, ainsi qu'à l'état d'hydrate ou de solvat.

**4.** Procédé de préparation des composés de formule (I) selon l'une quelconque des revendications 1 à 3 dans laquelle $R_4$ représente un groupe $-CR_{11} = N-O-R_{12}$, **caractérisé en ce que** :

on fait réagir un composé de formule :

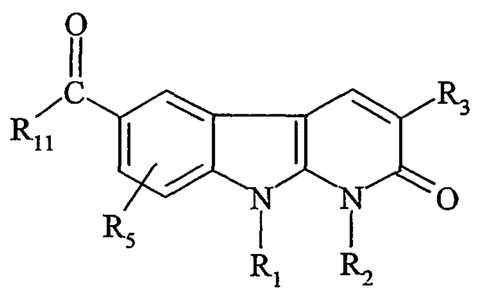

(II)

dans laquelle $R_1$, $R_2$, $R_3$, $R_5$ et $R_{11}$ sont tels que définis pour un composé de formule (I) dans la revendication 1, avec un dérivé d'hydroxylamine de formule :

$H_2N-O-R_{12}$ (III)

dans laquelle $R_{12}$ est tel que défini pour un composé de formule (I) dans la revendication 1.

**5.** Procédé de préparation des composés de formule (I) selon l'une quelconque des revendications 1 à 3, dans laquelle

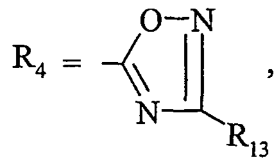

**caractérisé en ce que** :
on fait réagir un composé de formule :

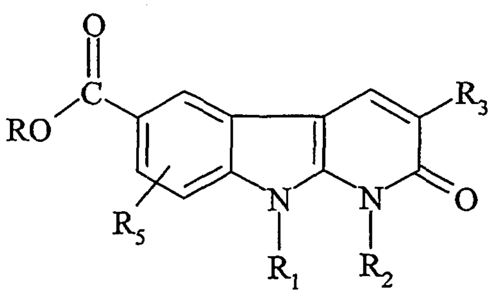

(IV)

dans laquelle $R_1$, $R_2$, $R_3$ et $R_5$ sont tels que définis pour un composé de formule (I) dans la revendication 1 et R représente un atome d'hydrogène ou un groupe $(C_1$-$C_4)$alkyle, avec un dérivé d'oxime de formule :

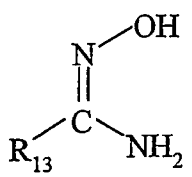

(V)

dans laquelle $R_{13}$ est tel que défini pour un composé de formule (I) dans la revendication 1.

**6.** Procédé de préparation des composés de formule (I) selon l'une quelconque des revendications 1 à 3 dans laquelle

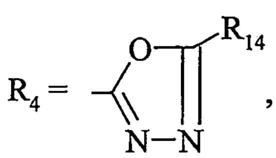

**caractérisé en ce que** :
on cyclise un composé de formule :

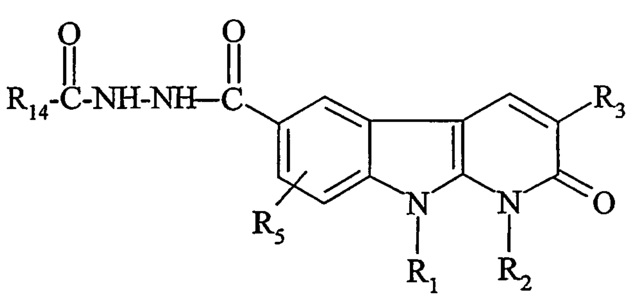

(IX)

dans laquelle $R_1$, $R_2$, $R_3$, $R_5$ et $R_{14}$ sont tels que définis pour un composé de formule (I) dans la revendication 1.

**7.** Procédé de préparation des composés de formule (I) selon l'une quelconque des revendications 1 à 3 dans laquelle

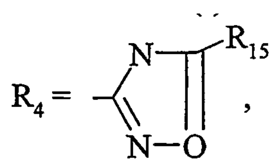

ractérisé en ce que :
on fait réagir un composé de formule :

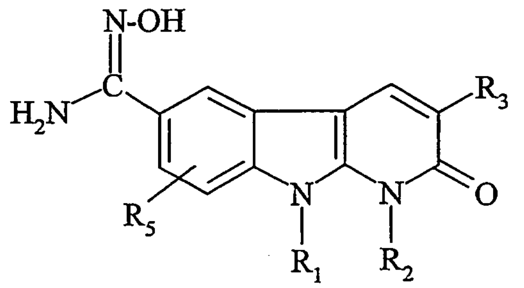
(XI)

dans laquelle $R_1$, $R_2$, $R_3$ et $R_5$ sont tels que définis pour un composé de formule (I) dans la revendication 1, avec :

a) -soit le chlorure de trichloroacétyle, en présence d'une base, pour obtenir un composé de formule :

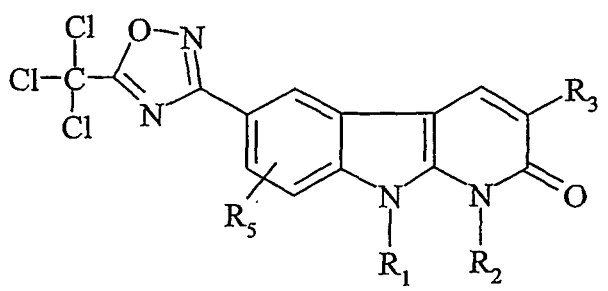
(XII)

et on fait réagir le composé de formule (XII) ainsi obtenu avec une amine de formule $HNR_{19}R_{20}$, lorsqu'on doit préparer un composé de formule (I) dans laquelle $R_{15} = NR_{19}R_{20}$;
b) - soit un anhydride de formule $(R_{15}CO)_2O$, lorsqu'on doit préparer un composé de formule (I) dans laquelle $R_{15} = (C_1\text{-}C_4)$alkyle ;
c) - soit un dérivé de l'acide oxalique de formule

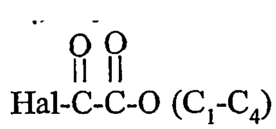

alkyle dans laquelle Hal représente un atome d'halogène, lorsqu'on doit préparer un composé de formule (I) dans laquelle $R_{15} = COO(C_1\text{-}C_4)$alkyle.

**8.** Composé de formule :

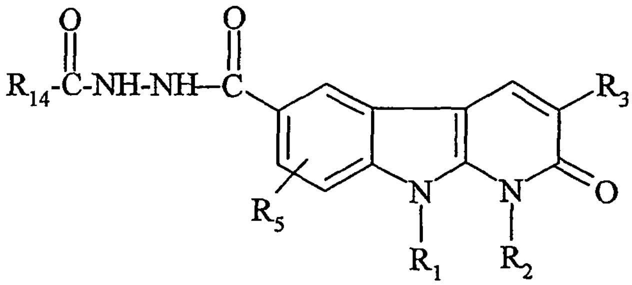

(IX)

dans laquelle :

$R_1$ représente un atome d'hydrogène ; un groupe $(C_1-C_4)$alkyle ; un groupe $-(CH_2)_mOH$ ; un groupe $-(CH_2)_mCN$ ; un groupe $-(CH_2)_mNR_9R_{10}$ ;
$R_2$ représente un atome d'hydrogène ou un groupe $(C_1-C_4)$alkyle ;
$R_3$ représente un phényle substitué par $R_6$, $R_7$, $R_8$ ;

- $R_5$ représente un atome d'hydrogène ou un groupe $(C_1-C_4)$alkyle ;
- $R_6$, $R_7$ et $R_8$ représentent chacun indépendamment l'un de l'autre un atome d'hydrogène ; un atome d'halogène ; un groupe $(C_1-C_4)$alkyle ; un groupe $(C_1-C_4)$alcoxy ; un hydroxy ; un cyano ; un groupe $-(CH_2)_nNR_9R_{10}$ ; un groupe $-O-(CH_2)_mNR_9R_{10}$ ;
- $R_9$ et $R_{10}$ représentent chacun indépendamment l'un de l'autre un atome d'hydrogène ou un groupe $(C_1-C_4)$alkyle ;
- ou bien $R_9$ et $R_{10}$ ensemble avec l'atome d'azote auquel ils sont liés constituent un radical hétérocyclique choisi parmi la pyrrolidin-1-yle, la pipéridin-1-yle, la morpholin-4-yle ou la pipérazin-1-yle non substitué ou substitué en position -4- par un $(C_1-C_4)$alkyle ;
- $R_{14}$ représente un atome d'hydrogène ; un groupe $(C_1-C_4)$alkyle ; un groupe $-NR_{17}R_{18}$
- $R_{17}$ et $R_{18}$ représentent chacun indépendamment un atome d'hydrogène ou un groupe $(C_1-C_4)$alkyle ; $R_{18}$ peut de plus représenter un groupe $-COR_{21}$ ; un groupe $-SO_2R_{22}$ ;
- $R_{21}$ représente un groupe $(C_1-C_4)$alkyle ; un groupe $(C_3-C_6)$cycloalkyle ; un groupe $-(CH_2)_mNR_9R_{10}$;
- $R_{22}$ représente un groupe $(C_1-C_4)$alkyle ;
- m est 1, 2 ou 3 ;
- n est 0, 1, 2 ou 3.

**9.** Composé de formule :

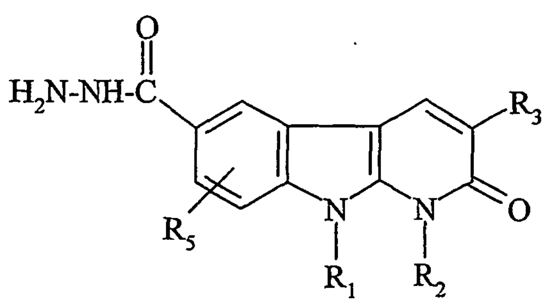

(X)

dans laquelle :

$R_1$ représente un atome d'hydrogène ; un groupe $(C_1-C_4)$alkyle ; un groupe $-(CH_2)_mOH$; un groupe $-(CH_2)_mCN$ ; un groupe $-(CH_2)_mNR_9R_{10}$ ;
$R_2$ représente un atome d'hydrogène ou un groupe $(C_1-C_4)$alkyle ;
$R_3$ représente un phényle substitué par $R_6$, $R_7$, $R_8$ ;

- $R_5$ représente un atome d'hydrogène ou un groupe $(C_1-C_4)$alkyle ;
- $R_6$, $R_7$ et $R_8$ représentent chacun indépendamment l'un de l'autre un atome d'hydrogène ; un atome d'halogène ; un groupe $(C_1-C_4)$alkyle ; un groupe $(C_1-C_4)$alcoxy ; un hydroxy ; un cyano ; un groupe

$-(CH_2)_nNR_9R_{10}$ ; un groupe $-O-(CH_2)_mNR_9R_{10}$;
- $R_9$ et $R_{10}$ représentent chacun indépendamment l'un de l'autre un atome d'hydrogène ou un groupe $(C_1-C_4)$alkyle ;
- ou bien $R_9$ et $R_{10}$ ensemble avec l'atome d'azote auquel ils sont liés constituent un radical hétérocyclique choisi parmi la pyrrolidin-1-yle, la pipéridin-1-yle, la morpholin-4-yle ou la pipérazin-1-yle non substitué ou substitué en position -4- par un $(C_1-C_4)$alkyle ;
- m est 1, 2 ou 3 ;
- n est 0, 1, 2 ou 3.

**10.** Composé de formule :

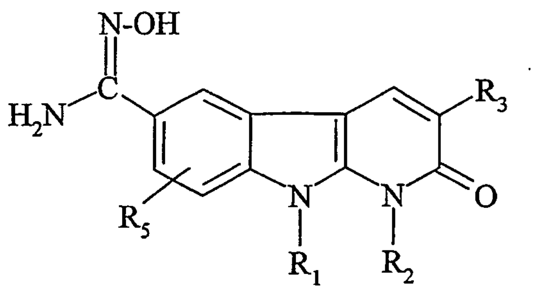

(XI)

dans laquelle :

- $R_1$ représente un atome d'hydrogène ; un groupe $(C_1-C_4)$alkyle ; un groupe $-(CH_2)_mOH$ ; un groupe $-(CH_2)_mCN$ ; un groupe $-(CH_2)_mNR_9R_{10}$ ;
- $R_2$ représente un atome d'hydrogène ou un groupe $(C_1-C_4)$alkyle ;
- $R_3$ représente un phényle substitué par $R_6$, $R_7$, $R_8$ ;
- $R_5$ représente un atome d'hydrogène ou un groupe $(C_1-C_4)$alkyle ;
- $R_6$, $R_7$ et $R_8$ représentent chacun indépendamment l'un de l'autre un atome d'hydrogène ; un atome d'halogène ; un groupe $(C_1-C_4)$alkyle ; un groupe $(C_1-C_4)$alcoxy ; un hydroxy ; un cyano ; un groupe $-(CH_2)_nNR_9R_{10}$ ; un groupe $-O-(CH_2)_mNR_9R_{10}$ ;
- $R_9$ et $R_{10}$ représentent chacun indépendamment l'un de l'autre un atome d'hydrogène ou un groupe $(C_1-C_4)$ alkyle ;
- ou bien $R_9$ et $R_{10}$ ensemble avec l'atome d'azote auquel ils sont liés constituent un radical hétérocyclique choisi parmi la pyrrolidin-1-yle, la pipéridin-1-yle, la morpholin-4-yle ou la pipérazin-1-yle non substitué ou substitué en position -4- par un $(C_1-C_4)$alkyle;
- m est 1, 2 ou 3 ;
- n est 0, 1, 2 ou 3.

**11.** Composé de formule :

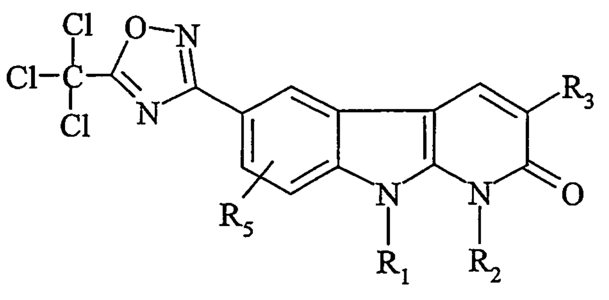

(XII)

dans laquelle :

- $R_1$ représente un atome d'hydrogène ; un groupe $(C_1-C_4)$alkyle ; un groupe $-(CH_2)_mOH$ ; un groupe $-(CH_2)_mCN$ ; un groupe $-(CH_2)_mNR_9R_{10}$ ;

- $R_2$ représente un atome d'hydrogène ou un groupe $(C_1\text{-}C_4)$alkyle ;
- $R_3$ représente un phényle substitué par $R_6$, $R_7$, $R_8$ ;
- $R_5$ représente un atome d'hydrogène ou un groupe $(C_1\text{-}C_4)$alkyle ;
- $R_6$, $R_7$ et $R_8$ représentent chacun indépendamment l'un de l'autre un atome d'hydrogène ; un atome d'halogène ; un groupe $(C_1\text{-}C_4)$alkyle ; un groupe $(C_1\text{-}C_4)$alcoxy ; un hydroxy ; un cyano ; un groupe $-(CH_2)_nNR_9R_{10}$ ; un groupe $-O-(CH_2)_mNR_9R_{10}$ ;
- $R_9$ et $R_{10}$ représentent chacun indépendamment l'un de l'autre un atome d'hydrogène ou un groupe $(C_1\text{-}C_4)$ alkyle ;
- ou bien $R_9$ et $R_{10}$ ensemble avec l'atome d'azote auquel ils sont liés constituent un radical hétérocyclique choisi parmi la pyrrolidin-1-yle, la pipéridin-1-yle, la morpholin-4-yle ou la pipérazin-1-yle non substitué ou substitué en position -4- par un $(C_1\text{-}C_4)$alkyle ;
- ni est 1, 2 ou 3 ;
- n est 0, 1, 2 ou 3.

**12.** Médicament, **caractérisé en ce qu'**il comprend un composé de formule (I) selon l'une quelconque des revendications 1 à 3, ou un sel d'addition de ce composé à un acide pharmaceutiquement acceptable, ou encore un hydrate ou un solvat du composé de formule (I).

**13.** Composition pharmaceutique, **caractérisée en ce qu'**elle comprend un composé de formule (I) selon l'une quelconque des revendications 1 à 3, ou un sel pharmaceutiquement acceptable, un hydrate ou un solvat de ce composé, ainsi qu'au moins un excipient pharmaceutiquement acceptable.

**14.** Utilisation d'un composé de formule (I) selon l'une quelconque des revendications 1 à 3, pour la préparation d'un médicament destiné au traitement et à la prévention de maladies causées ou exacerbées par la prolifération des cellules tumorales.

## Claims

**1.** Compound conforming to the formula

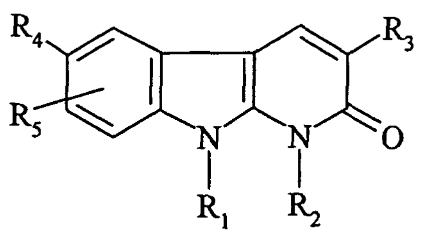 , (I)

in which

- $R_1$ represents a hydrogen atom; a $(C_1\text{-}C_4)$alkyl group; a group $-(CH_2)_mOH$; a group $-(CH_2)_mCN$; or a group $-(CH_2)_mNR_9R_{10}$;
- $R_2$ represents a hydrogen atom or a $(C_1\text{-}C_4)$alkyl group;
- $R_3$ represents a phenyl substituted by $R_6$, $R_7$, $R_8$;
- $R_4$ represents

. a group

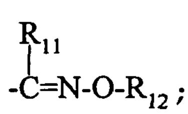

or

. a heterocyclic radical selected from

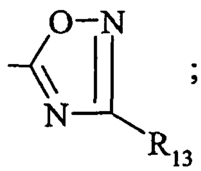 ; 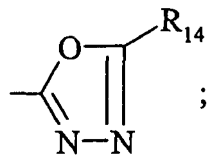 ; 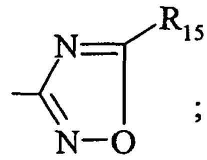 ;

- $R_5$ represents a hydrogen atom or a ($C_1$-$C_4$) alkyl group;
- $R_6$, $R_7$ and $R_8$ represent, each independently of one another, a hydrogen atom; a halogen atom; a ($C_1$-$C_4$) alkyl group; a ($C_1$-$C_4$)alkoxy group; a hydroxyl; a cyano; a group $-(CH_2)_nNR_9R_{10}$; or a group $-O-(CH_2)_mNR_9R_{10}$;
- $R_9$ and $R_{10}$ represent, each independently of one another, a hydrogen atom or a ($C_1$-$C_4$)alkyl group;
- or else $R_9$ and $R_{10}$, together with the nitrogen atom to which they are bonded, constitute a heterocyclic radical selected from pyrrolidin-1-yl, piperidin-1-yl, morpholin-4-yl or piperazin-1-yl unsubstituted or substituted in position 4 by a ($C_1$-$C_4$)alkyl;
- $R_{11}$ represents a hydrogen atom or a ($C_1$-$C_4$)alkyl group;
- $R_{12}$ represents a hydrogen atom; a ($C_1$-$C_4$)alkyl group; or a group $-(CH_2)_m-CO-R_{16}$;
- $R_{13}$ represents a hydrogen atom; a ($C_1$-$C_4$)alkyl group; a phenyl; a group $-NR_{17}R_{18}$; or a

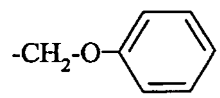

group;
- $R_{14}$ represents a hydrogen atom; a ($C_1$-$C_4$) alkyl group; or a group $-NR_{17}R_{18}$;
- $R_{15}$ represents a hydrogen atom; a ($C_1$-$C_4$) alkyl group; a group $-NR_{19}R_{20}$; or a -COO ($C_1$-$C_4$) alkyl group;
- $R_{16}$ represents a hydroxyl; a ($C_1$-$C_4$)alkoxy; or a group $-NR_9R_{10}$;
- $R_{17}$ and $R_{18}$ represent, each independently, a hydrogen atom or a ($C_1$-$C_4$) alkyl group; $R_{18}$ may also represent a group $-COR_{21}$; or a group $-SO_2R_{22}$;
- $R_{19}$ and $R_{20}$ represent, each independently, a hydrogen atom or a ($C_1$-$C_4$) alkyl group; $R_{20}$ may also represent a ($C_3$-$C_6$) cycloalkyl group, a ($C_3$-$C_6$) cycloalkylmethyl group or a group$-(CH_2)_mNR_9R_{10}$;
- $R_{21}$ represents a ($C_1$-$C_4$) alkyl group; a ($C_3$-$C_6$)cycloalkyl group; or a group $-(CH_2)_mNR_9R_{10}$;
- $R_{22}$ represents a ($C_1$-$C_4$) alkyl group;
- m is 1, 2 or 3; and
- n is 0, 1, 2 or 3

in the form of the base or addition salt with an acid, and in the hydrate or solvate form.

**2.** Compound according to Claim 1, **characterized in that**

- $R_1$ represents a hydrogen atom, a methyl, an ethyl, a cyanomethyl or a 2-morpholin-4-ylethyl;
- $R_2$ represents a methyl;
- $R_3$ represents a phenyl, a 3-bromophenyl, a 4-bromophenyl, a 2-chlorophenyl, a 3-chlorophenyl, a 4-chlorophenyl, a 3-fluorophenyl, a 4-fluorophenyl, a 3-methylphenyl, a 2-methoxyphenyl, a 3-methoxyphenyl, a 4-methoxyphenyl, a 3-cyanophenyl, a 4-cyanophenyl, a 2,4-dichlorophenyl, a 3,5-difluorophenyl, a 2,4-dimethylphenyl, a 2,4-dimethoxyphenyl, a 2-methyl-5-fluorophenyl, a 3-fluoro-4-methylphenyl, a 3-methyl-4-fluorophenyl, a 4-(aminomethyl)phenyl, a 4-(morpholin-4-ylmethyl)phenyl or a 4-(2-morpholin-4-ylethoxy)phenyl;
- $R_4$ represents:

. a (hydroxyimino)methyl group, an N-hydroxyethan-imidoyl group, an (ethoxyimino)methyl group, an N-ethoxyethanimidoyl group, an (isobutoxyimino)-methyl group, a [(carboxymethoxy)imino]methyl group, a [(2-ethoxy-2-oxoethoxy)imino]methyl group or a [(2-morpholin-4-yl-2-oxoethoxy)imino]methyl group;
. a 3-methyl-1,2,4-oxadiazol-5-yl, a 3-phenyl-1,2,4-oxadiazol-5-yl, a 3-amino-1,2,4-oxadiazol-5-yl, a 3-(dimethylamino)-1,2,4-oxadiazol-5-yl, a 3-[(cyclopropylcarbonyl)amino]-1,2,4-oxadiazol-5-yl, a 3-[(N,N-dimethylglycyl)amino]-1,2,4-oxa-diazol-5-yl, a 3[(methylsulphonyl)amino]-1,2,4-oxadiazol-5-yl or a 3-(phenoxymethyl)-1,2,4-oxa-diazol-5-yl;
. a 5-methyl-1,3,4-oxadiazol-2-yl or a 5-amino-1,3,4-oxadiazol-2-yl; or

. a 5-methyl-1,2,4-oxadiazol-3-yl, a 5-amino-1,2,4-oxadiazol-3-yl, a 5-(dimethylamino)-1,2,4-oxadiazol-3-yl, a 5-(cyclopropylamino)-1,2,4-oxa-diazol-3-yl, a 5-[(cyclopropylmethyl)amino]-1,2,4-oxadiazol-3-yl, a 5-[(3-morpholin-4-ylpropyl)-amino]-1,2,4-oxadiazol-3-yl, a 5-[[2-(dimethyl-amino)ethyl]amino]-1,2,4-oxadiazol-3-yl or a 5-(ethoxycarbonyl)-1,2,4-oxadiazol-3-yl;

- $R_5$ represents a hydrogen atom;

in the form of the base or addition salt with an acid, and in the hydrate or solvate form.

**3.** Compound of formula (I), according to Claim 1, selected from

- 6-(3-amino-1,2,4-oxadiazol-5-yl)-3-(2,4-dichlorophenyl)-1,9-dimethyl-1,9-dihydro-2*H*-pyrido[2,3-b]-indol-2-one;
- 3-(2,4-dichlorophenyl)-1,9-dimethyl-2-oxo-2,9-dihydro-1H-pyrido[2,3-b]indole-6-carbaldehyde oxime;
- 3-(2,4-dichlorophenyl)-1,9-dimethyl-2-oxo-2,9-dihydro-1H-pyrido[2,3-b]indole-6-carbaldehyde O-ethyloxime;
- 5-[3-(4-chlorophenyl)-1,9-dimethyl-2,9-dihydro-1H-pyrido[2,3-b]indol-6-yl]-1,2,4-oxadiazol-3-amine;
- 5-[3-(3-fluorophenyl)-1,9-dimethyl-2,9-dihydro-1H-pyrido[2,3-b]indol-6-yl]-1,2,4-oxadiazol-3-amine;
- 5-[1,9-dimethyl-3-(3-methylphenyl)-2,9-dihydro-1*H*-pyrido[2,3-b]indol-6-yl]-1,2,4-oxadiazol-3-amine;
- 3-[4-(aminomethyl)phenyl]-6-(3-amino-1,2,4-oxadiazol-5-yl)-1,9-dimethyl-1,9-dihydro-2*H*-pyrido[2,3-b]indol-2-one;
- 5-[1,9-dimethyl-3-[4-morpholin-4-ylmethyl)phenyl]-2,9-dihydro-1*H*-pyrido[2,3-b]indol-6-yl]-1,2,4-oxa-diazol-3-amine;
- 5-[3-(2,4-dichlorophenyl)-1,9-dimethyl-2,9-dihydro-1*H*-pyrido[2,3-b]indol-6-yl]-1,3,4-oxadiazol-2-amine;
- N'-[3-[3-(2,4-dichlorophenyl)-1,9-dimethyl-2,9-dihydro-1*H*-pyrido[2,3-b]indol-6-yl]-1,2,4-oxadiazol-5-yl]-N,N-dimethylethane-1,2-diamine;

in the form of the base or addition salt with an acid, and in the hydrate or solvate form.

**4.** Process for preparing compounds of formula (I) according to any one of Claims 1 to 3 in which $R_4$ represents a group $-CR_{11}=N-O-R_{12}$, **characterized in that** a compound of formula

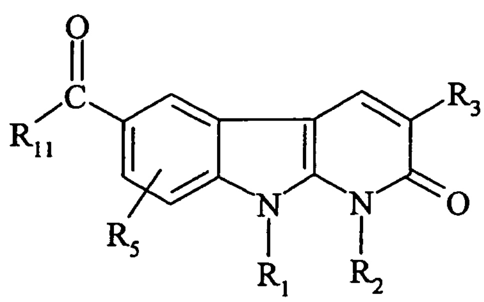

(II)

in which $R_1$, $R_2$, $R_3$, $R_5$ and $R_{11}$ are as defined for a compound of formula (I) in Claim 1 is reacted with a hydroxylamine derivative of formula

$$H_2N-O-R_{12} \qquad (III)$$

in which $R_{12}$ is as defined for a compound of formula (I) in Claim 1.

**5.** Process for preparing compounds of formula (I) according to any one of Claims 1 to 3 in which

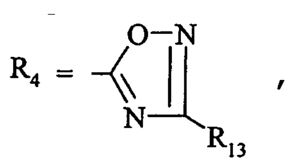

**characterized in that**
a compound of formula

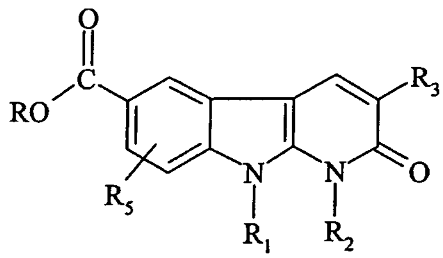

(IV)

in which $R_1$, $R_2$, $R_3$ and $R_5$ are as defined for a compound of formula (I) in Claim 1 and R represents a hydrogen atom or a $(C_1-C_4)$alkyl group is reacted with an oxime derivative of formula

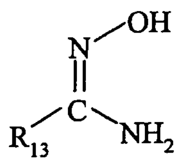

(V)

in which $R_{13}$ is as defined for a compound of formula (I) in Claim 1.

**6.** Process for preparing compounds of formula (I) according to any one of Claims 1 to 3 in which

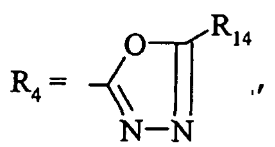

**characterized in that**
a compound of formula

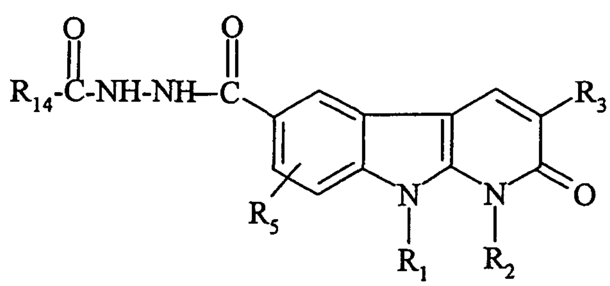

(IX)

in which $R_1$, $R_2$, $R_3$, $R_5$ and $R_{14}$ are as defined for a compound of formula (I) in Claim 1 is cyclized.

**7.** Process for preparing compounds of formula (I) according to any one of Claims 1 to 3 in which

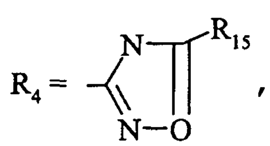

**characterized in that**
a compound of formula

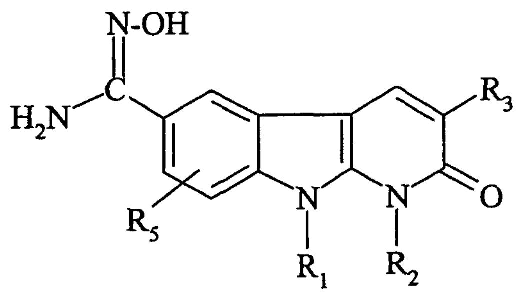

(XI)

in which $R_1$, $R_2$, $R_3$ and $R_5$ are as defined for a compound of formula (I) in Claim 1 is reacted with

a)-alternatively trichloroacetyl chloride, in the presence of a base, to give a compound of formula

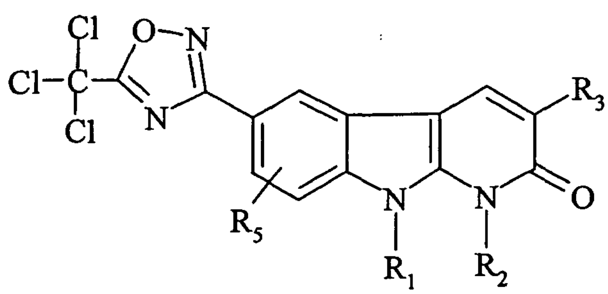

(XII)

and the compound of formula (XII) thus obtained is reacted with an amine of formula $HNR_{19}R_{20}$, when the preparation is required of a compound of formula (I) in which $R_{15} = NR_{19}R_{20}$;
b)- or an anhydride of formula $(R_{15}CO)_2O$, when the preparation is required of a compound of formula (I) in which $R_{15} = (C_1\text{-}C_9)$ alkyl;
c)-or a derivative of oxalic acid of formula

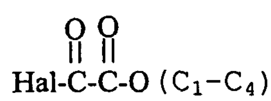

alkyl in which Hal represents a halogen atom, when the preparation is required of a compound of formula (I) in which $R_{15} = COO(C_1\text{-}C_4)$alkyl.

**8.** Compound of formula

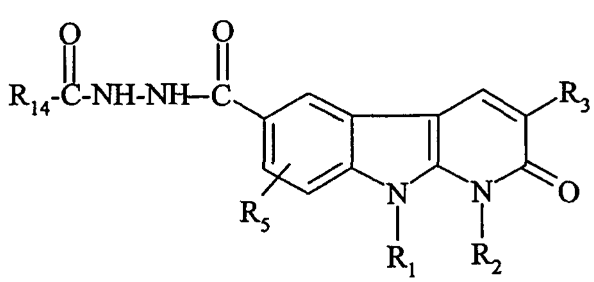

(IX)

in which

- $R_1$ represents a hydrogen atom; a ($C_1$-$C_4$) alkyl group; a group -$(CH_2)_mOH$; a group -$(CH_2)_mCN$; or a group -$(CH_2)_mNR_9R_{10}$;
- $R_2$ represents a hydrogen atom or a ($C_1$-$C_9$) alkyl group;
- $R_3$ represents a phenyl substituted by $R_6$, $R_7$, $R_8$;
- $R_5$ represents a hydrogen atom or a ($C_1$-$C_4$)alkyl group;
- $R_6$, $R_7$ and $R_8$ represent, each independently of one another, a hydrogen atom; a halogen atom; a ($C_1$-$C_4$) alkyl group; a ($C_1$-$C_4$)alkoxy group; a hydroxyl; a cyano; a group - $(CH_2)_nNR_9R_{10}$; or a group -O-$(CH_2)_mNR_9R_{10}$;
- $R_9$ and $R_{10}$ represent, each independently of one another, a hydrogen atom or a ($C_1$-$C_4$)alkyl group;
- or else $R_9$ and $R_{10}$, together with the nitrogen atom to which they are bonded, constitute a heterocyclic radical selected from pyrrolidin-1-yl, piperidin-1-yl, morpholin-4-yl or piperazin-1-yl unsubstituted or substituted in position 4 by a ($C_1$-$C_4$)alkyl;
- $R_{14}$ represents a hydrogen atom; a ($C_1$-$C_4$)alkyl group; or a group -$NR_{17}R_{18}$;
- $R_{17}$ and $R_{18}$ represent, each independently, a hydrogen atom or a ($C_1$-$C_4$)alkyl group; $R_{18}$ may also represent a group -$COR_{21}$; or a group -$SO_2R_{22}$;
- $R_{21}$ represents a ($C_1$-$C_4$)alkyl group; a ($C_3$-$C_6$)cycloalkyl group; or a group -$(CH_2)_mNR_9R_{10}$;
- $R_{22}$ represents a ($C_1$-$C_4$)alkyl group;
- m is 1, 2 or 3; and
- n is 0, 1, 2 or 3.

**9.** Compound of formula

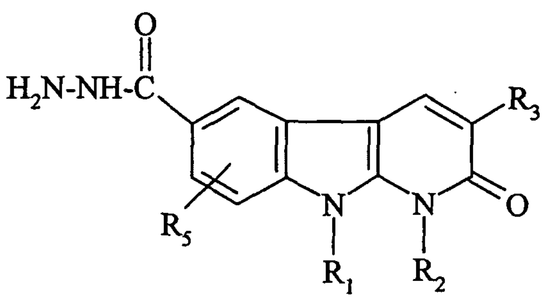

(X)

in which

- $R_1$ represents a hydrogen atom; a ($C_1$-$C_4$) alkyl group; a group -$(CH_2)_mOH$; a group -$(CH_2)_mCN$; or a group -$(CH_2)_mNR_9R_{10}$;
- $R_2$ represents a hydrogen atom or a ($C_1$-$C_4$)alkyl group;
- $R_3$ represents a phenyl substituted by $R_6$, $R_7$, $R_8$;
- $R_5$ represents a hydrogen atom or a ($C_1$-$C_4$)alkyl group;
- $R_6$, $R_7$ and $R_8$ represent, each independently of one another, a hydrogen atom; a halogen atom; a ($C_1$-$C_4$) alkyl group; a ($C_1$-$C_4$)alkoxy group; a hydroxyl; a cyano; a group - $(CH_2)_nNR_9R_{10}$; or a group -O-$(CH_2)_mNR_9R_{10}$;
- $R_9$ and $R_{10}$ represent, each independently of one another, a hydrogen atom or a ($C_1$-$C_4$)alkyl group;
- or else $R_9$ and $R_{10}$, together with the nitrogen atom to which they are bonded, constitute a heterocyclic radical selected from pyrrolidin-1-yl, piperidin-1-yl, morpholin-4-yl or piperazin-1-yl unsubstituted or substituted in position 4 by a ($C_1$-$C_4$)alkyl;
- m is 1, 2 or 3; and
- n is 0, 1, 2 or 3.

**10.** Compound of formula

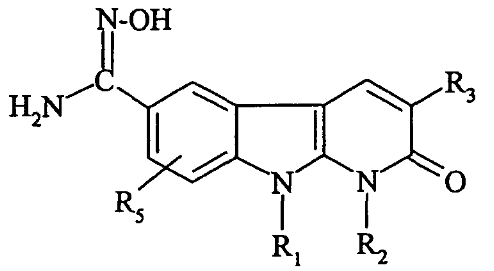

(XI)

in which

- $R_1$ represents a hydrogen atom; a $(C_1-C_4)$alkyl group; a group $-(CH_2)_mOH$; a group $-(CH_2)_mCN$; or a group $-(CH_2)_mNR_9R_{10}$;
- $R_2$ represents a hydrogen atom or a $(C_1-C_4)$alkyl group;
- $R_3$ represents a phenyl substituted by $R_6$, $R_7$, $R_8$;
- $R_5$ represents a hydrogen atom or a $(C_1-C_4)$alkyl group;
- $R_6$, $R_7$ and $R_8$ represent, each independently of one another, a hydrogen atom; a halogen atom; a $(C_1-C_9)$ alkyl group; a $(C_1-C_9)$ alkoxy group; a hydroxyl; a cyano; a group $-(CH_2)_nNR_9R_{10}$; or a group $-O-(CH_2)_mNR_9R_{10}$;
- $R_9$ and $R_{10}$ represent, each independently of one another, a hydrogen atom or a $(C_1-C_4)$alkyl group;
- or else $R_9$ and $R_{10}$, together with the nitrogen atom to which they are bonded, constitute a heterocyclic radical selected from pyrrolidin-1-yl, piperidin-1-yl, morpholin-4-yl or piperazin-1-yl unsubstituted or substituted in position 4 by a $(C_1-C_4)$alkyl;
- m is 1, 2 or 3; and
- n is 0, 1, 2 or 3.

**11.** Compound of formula

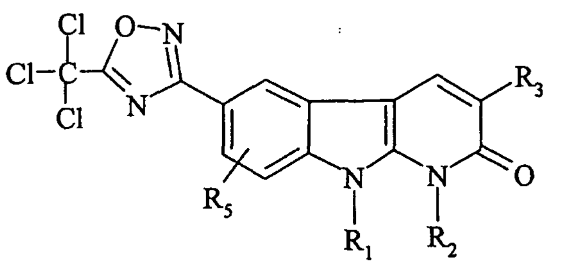

(XII)

in which

- $R_1$ represents a hydrogen atom; a $(C_1-C_4)$alkyl group; a group $-(CH_2)_mOH$; a group $-(CH_2)_mCN$; or a group $-(CH_2)_mNR_9R_{10}$;
- $R_2$ represents a hydrogen atom or a $(C_1-C_4)$alkyl group;
- $R_3$ represents a phenyl substituted by $R_6$, $R_7$, $R_8$;
- $R_5$ represents a hydrogen atom or a $(C_1-C_4)$alkyl group;
- $R_6$, $R_7$ and $R_8$ represent, each independently of one another, a hydrogen atom; a halogen atom; a $(C_1-C_4)$ alkyl group; a $(C_1-C_4)$alkoxy group; a hydroxyl; a cyano; a group $-(CH_2)_nNR_9R_{10}$; or a group $-O-(CH_2)_mNR_9R_{10}$;
- $R_9$ and $R_{10}$ represent, each independently of one another, a hydrogen atom or a $(C_1-C_4)$alkyl group;
- or else $R_9$ and $R_{10}$, together with the nitrogen atom to which they are bonded, constitute a heterocyclic radical selected from pyrrolidin-1-yl, piperidin-1-yl, morpholin-4-yl or piperazin-1-yl unsubstituted or substituted in position 4 by a $(C_1-C_4)$alkyl;
- m is 1, 2 or 3; and
- n is 0, 1, 2 or 3.

**12.** Medicinal product **characterized in that** it comprises a compound of formula (I) according to any one of Claims 1 to 3, or an addition salt of this compound with a pharmaceutically acceptable acid, or else a hydrate or a solvate of

the compound of formula (I).

**13.** Pharmaceutical composition **characterized in that** it comprises a compound of formula (I) according to any one of Claims 1 to 3, or a pharmaceutically acceptable salt, a hydrate or a solvate of this compound, and at least one pharmaceutically acceptable excipient.

**14.** Use of a compound of formula (I) according to any one of Claims 1 to 3 for preparing a medicinal product intended for the treatment and the prevention of diseases caused or exacerbated by the proliferation of tumour cells.

**Patentansprüche**

**1.** Verbindung der Formel:

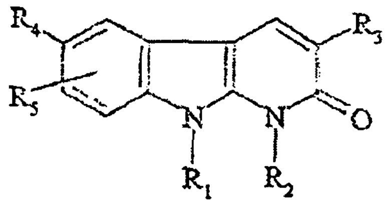

(I)

worin:

- $R_1$ ein Wasserstoffatom, eine $(C_1\text{-}C_4)$-Alkylgruppe, eine Gruppe $-(CH_2)_mOH$, eine Gruppe $-(CH_2)_mCN$, eine Gruppe
- $(CH_2)_mNR_9R_{10}$ darstellt;
- $R_2$ ein Wasserstoffatom oder eine $(C_1\text{-}C_4)$-Alkylgruppe darstellt;
- $R_3$ ein Phenyl darstellt, das mit $R_6$, $R_7$, $R_8$ substituiert ist;
- $R_4$ Folgendes darstellt:

. eine Gruppe

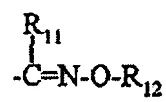

. einen heterocyclischen Rest, ausgewählt aus

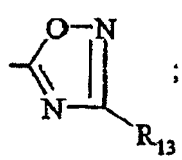 ; 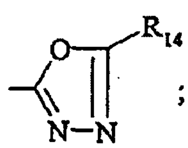 ; 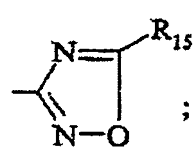 ;

- $R_5$ ein Wasserstoffatom oder eine $(C_1\text{-}C_4)$-Alkylgruppe darstellt;
- $R_6$, $R_7$ und $R_8$, jeweils unabhängig voneinander, ein Wasserstoffatom, ein Halogenatom, eine $(C_1\text{-}C_4)$-Alkylgruppe, eine $(C_1\text{-}C_4)$-Alkoxygruppe; ein Hydroxy; ein Cyano, eine Gruppe $-(CH_2)_nNR_9R_{10}$, eine Gruppe $-O(CH_2)_mNR_9R_{10}$ darstellen;
- $R_9$ und $R_{10}$, jeweils unabhängig voneinander, ein Wasserstoffatom oder eine $(C_1\text{-}C_4)$-Alkylgruppe darstellen;
- oder $R_9$ und $R_{10}$ zusammen mit dem Stickstoffatom, an das sie gebunden sind, einen heterocyclischen Rest bilden, ausgewählt aus Pyrrolidin-1-yl, Piperidin-1-yl, Morpholin-4-yl oder Piperazin-1-yl, das unsubstituiert oder

in Position 4 mit einem ($C_1$-$C_4$) -Alkyl substituiert ist;
- $R_{11}$ ein Wasserstoffatom oder eine ($C_1$-$C_4$)-Alkylgruppe darstellt;
- $R_{12}$ ein Wasserstoffatom, eine ($C_1$-$C_4$)-Alkylgruppe, eine Gruppe $-(CH_2)_m$-CO-$R_{16}$ darstellt;
- $R_{13}$ ein Wasserstoffatom, eine ($C_1$-$C_4$)-Alkylgruppe, ein Phenyl, eine Gruppe -$NR_{17}R_{18}$, eine Gruppe

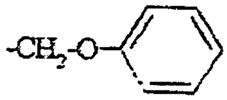

darstellt;
- $R_{14}$ ein Wasserstoffatom, eine ($C_1$-$C_4$) -Alkylgruppe, eine Gruppe -$NR_{17}R_{18}$ darstellt;
- $R_{15}$ ein Wasserstoffatom, eine ($C_1$-$C_4$)-Alkylgruppe, eine Gruppe -$NR_{19}R_{20}$, eine -COO ($C_1$-$C_4$) -Alkylgruppe darstellt;
- $R_{16}$ ein Hydroxy, eine ($C_1$-$C_4$)-Alkoxy, eine Gruppe
- $NR_9R_{10}$ darstellt;
- $R_{17}$ und $R_{18}$, jeweils unabhängig, ein Wasserstoffatom oder eine ($C_1$-$C_4$)-Alkylgruppe darstellen; $R_{18}$ kann außerdem eine Gruppe -$COR_{21}$, eine Gruppe -$SO_2R_{22}$ darstellen;
- $R_{19}$ und $R_{20}$, jeweils unabhängig, ein Wasserstoffatom oder eine ($C_1$-$C_4$)-Alkylgruppe darstellen; $R_{20}$ kann außerdem eine ($C_3$-$C_6$)-Cycloalkylgruppe, eine ($C_3$-$C_6$)-Cyclo-alkylmethylgruppe, eine Gruppe $-(CH_2)_mNR_9R_{10}$ darstellen;
- $R_{21}$ eine ($C_1$-$C_4$)-Alkylgruppe, eine ($C_3$-$C_6$)-Cycloalkylgruppe, eine Gruppe $-(CH_2)_mNR_9R_{10}$ darstellt;
- $R_{22}$ eine ($C_1$-$C_4$)-Alkylgruppe darstellt;
- m 1, 2 oder 3 ist und
- n 0, 1, 2 oder 3 ist;

im Zustand der Base oder eines Säureadditionssalzes sowie im Zustand des Hydrats oder Solvats.

**2.** Verbindung nach Anspruch 1, **dadurch gekennzeichnet, dass**:

- $R_1$ ein Wasserstoffatom, ein Methyl, ein Ethyl, ein Cyanomethyl, ein 2-Morpholin-4-ylethyl darstellt;
- $R_2$ ein Methyl darstellt;
- $R_3$ ein Phenyl, ein 3-Bromphenyl, ein 4-Bromphenyl, ein 2-Chlorphenyl, ein 3-Chlorphenyl, ein 4-Chlorphenyl, ein 3-Fluorphenyl, ein 4-Fluorphenyl, ein 3-Methylphenyl, ein 2-Methoxyphenyl, ein 3-Methoxyphenyl, ein 4-Methoxyphenyl, ein 3-Cyanophenyl, ein 4-Cyanophenyl, ein 2,4-Dichlorphenyl, ein 3,5-Difluorphenyl, ein 2,4-Dimethylphenyl, ein 2,4-Dimethoxyphenyl, ein 2-Methyl-5-fluorphenyl, ein 3-Fluor-4-methylphenyl, ein 3-Methyl-4-fluorphenyl, ein 4-(Aminomethyl)phenyl, ein 4-(Morpholin-4-ylmethyl)phenyl, ein 4-(2-Morpholin-4-ylethoxy)phenyl darstellt;
- $R_4$ Folgendes darstellt:

  . eine (Hydroxyimino)methylgruppe, eine N-Hydroxyethanimidoylgruppe, eine (Ethoxyimino)methylgruppe, eine N-Ethoxyethanimidoylgruppe, eine (Isobutoxyimino)methylgruppe, eine [(Carboxymethoxy)imino]methylgruppe, eine [(2-Ethoxy-2-oxoethoxy)imino]methylgruppe, eine [(2-Morpholin-4-yl-2-oxoethoxy)imino] methylgruppe;
  . ein 3-Methyl-1,2,4-oxadiazol-5-yl, ein 3-Phenyl-1,2,4-oxadiazol-5-yl, ein 3-Amino-1,2,4-oxadiazol-5-yl, ein 3-(Dimethylamino)-1,2,4-oxadiazol-5-yl, ein 3-[(Cyclopropylcarbonyl)amino]-1,2,4-oxadiazol-5-yl, ein 3-[(N,N-Dimethylglycyl)amino]-1,2,4-oxadiazol-5-yl, ein 3-[(Methylsulfonyl)amino]-1,2,4-oxadiazol-5-yl, ein 3-(Phenoxymethyl)-1,2,4-oxadiazol-5-yl;
  . ein 5-Methyl-1,3,4-oxadiazol-2-yl, ein 5-Amino-1,3,4-oxadiazol-2-yl;
  . ein 5-Methyl-1,2,4-oxadiazol-3-yl, ein 5-Amino-1,2,4-oxadiazol-3-yl, ein 5-(Dimethylamino)-1,2,4-oxadiazol-3-yl, ein 5-(Cyclopropylamino)-1,2,4-oxadiazol-3-yl, ein 5-[(Cyclopropylmethyl)amino]-1,2,4-oxadiazol-3-yl, ein 5-[(3-Morpholin-4-ylpropyl)amino]-1,2,4-oxadiazol-3-yl, ein 5-[[2-(Dimethylamino)ethyl]-amino]-1,2,4-oxadiazol-3-yl, ein 5-(Ethoxycarbonyl)-1,2,4-oxadiazol-3-yl;

- $R_5$ ein Wasserstoffatom darstellt;

im Zustand der Base oder eines Säureadditionssalzes sowie im Zustand des Hydrats oder Solvats.

3. Verbindung der Formel (I) nach Anspruch 1, ausgewählt aus:

6-(3-Amino-1,2,4-oxadiazol-5-yl)-3(2,4-dichlorphenyl)-1,9-dimethyl-1,9-dihydro-2H-pyrido[2,3-b]indol-2-on;
3-(2,4-Dichlorphenyl)-1,9-dimethyl-2-oxo-2,9-dihydro-1*H*-pyrido[2,3-b]indol-6-carbaldehydoxim;
3-(2,4-Dichlorphenyl)-1,9-dimethyl-2-oxo-2,9-dihydro-1*H*-pyrido[2,3-b]indol-6-carbaldehyd-O-ethyloxim;
5-[3-(4-Chlorphenyl)-1,9-dimethyl-2,9-dihydro-1*H*-pyrido[2,3-b]indol-6-yl]-1,2,4-oxadiazol-3-amin;
5-[3-(3-Fluorphenyl)-1,9-dimethyl-2,9-dihydro-1*H*-pyrido[2,3-b]indol-6-yl]-1,2,4-oxadiazol-3-amin;
5-[1,9-Dimethyl-3-(3-methylphenyl)-2,9-dihydro-1*H*-pyrido[2,3-b]indol-6-yl]-1,2,4-oxadiazol-3-amin;
3-[(4-Aminomethyl)phenyl]-6-(3-amino-1,2,4-oxadiazol-5-yl)-1,9-dimethyl-1,9-dihydro-2*H*-pyrido[2,3-b] indol-2-on;
5-[1,9-Dimethyl-3-[4-morpholin-4-ylmethyl)phenyl]-2,9-dihydro-1*H*-pyrido[2,3-b]indol-6-yl]-1,2,4-oxadiazol-3-amin;
5-[3-(2,4-Dichlorphenyl)-1,9-dimethyl-2,9-dihydro-1*H*-pyrido[2,3-b]indol-6-yl]-1,3,4-oxadiazol-2-amin;
N'-[3-[3-(2,4-Dichlorphenyl)-1,9-dimethyl-2,9-dihydro-1H-pyrido[2,3-b]indol-6-yl]-1,2,4-oxadiazol-5-yl]-N,N-dimethylethan-1,2-diamin;

im Zustand der Base oder eines Säureadditionssalzes sowie im Zustand des Hydrats oder Solvats.

4. Verfahren zur Herstellung von Verbindungen der Formel (I) nach einem der Ansprüche 1 bis 3, worin $R_4$ eine Gruppe $-CR_{11}=N-O-R_{12}$ darstellt, **dadurch gekennzeichnet, dass**:

man eine Verbindung der Formel:

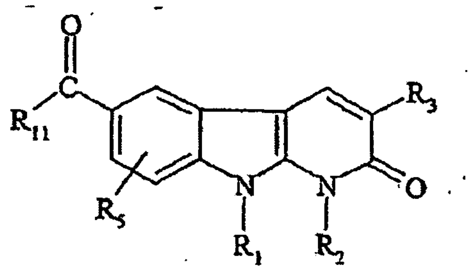

(II)

worin $R_1$, $R_2$, $R_3$, $R_5$ und $R_{11}$ wie für eine Verbindung der Formel (I) in Anspruch 1 definiert sind, mit einem Hydroxylaminderivat der Formel:

$H_2N-O-R_{12}$ (III)

worin $R_{12}$ wie für eine Verbindung der Formel (I) in Anspruch 1 definiert ist, umsetzt.

5. Verfahren zur Herstellung von Verbindungen der Formel (I) nach einem der Ansprüche 1 bis 3, worin $R_4$ =

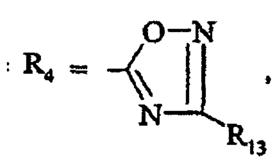

**dadurch gekennzeichnet, dass**:
man eine Verbindung der Formel:

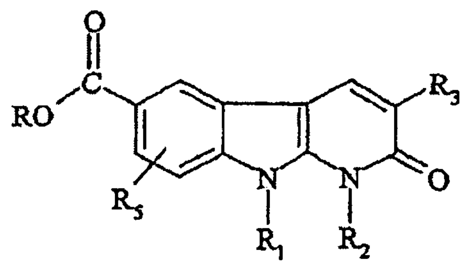

(IV)

worin $R_1$, $R_2$, $R_3$ und $R_5$ wie für eine Verbindung der Formel (I) in Anspruch 1 definiert sind und R ein Wasserstoffatom oder eine ($C_1$-$C_4$)-Alkylgruppe darstellt, mit einem Oximderivat der Formel:

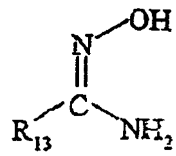

(V)

worin $R_{13}$ wie für eine Verbindung der Formel (I) in Anspruch 1 definiert ist, umsetzt.

**6.** Verfahren zur Herstellung von Verbindungen der Formel (I) nach einem der Ansprüche 1 bis 3, worin $R_4$ =

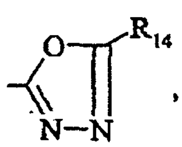

,

**dadurch gekennzeichnet, dass**:
man eine Verbindung der Formel:

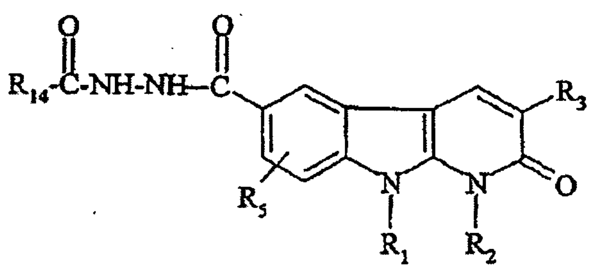

(IX)

worin $R_1$, $R_2$, $R_3$, $R_5$ und $R_{14}$ wie für eine Verbindung der Formel (I) in Anspruch 1 definiert sind, zyklisiert.

**7.** Verfahren zur Herstellung von Verbindungen der Formel (I) nach einem der Ansprüche 1 bis 3, worin $R_4$ =

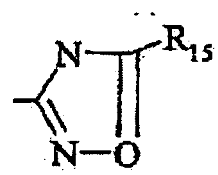

,

**dadurch gekennzeichnet, dass**: man eine Verbindung der Formel:

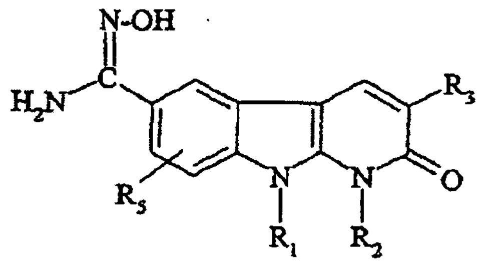

(XI)

worin $R_1$, $R_2$, $R_3$ und $R_5$ wie für eine Verbindung der Formel (I) in Anspruch 1 definiert sind, umsetzt mit:

a) entweder einem Trichloracetylchlorid in Anwesenheit einer Base, um eine Verbindung der Formel:

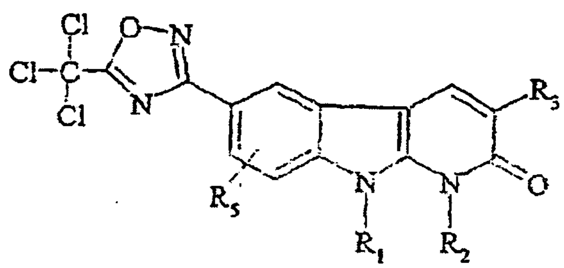

(XII)

zu erhalten, und die so erhaltene Verbindung der Formel (XII) mit einem Amin der Formel $HNR_{19}R_{20}$ umsetzt, wenn man eine Verbindung der Formel (I) herstellen muss, worin $R_{15}$ = $NR_{19}R_{20}$; oder
b) einem Anhydrid der Formel $(R_{15}CO)_2O$, wenn man eine Verbindung der Formel (I) herstellen muss, worin $R_{15}$ = $(C_1$-$C_4)$-Alkyl; oder
c) einem Oxalsäurederivat der Formel

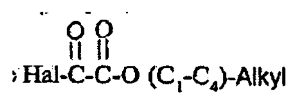

,

worin Hal ein Halogenatom darstellt, wenn man eine Verbindung der Formel (I) herstellen muss, worin $R_{15}$ = COO $(C_1$-$C_4)$-Alkyl.

**8.** Verbindung der Formel:

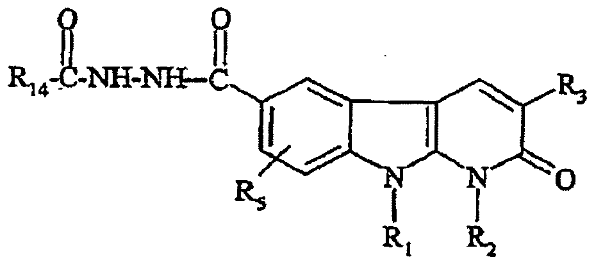

(IX)

worin:

- $R_1$ ein Wasserstoffatom, eine $(C_1-C_4)$-Alkylgruppe, eine Gruppe $-(CH_2)_mOH$, eine Gruppe $-(CH_2)_mCN$, eine Gruppe $-(CH_2)_mNR_9R_{10}$ darstellt;
- $R_2$ ein Wasserstoffatom oder eine $(C_1-C_4)$-Alkylgruppe darstellt;
- $R_3$ ein Phenyl darstellt, das mit $R_6$, $R_7$, $R_8$ substituiert ist;
- $R_5$ ein Wasserstoffatom oder eine $(C_1-C_4)$ - Alkylgruppe darstellt;
- $R_6$, $R_7$ und $R_8$, jeweils unabhängig voneinander, ein Wasserstoffatom, ein Halogenatom, eine $(C_1-C_4)$-Alkylgruppe, eine $(C_1-C_4)$-Alkoxygruppe; ein Hydroxy; ein Cyano, eine Gruppe $-(CH_2)_nNR_9R_{10}$, eine Gruppe $-O(CH_2)_mNR_9R_{10}$ darstellen;
- $R_9$ und $R_{10}$, jeweils unabhängig voneinander, ein Wasserstoffatom oder eine $(C_1-C_4)$-Alkylgruppe darstellen;
- oder $R_9$ und $R_{10}$ zusammen mit dem Stickstoffatom, an das sie gebunden sind, einen heterocyclischen Rest bilden, ausgewählt aus Pyrrolidin-1-yl, Piperidin-1-yl, Morpholin-4-yl oder Piperazin-1-yl, das unsubstituiert oder in Position 4 mit einem $(C_1-C_4)$-Alkyl substituiert ist;
- $R_{14}$ ein Wasserstoffatom, eine $(C_1-C_4)$-Alkylgruppe oder eine Gruppe $-NR_{17}R_{18}$ darstellt;
- $R_{17}$ und $R_{18}$, jeweils unabhängig, ein Wasserstoffatom oder eine $(C_1-C_4)$-Alkylgruppe darstellen; $R_{18}$ kann auch eine Gruppe $-COR_{21}$, eine Gruppe $-SO_2R_{22}$ darstellen;
- $R_{21}$ eine $(C_1-C_4)$-Alkylgruppe, eine $(C_3-C_6)$-Cycloalkylgruppe oder eine Gruppe $-(CH_2)_mNR_9R_{10}$ darstellt;
- $R_{22}$ eine $(C_1-C_4)$-Alkylgruppe darstellt;
- m 1, 2 oder 3 ist,
- n 0, 1, 2 oder 3 ist.

**9.** Verbindung der Formel:

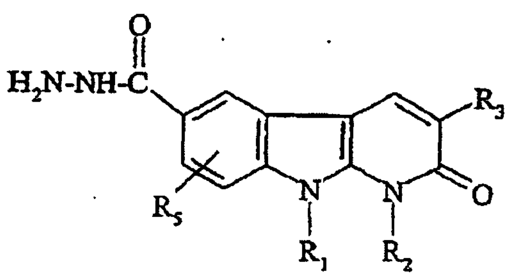

(X)

worin:

- $R_1$ ein Wasserstoffatom, eine $(C_1-C_4)$-Alkylgruppe, eine Gruppe $-(CH_2)_mOH$, eine Gruppe $-(CH_2)_mCN$, eine Gruppe $-(CH_2)_mNR_9R_{10}$ darstellt;
- $R_2$ ein Wasserstoffatom oder eine $(C_1-C_4)$-Alkylgruppe darstellt;
- $R_3$ ein Phenyl darstellt, das mit $R_6$, $R_7$, $R_8$ substituiert ist;
- $R_5$ ein Wasserstoffatom oder eine $(C_1-C_4)$-Alkylgruppe darstellt;
- $R_6$, $R_7$ und $R_8$, jeweils unabhängig voneinander, ein Wasserstoffatom, ein Halogenatom, eine $(C_1-C_4)$-Alkylgruppe, eine $(C_1-C_4)$-Alkoxygruppe; ein Hydroxy; ein Cyano, eine Gruppe $-(CH_2)_nNR_9R_{10}$, eine Gruppe $-O(CH_2)_mNR_9R_{10}$ darstellen;

- $R_9$ und $R_{10}$, jeweils unabhängig voneinander, ein Wasserstoffatom oder eine $(C_1-C_4)$-Alkylgruppe darstellen;
- oder $R_9$ und $R_{10}$ zusammen mit dem Stickstoffatom, an das sie gebunden sind, einen heterocyclischen Rest bilden, ausgewählt aus Pyrrolidin-1-yl, Piperidin-1-yl, Morpholin-4-yl oder Piperazin-1-yl, das unsubstituiert oder in Position 4 mit einem $(C_1-C_4)$-Alkyl substituiert ist;
- m 1, 2 oder 3 ist,
- n 0, 1, 2 oder 3 ist.

**10.** Verbindung der Formel:

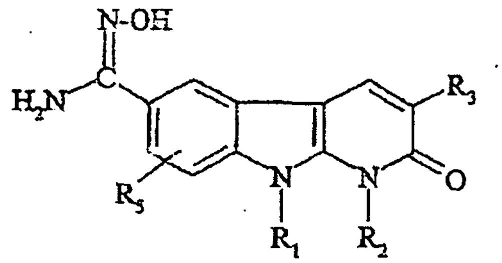

(XI)

worin:

- $R_1$ ein Wasserstoffatom, eine $(C_1-C_4)$ -Alkylgruppe, eine Gruppe $-(CH_2)_mOH$, eine Gruppe $-(CH_2)_mCN$, eine Gruppe $-(CH_2)_mNR_9R_{10}$ darstellt;
- $R_2$ ein Wasserstoffatom oder eine $(C_1-C_4)$-Alkylgruppe darstellt;
- $R_3$ ein Phenyl darstellt, das mit $R_6$, $R_7$, $R_8$ substituiert ist;
- $R_5$ ein Wasserstoffatom oder eine $(C_1-C_4)$-Alkylgruppe darstellt;
- $R_6$, $R_7$ und $R_8$, jeweils unabhängig voneinander, ein Wasserstoffatom, ein Halogenatom, eine $(C_1-C_4)$-Alkylgruppe, eine $(C_1-C_4)$-Alkoxygruppe; ein Hydroxy; ein Cyano, eine Gruppe $-(CH_2)_nNR_9R_{10}$ oder eine Gruppe $-O(CH_2)_mNR_9R_{10}$ darstellen;
- $R_9$ und $R_{10}$, jeweils unabhängig voneinander, ein Wasserstoffatom oder eine $(C_1-C_4)$-Alkylgruppe darstellen;
- oder $R_9$ und $R_{10}$ zusammen mit dem Stickstoffatom, an das sie gebunden sind, einen heterocyclischen Rest bilden, ausgewählt aus Pyrrolidin-1-yl, Piperidin-1-yl, Morpholin-4-yl oder Piperazin-1-yl, das unsubstituiert oder in Position 4 mit einem $(C_1-C_4)$-Alkyl substituiert ist;
- m 1, 2 oder 3 ist,
- n 0, 1, 2 oder 3 ist.

**11.** Verbindung der Formel:

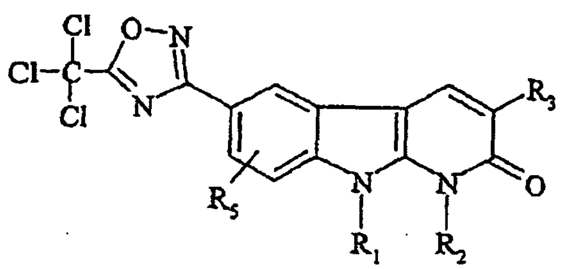

(XII)

worin:

- $R_1$ ein Wasserstoffatom, eine $(C_1-C_9)$-Alkylgruppe, eine Gruppe $-(CH_2)_mOH$, eine Gruppe $-(CH_2)_mCN$, eine Gruppe $-(CH_2)_mNR_9R_{10}$ darstellt;
- $R_2$ ein Wasserstoffatom oder eine $(C_1-C_4)$ - Alkylgruppe darstellt;
- $R_3$ ein Phenyl darstellt, das mit $R_6$, $R_7$, $R_8$ substituiert ist;

- $R_5$ ein Wasserstoffatom oder eine ($C_1$-$C_4$) - Alkylgruppe darstellt;
- $R_6$, $R_7$ und $R_8$, jeweils unabhängig voneinander, ein Wasserstoffatom, ein Halogenatom, eine ($C_1$-$C_4$)-Alkylgruppe, eine ($C_1$-$C_4$) -Alkoxygruppe; ein Hydroxy; ein Cyano, eine Gruppe -$(CH_2)_nNR_9R_{10}$, eine Gruppe -O$(CH_2)_mNR_9R_{10}$ darstellen;
- $R_9$ und $R_{10}$, jeweils unabhängig voneinander, ein Wasserstoffatom oder eine ($C_1$-$C_4$)-Alkylgruppe darstellen;
- oder $R_9$ und $R_{10}$ zusammen mit dem Stickstoffatom, an das sie gebunden sind, einen heterocyclischen Rest bilden, ausgewählt aus Pyrrolidin-1-yl, Piperidin-1-yl, Morpholin-4-yl oder Piperazin-1-yl, das unsubstituiert oder in Position 4 mit einem ($C_1$-$C_4$)-Alkyl substituiert ist;
- m 1, 2 oder 3 ist,
- n 0, 1, 2 oder 3 ist.

**12.** Arzneimittel, **dadurch gekennzeichnet, dass** es eine Verbindung der Formel (I) nach einem der Ansprüche 1 bis 3 oder ein Additionssalz dieser Verbindung mit einer pharmazeutisch annehmbaren Säure oder auch ein Hydrat oder ein Solvat der Verbindung der Formel (I) umfasst.

**13.** Pharmazeutische Zusammensetzung, **dadurch gekennzeichnet, dass** sie eine Verbindung der Formel (I) nach einem der Ansprüche 1 bis 3 oder ein pharmazeutisch annehmbares Salz, ein Hydrat oder ein Solvat dieser Verbindung sowie mindestens einen pharmazeutisch annehmbaren Hilfsstoff umfasst.

**14.** Verwendung einer Verbindung der Formel (I) nach einem der Ansprüche 1 bis 3 zur Herstellung eines Arzneimittels zur Behandlung oder Vorbeugung von Erkrankungen, die durch Proliferation von Tumorzellen verursacht oder verschlimmert werden.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 9708409
- WO 2002087574 A
- WO 2002087575 A
- WO 2004041817 A


### Littérature non-brevet citée dans la description


- **GREEN et al.** Protective Groups in Organic Synthesis. John Wiley & Sons; Inc, 1991
- **J. MARCH.** Advanced Organic Chemistry. Wiley Interscience, 1985, 310-316
- *J. Qrg. Chem. USSR,* 1989, vol. 25 (5), 935-940
- *J. Org. Chem.,* 1982, vol. 47, 2846-2851
- **LIEBIGS.** *Ann. Chem.,* 1980, vol. 3, 344-357
- *J. Am. Chem. Soc.,* 1974, vol. 96, 2127-2129
- **J.M. DEROCQ et al.** *FEBS Letters,* 1998, vol. 425, 419-425
- **E. COLLOMB ; C. DUSSERT ; P.M. MARTIN.** *Cytometry,* 1991, vol. 12 (1), 15-25